# EUROPEAN PATENT APPLICATION

(11) **EP 4 169 912 A1**
(43) Date of publication of application: **26.04.2023**
(21) Application number: 21826599.9
(22) Date of filing: 15.06.2021
(51) Int. Cl.: C07D 311/78, C07D 407/12, C07D 407/14, H01L 51/00, H01L 51/50

(54) **HETEROCYCLIC COMPOUND AND ORGANIC LIGHT-EMITTING DEVICE COMPRISING SAME**

(30) Priority: 17.06.2020 KR 20200073611
(71) Applicant: LT Materials Co., Ltd., Yongin-si, Gyeonggi-do 17118 (KR)
(72) Inventor: PARK, Ji-Yeon, Yongin-si, Gyeonggi-do 17118 (KR); LEE, Nam-Jin, Yongin-si, Gyeonggi-do 17118 (KR); JEONG, Won-Jang, Yongin-si, Gyeonggi-do 17118 (KR); KIM, Dong-Jun, Yongin-si, Gyeonggi-do 17118 (KR)
(74) Representative: Goddar, Heinz J.
(86) International application number: PCT/KR2021/007496
(87) International publication number: WO 2021/256836

(57) **Abstract**

The present specification relates to a heterocyclic compound represented by Chemical Formula 1, and an organic light emitting device comprising the same.

## Description

### [Technical Field]

The present specification relates to a heterocyclic compound, and an organic light emitting device comprising the same.

This application claims priority to and the benefits of Korean Patent Application No. 10-2020-0073611, filed with the Korean Intellectual Property Office on June 17, 2020, the entire contents of which are incorporated herein by reference.

### [Background Art]

An electroluminescent device is one type of self-emissive display devices, and has an advantage of having a wide viewing angle, and a high response speed as well as having an excellent contrast.

An organic light emitting device has a structure disposing an organic thin film between two electrodes. When a voltage is applied to an organic light emitting device having such a structure, electrons and holes injected from the two electrodes bind and pair in the organic thin film, and light emits as these annihilate. The organic thin film may be formed in a single layer or a multilayer as necessary.

A material of the organic thin film may have a light emitting function as necessary. For example, as a material of the organic thin film, compounds capable of forming a light emitting layer themselves alone may be used, or compounds capable of performing a role of a host or a dopant of a host-dopant-based light emitting layer may also be used. In addition thereto, compounds capable of performing roles of hole injection, hole transfer, electron blocking, hole blocking, electron transfer, electron injection and the like may also be used as a material of the organic thin film.

Development of an organic thin film material has been continuously required for enhancing performance, lifetime or efficiency of an organic light emitting device.

### [Disclosure]

### [Technical Problem]

The present specification is directed to providing a heterocyclic compound, and an organic light emitting device comprising the same.

### [Technical Solution]

One embodiment of the present specification provides a heterocyclic compound represented by the following Chemical Formula 1.

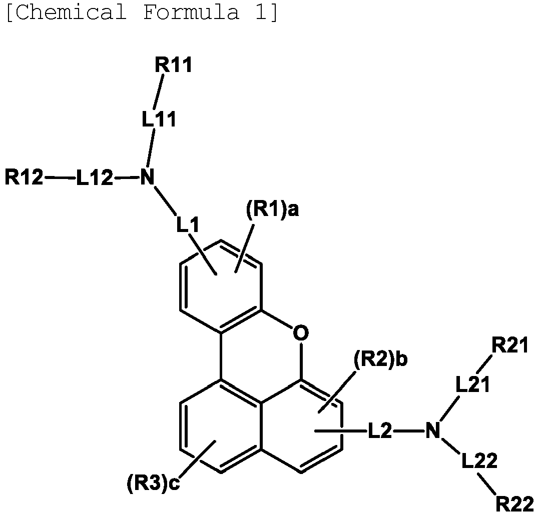

In Chemical Formula 1,
L1, L2, L11, L12, L21 and L22 are each independently a direct bond; a substituted or unsubstituted C6 to C60 arylene group; or a substituted or unsubstituted C2 to C60 heteroarylene group,
R1 to R3, R11, R12, R21 and R22 are each independently hydrogen; deuterium; a halogen group; a cyano group; a substituted or unsubstituted C1 to C60 alkyl group; a substituted or unsubstituted C6 to C60 aryl group; or a substituted or unsubstituted C2 to C60 heteroaryl group,
a and c are each an integer of 1 to 3,
b is 1 or 2,
when a is 2 or greater, R1s are the same as or different from each other,
when b is 2, R2s are the same as or different from each other, and
when c is 2 or greater, R3s are the same as or different from each other.

In addition, one embodiment of the present application provides an organic light emitting device comprising a first electrode; a second electrode provided opposite to the first electrode; and one or more organic material layers provided between the first electrode and the second electrode, wherein one or more layers of the organic material layers comprise one or more types of the heterocyclic compound represented by Chemical Formula 1.

### [Advantageous Effects]

A heterocyclic compound described in the present specification can be used as a material of an organic material layer of an organic light emitting device. The heterocyclic compound is capable of performing a role of a hole injection material, a hole transfer material, a light emitting material, an electron transfer material, an electron injection material and the like in an organic light emitting device. Particularly, the heterocyclic compound can be used as a material of a hole transfer layer or an electron blocking layer of an organic light emitting device.

Chemical Formula 1 has benzoxanthene as a core structure, and by the benzene rings on both sides of the pyran ring each having an amine group as a substituent, steric hindrance occurs resulting in an increase in the T1 value, and as a result, a device driving voltage can be lowered, light emission efficiency can be enhanced, and device lifetime properties can be enhanced.

### [Description of Drawings]

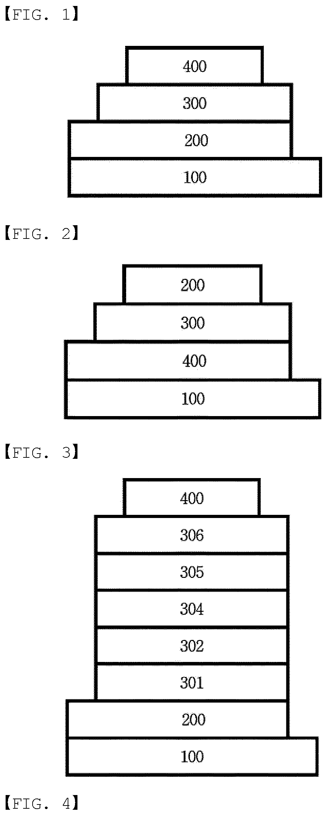

FIG. 1 to FIG. 4 are diagrams each illustrating a lamination structure of an organic light emitting device according to one embodiment of the present specification.
- 100:: Substrate
- 200:: Anode
- 300:: Organic Material Layer
- 301:: Hole Injection Layer
- 302:: Hole Transfer Layer
- 303:: Electron Blocking Layer
- 304:: Light Emitting Layer
- 305:: Electron Transfer Layer
- 306:: Electron Injection Layer
- 400:: Cathode

### [Mode for Disclosure]

Hereinafter, the present specification will be described in more detail.

In the present specification, a description of a certain part "comprising" certain constituents means capable of further including other constituents, and does not exclude other constituents unless particularly stated on the contrary.

In the present specification, "room temperature" means 20°C±10°C.

In the present specification, a T1 value means an energy level value in a triplet state.

A term "substitution" means a hydrogen atom bonding to a carbon atom of a compound being changed to another substituent, and the position of substitution is not limited as long as it is a position at which the hydrogen atom is substituted, that is, a position at which a substituent can substitute, and when two or more substituents substitute, the two or more substituents may be the same as or different from each other.

In the present specification, 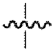 means a substituted position.

In the present specification, "substituted or unsubstituted" means being substituted with one or more substituents selected from the group consisting of deuterium; a halogen group; a cyano group; a C1 to C60 alkyl group; a C2 to C60 alkenyl group; a C2 to C60 alkynyl group; a C3 to C60 cycloalkyl group; a C2 to C60 heterocycloalkyl group; a C6 to C60 aryl group; a C2 to C60 heteroaryl group; a silyl group; a phosphine oxide group; and an amine group, or being unsubstituted, or being substituted with a substituent linking two or more substituents selected from among the substituents illustrated above, or being unsubstituted.

In the present specification, a "case of a substituent being not indicated in a chemical formula or compound structure" means that a hydrogen atom bonds to a carbon atom. However, since deuterium (²H) is an isotope of hydrogen, some hydrogen atoms may be deuterium.

In one embodiment of the present application, a "case of a substituent being not indicated in a chemical formula or compound structure" may mean that positions that may come as a substituent may all be hydrogen or deuterium. In other words, since deuterium is an isotope of hydrogen, some hydrogen atoms may be deuterium that is an isotope, and herein, a content of the deuterium may be from 0% to 100%.

In one embodiment of the present application, in a "case of a substituent being not indicated in a chemical formula or compound structure", hydrogen and deuterium may be mixed in compounds when deuterium is not explicitly excluded such as a deuterium content being 0%, a hydrogen content being 100% or substituents being all hydrogen.

In one embodiment of the present application, deuterium is one of isotopes of hydrogen, is an element having deuteron formed with one proton and one neutron as a nucleus, and may be expressed as hydrogen-2, and the elemental symbol may also be written as D or ²H.

In one embodiment of the present application, an isotope means an atom with the same atomic number (Z) but with a different mass number (A), and may also be interpreted as an element with the same number of protons but with a different number of neutrons.

In one embodiment of the present application, a meaning of a content T% of a specific substituent may be defined as T2/T1×100=T% when the total number of substituents that a basic compound may have is defined as T1, and the number of specific substituents among these is defined as T2.

In other words, in one example, having a deuterium content of 20% in a phenyl group represented by 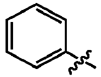 means that the total number of substituents that the phenyl group may have is 5 (T1 in the formula), and the number of deuterium among these is 1 (T2 in the formula). In other words, having a deuterium content of 20% in a phenyl group may be represented by the following structural formulae.

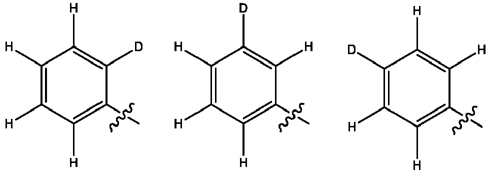

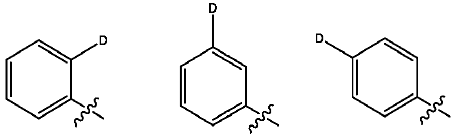

In addition, in one embodiment of the present application, "a phenyl group having a deuterium content of 0%" may mean a phenyl group that does not include a deuterium atom, that is, a phenyl group that has 5 hydrogen atoms.

In the present specification, the halogen group may be fluorine, chlorine, bromine or iodine.

In the present specification, the alkyl group includes linear or branched, and may be further substituted with other substituents. The number of carbon atoms of the alkyl group may be from 1 to 60, specifically from 1 to 40 and more specifically from 1 to 20. Specific examples thereof may include a methyl group, an ethyl group, a propyl group, an n-propyl group, an isopropyl group, a butyl group, an n-butyl group, an isobutyl group, a tert-butyl group, a sec-butyl group, a 1-methyl-butyl group, a 1-ethyl-butyl group, a pentyl group, an n-pentyl group, an isopentyl group, a neopentyl group, a tert-pentyl group, a hexyl group, an n-hexyl group, a 1-methylpentyl group, a 2-methylpentyl group, a 4-methyl-2-pentyl group, a 3,3-dimethylbutyl group, a 2-ethylbutyl group, a heptyl group, an n-heptyl group, a 1-methylhexyl group, an octyl group, an n-octyl group, a tert-octyl group, a 1-methylheptyl group, a 2-ethylhexyl group, a 2-propylpentyl group, an n-nonyl group, a 2,2-dimethylheptyl group, a 1-ethylpropyl group, a 1,1-dimethyl-propyl group, an isohexyl group, a 2-methylpentyl group, a 4-methylhexyl group, a 5-methylhexyl group and the like, but are not limited thereto.

In the present specification, the alkenyl group includes linear or branched, and may be further substituted with other substituents. The number of carbon atoms of the alkenyl group may be from 2 to 60, specifically from 2 to 40 and more specifically from 2 to 20. Specific examples thereof may include a vinyl group, a 1-propenyl group, an isopropenyl group, a 1-butenyl group, a 2-butenyl group, a 3-butenyl group, a 1-pentenyl group, a 2-pentenyl group, a 3-pentenyl group, a 3-methyl-1-butenyl group, a 1,3-butadienyl group, an allyl group, a 1-phenylvinyl-1-yl group, a 2-phenylvinyl-1-yl group, a 2,2-diphenylvinyl-1-yl group, a 2-phenyl-2-(naphthyl-1-yl)vinyl-1-yl group, a 2,2-bis(diphenyl-1-yl)vinyl-1-yl group, a stilbenyl group, a styrenyl group and the like, but are not limited thereto.

In the present specification, the alkynyl group includes linear or branched, and may be further substituted with other substituents. The number of carbon atoms of the alkynyl group may be from 2 to 60, specifically from 2 to 40 and more specifically from 2 to 20.

In the present specification, the cycloalkyl group includes monocyclic or polycyclic, and may be further substituted with other substituents. Herein, the polycyclic means a group in which the cycloalkyl group is directly linked to or fused with other cyclic groups. Herein, the other cyclic groups may be a cycloalkyl group, but may also be different types of cyclic groups such as a heterocycloalkyl group, an aryl group and a heteroaryl group. The number of carbon groups of the cycloalkyl group may be from 3 to 60, specifically from 3 to 40 and more specifically from 5 to 20. Specific examples thereof may include a cyclopropyl group, a cyclobutyl group, a cyclopentyl group, a 3-methylcyclopentyl group, a 2,3-dimethylcyclopentyl group, a cyclohexyl group, a 3-methylcyclohexyl group, a 4-methylcyclohexyl group, a 2,3-dimethylcyclohexyl group, a 3,4,5-trimethylcyclohexyl group, a 4-tert-butylcyclohexyl group, a cycloheptyl group, a cyclooctyl group and the like, but are not limited thereto.

In the present specification, the heterocycloalkyl group includes O, S, Se, N or Si as a heteroatom, includes monocyclic or polycyclic, and may be further substituted with other substituents. Herein, the polycyclic means a group in which the heterocycloalkyl group is directly linked to or fused with other cyclic groups. Herein, the other cyclic groups may be a heterocycloalkyl group, but may also be different types of cyclic groups such as a cycloalkyl group, an aryl group and a heteroaryl group. The number of carbon atoms of the heterocycloalkyl group may be from 2 to 60, specifically from 2 to 40 and more specifically from 3 to 20.

In the present specification, the aryl group includes monocyclic or polycyclic, and may be further substituted with other substituents. Herein, the polycyclic means a group in which the aryl group is directly linked to or fused with other cyclic groups. Herein, the other cyclic groups may be an aryl group, but may also be different types of cyclic groups such as a cycloalkyl group, a heterocycloalkyl group and a heteroaryl group. The aryl group includes a spiro group. The number of carbon atoms of the aryl group may be from 6 to 60, specifically from 6 to 40 and more specifically from 6 to 25. Specific examples of the aryl group may include a phenyl group, a biphenyl group, a ter-phenyl group, a naphthyl group, an anthryl group, a chrysenyl group, a phenanthrenyl group, a perylenyl group, a fluoranthenyl group, a triphenylenyl group, a phenalenyl group, a pyrenyl group, a tetracenyl group, a pentacenyl group, a fluorenyl group, an indenyl group, an acenaphthylenyl group, a benzofluorenyl group, a spirobifluorenyl group, a 2,3-dihydro-1H-indenyl group, a fused ring group thereof, and the like, but are not limited thereto.

In the present specification, the ter-phenyl group may be substituted, and, like the following structural formulae, includes linear or branched.

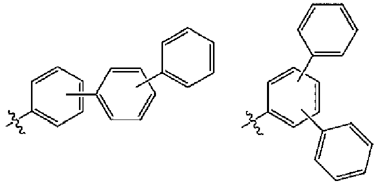

In the present specification, the fluorenyl group may be substituted, and adjacent substituents may bond to each other to form a ring.

When the fluorenyl group is substituted, 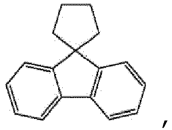 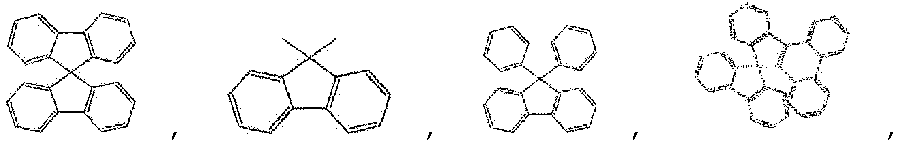 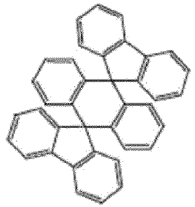 and the like may be included, however, the structure is not limited thereto.

In the present specification, the heteroaryl group includes O, S, SO₂, Se, N or Si as a heteroatom, includes monocyclic or polycyclic, and may be further substituted with other substituents. Herein, the polycyclic means a group in which the heteroaryl group is directly linked to or fused with other cyclic groups. Herein, the other cyclic groups may be a heteroaryl group, but may also be different types of cyclic groups such as a cycloalkyl group, a heterocycloalkyl group and an aryl group. The number of carbon atoms of the heteroaryl group may be from 2 to 60, specifically from 2 to 40 and more specifically from 3 to 25. Specific examples of the heteroaryl group may include a pyridyl group, a pyrrolyl group, a pyrimidyl group, a pyridazinyl group, a furanyl group, a thiophene group, an imidazolyl group, a pyrazolyl group, an oxazolyl group, an isoxazolyl group, a thiazolyl group, an isothiazolyl group, a triazolyl group, a furazanyl group, an oxadiazolyl group, a thiadiazolyl group, a dithiazolyl group, a tetrazolyl group, a pyranyl group, a thiopyranyl group, a diazinyl group, an oxazinyl group, a thiazinyl group, a dioxynyl group, a triazinyl group, a tetrazinyl group, a quinolyl group, an isoquinolyl group, a quinazolinyl group, an isoquinazolinyl group, a qninozolinyl group, a naphthyridyl group, an acridinyl group, a phenanthridinyl group, an imidazopyridinyl group, a diazanaphthalenyl group, a triazaindene group, an indolyl group, an indolizinyl group, a benzothiazolyl group, a benzoxazolyl group, a benzimidazolyl group, a benzothiophene group, a benzofuran group, a dibenzothiophene group, a dibenzofuran group, a carbazolyl group, a benzocarbazolyl group, a dibenzocarbazolyl group, a phenazinyl group, a dibenzosilole group, spirobi(dibenzosilole), a dihydrophenazinyl group, a phenoxazinyl group, a phenanthridyl group, a thienyl group, an indolo[2,3-a]carbazolyl group, an indolo[2,3-b]carbazolyl group, an indolinyl group, a 10,11-dihydro-dibenzo[b,f]azepine group, a 9,10-dihydroacridinyl group, a phenanthrazinyl group, a phenothiathiazinyl group, a phthalazinyl group, a naphthylidinyl group, a phenanthrolinyl group, a benzo[c][1,2,5]thiadiazolyl group, 5,10-dihydrobenzo[b,e][1,4]azasilinyl, a pyrazolo[1,5-c]quinazolinyl group, a pyrido[1,2-b]indazolyl group, a pyrido[1,2-a]imidazo[1,2-e]indolinyl group, a benzofuro[2,3-d]pyrimidyl group; a benzothieno[2,3-d]pyrimidyl group; a benzofuro[2,3-a]carbazolyl group, a benzothieno[2,3-a]carbazolyl group, a 1,3-dihydroindolo[2,3-a]carbazolyl group, a benzofuro[3,2-a]carbazolyl group, a benzothieno[3,2-a]carbazolyl group, a 1,3-dihydroindolo[3,2-a]carbazolyl group, a benzofuro[2,3-b]carbazolyl group, a benzothieno[2,3-b]carbazolyl group, a 1,3-dihydroindolo[2,3-b]carbazolyl group, a benzofuro[3,2-b]carbazolyl group, a benzothieno[3,2-b]carbazolyl group, a 1,3-dihydroindolo[3,2-b]carbazolyl group, a benzofuro[2,3-c]carbazolyl group, a benzothieno[2,3-c]carbazolyl group, a 1,3-dihydroindolo[2,3-c]carbazolyl group, a benzofuro[3,2-c]carbazolyl group, a benzothieno[3,2-c]carbazolyl group, a 1,3-dihydroindolo[3,2-c]carbazolyl group, a 1,3-dihydroindeno[2,1-b]carbazolyl group, a 5,11-dihydroindeno[1,2-b]carbazolyl group, a 5,12-dihydroindeno[1,2-c]carbazolyl group, a 5,8-dihydroindeno[2,1-c]carbazolyl group, a 7,12-dihydroindeno[1,2-a]carbazolyl group, a 11,12-dihydroindeno[2,1-a]carbazolyl group and the like, but are not limited thereto.

In the present specification, the silyl group is a substituent including Si, having the Si atom directly linked as a radical, and is represented by -Si(R101)(R102)(R103). R101 to R103 are the same as or different from each other, and may be each independently a substituent formed with at least one of hydrogen; deuterium; a halogen group; an alkyl group; an alkenyl group; an alkoxy group; a cycloalkyl group; an aryl group; and a heteroaryl group. Specific examples of the silyl group may include a trimethylsilyl group, a triethylsilyl group, a t-butyldimethylsilyl group, a vinyldimethylsilyl group, a propyldimethylsilyl group, a triphenylsilyl group, a diphenylsilyl group, a phenylsilyl group and the like, but are not limited thereto.

In the present specification, the phosphine oxide group is represented by -P(=O) (R104) (R105), and R104 and R105 are the same as or different from each other and may be each independently a substituent formed with at least one of hydrogen; deuterium; a halogen group; an alkyl group; an alkenyl group; an alkoxy group; a cycloalkyl group; an aryl group; and a heteroaryl group. Specifically, the phosphine oxide group may be substituted with an aryl group, and as the aryl group, the examples described above may be applied. Examples of the phosphine oxide group may include a dimethylphosphine oxide group, a diphenylphosphine oxide group, a dinaphthylphosphine oxide group and the like, but are not limited thereto.

In the present specification, the amine group is represented by -N(R106) (R107), and R106 and R107 are the same as or different from each other and may be each independently a substituent formed with at least one of hydrogen; deuterium; a halogen group; an alkyl group; an alkenyl group; an alkoxy group; a cycloalkyl group; an aryl group; and a heteroaryl group. The amine group may be selected from the group consisting of -NH₂; a monoalkylamine group; a monoarylamine group; a monoheteroarylamine group; a dialkylamine group; a diarylamine group; a diheteroarylamine group; an alkylarylamine group; an alkylheteroarylamine group; and an arylheteroarylamine group, and although not particularly limited thereto, the number of carbon atoms is preferably from 1 to 30. Specific examples of the amine group may include a methylamine group, a dimethylamine group, an ethylamine group, a diethylamine group, a phenylamine group, a naphthylamine group, a biphenylamine group, a dibiphenylamine group, an anthracenylamine group, a 9-methyl-anthracenylamine group, a diphenylamine group, a phenylnaphthylamine group, a ditolylamine group, a phenyltolylamine group, a triphenylamine group, a biphenylnaphthylamine group, a phenylbiphenylamine group, a biphenylfluorenylamine group, a phenyltriphenylenylamine group, a biphenyltriphenylenylamine group and the like, but are not limited thereto.

In the present specification, the examples of the aryl group described above may be applied to the arylene group except that the arylene group is a divalent group.

In the present specification, the examples of the heteroaryl group described above may be applied to the heteroarylene group except that the heteroarylene group is a divalent group.

In the present specification, the "adjacent" group may mean a substituent substituting an atom directly linked to an atom substituted by the corresponding substituent, a substituent sterically most closely positioned to the corresponding substituent, or another substituent substituting an atom substituted by the corresponding substituent. For example, two substituents substituting ortho positions in a benzene ring, and two substituents substituting the same carbon in an aliphatic ring may be interpreted as groups "adjacent" to each other.

As the aliphatic or aromatic hydrocarbon ring or the aliphatic or aromatic heteroring that adjacent groups may form, the structures illustrated as the cycloalkyl group, the heterocycloalkyl group, the aryl group and the heteroaryl group described above may be used except for those that are not a monovalent.

One embodiment of the present specification provides a heterocyclic compound represented by Chemical Formula 1.

In one embodiment of the present specification, L1 and L2 are each independently a direct bond; a substituted or unsubstituted C6 to C60 arylene group; or a substituted or unsubstituted C2 to C60 heteroarylene group.

In one embodiment of the present specification, L1 and L2 are each independently a direct bond; a substituted or unsubstituted C6 to C30 arylene group; or a substituted or unsubstituted C2 to C30 heteroarylene group.

In one embodiment of the present specification, L1 and L2 are each independently a direct bond; or a substituted or unsubstituted C6 to C20 arylene group.

In one embodiment of the present specification, L1 and L2 are each independently a direct bond; a substituted or unsubstituted phenylene group; a substituted or unsubstituted biphenylene group; a substituted or unsubstituted ter-phenylene group; a substituted or unsubstituted naphthylene group; a substituted or unsubstituted anthracenylene group; or a substituted or unsubstituted triphenylenylene group.

In one embodiment of the present specification, L1 and L2 are each independently a direct bond; a phenylene group; a biphenylene group; a ter-phenylene group; a naphthylene group; an anthracenylene group; or a triphenylenylene group.

In one embodiment of the present specification, L11, L12, L21 and L22 are each independently a direct bond; a substituted or unsubstituted C6 to C60 arylene group; or a substituted or unsubstituted C2 to C60 heteroarylene group.

In one embodiment of the present specification, L11, L12, L21 and L22 are each independently a direct bond; a substituted or unsubstituted C6 to C30 arylene group; or a substituted or unsubstituted C2 to C30 heteroarylene group.

In one embodiment of the present specification, L11, L12, L21 and L22 are each independently a direct bond; or a substituted or unsubstituted C6 to C30 arylene group.

In one embodiment of the present specification, L11, L12, L21 and L22 are each independently a direct bond; or a C6 to C20 arylene group.

In one embodiment of the present specification, L11, L12, L21 and L22 are each independently a direct bond; or a phenylene group.

In one embodiment of the present specification, R11, R12, R21 and R22 are each independently hydrogen; deuterium; a halogen group; a cyano group; a substituted or unsubstituted C1 to C60 alkyl group; a substituted or unsubstituted C6 to C60 aryl group; or a substituted or unsubstituted C2 to C60 heteroaryl group.

In one embodiment of the present specification, R11, R12, R21 and R22 are each independently hydrogen; deuterium; a halogen group; a cyano group; a substituted or unsubstituted C1 to C20 alkyl group; a substituted or unsubstituted C6 to C40 aryl group; or a substituted or unsubstituted C2 to C40 heteroaryl group.

In one embodiment of the present specification, R11, R12, R21 and R22 are each independently hydrogen; deuterium; a halogen group; a cyano group; a substituted or unsubstituted C6 to C40 aryl group; or a substituted or unsubstituted C2 to C40 heteroaryl group.

In one embodiment of the present specification, R11, R12, R21 and R22 are each independently hydrogen; deuterium; a substituted or unsubstituted C6 to C40 aryl group; or a substituted or unsubstituted C2 to C40 heteroaryl group.

In one embodiment of the present specification, R11, R12, R21 and R22 are each independently a substituted or unsubstituted C6 to C40 aryl group; or a substituted or unsubstituted C2 to C40 heteroaryl group.

In one embodiment of the present specification, R11, R12, R21 and R22 are each independently a substituted or unsubstituted C6 to C30 aryl group; or a substituted or unsubstituted C2 to C30 heteroaryl group.

In one embodiment of the present specification, R11, R12, R21 and R22 are each independently a substituted or unsubstituted phenyl group; a substituted or unsubstituted biphenyl group; a substituted or unsubstituted ter-phenyl group; a substituted or unsubstituted naphthyl group; a substituted or unsubstituted fluorenyl group; a substituted or unsubstituted dibenzofuran group; or a substituted or unsubstituted dibenzothiophene group.

In one embodiment of the present specification, R11, R12, R21 and R22 are each independently a phenyl group unsubstituted or substituted with an aryl group or a heteroaryl group; a biphenyl group; a ter-phenyl group; a naphthyl group; a fluorenyl group unsubstituted or substituted with an alkyl group or an aryl group; a dibenzofuran group; or a dibenzothiophene group.

In one embodiment of the present specification, R11, R12, R21 and R22 are each independently a phenyl group unsubstituted or substituted with an aryl group or a heteroaryl group; a biphenyl group; a ter-phenyl group; a naphthyl group; a dimethylfluorenyl group; a diphenylfluorenyl group; a dibenzofuran group; or a dibenzothiophene group.

In one embodiment of the present specification, R11 and R12 are the same as or different from each other.

In one embodiment of the present specification, R21 and R22 are the same as or different from each other.

In one embodiment of the present specification, R11 and R12 are the same as each other, and R21 and R22 are different from each other.

In one embodiment of the present specification, R11 and R12 are different from each other, and R21 and R22 are the same as each other.

In one embodiment of the present specification, R11 and R12, and R21 and R22 are each the same as each other.

In one embodiment of the present specification, R11 and R12, and R21 and R22 are each different from each other.

In one embodiment of the present specification, R1 to R3 are each independently hydrogen; deuterium; a halogen group; a cyano group; a substituted or unsubstituted C1 to C60 alkyl group; a substituted or unsubstituted C6 to C60 aryl group; or a substituted or unsubstituted C2 to C60 heteroaryl group.

In one embodiment of the present specification, R1 to R3 are each independently hydrogen; deuterium; a substituted or unsubstituted C6 to C60 aryl group; or a substituted or unsubstituted C2 to C60 heteroaryl group.

In one embodiment of the present specification, R1 to R3 are each independently hydrogen; deuterium; a substituted or unsubstituted C6 to C30 aryl group; or a substituted or unsubstituted C2 to C30 heteroaryl group.

In one embodiment of the present specification, R1 to R3 are each independently hydrogen; deuterium; a substituted or unsubstituted C6 to C20 aryl group; or a substituted or unsubstituted C2 to C20 heteroaryl group.

In one embodiment of the present specification, R1 to R3 are hydrogen; or deuterium.

In one embodiment of the present specification, R1 to R3 are hydrogen.

In one embodiment of the present specification, Chemical Formula 1 may be represented by the following Chemical Formula 1-1.

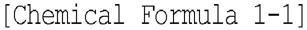

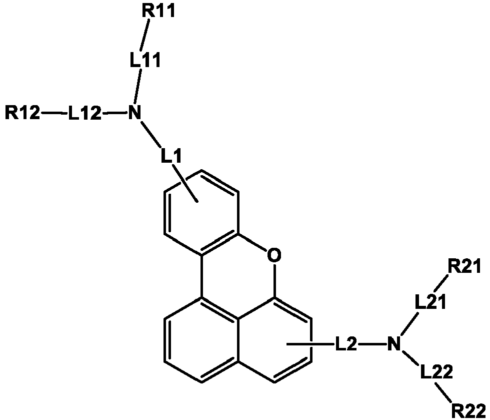

In Chemical Formula 1-1,
each substituent has the same definition as in Chemical Formula 1.

In one embodiment of the present specification, Chemical Formula 1 may be represented by any one of the following Chemical Formulae 2-1 to 2-3.

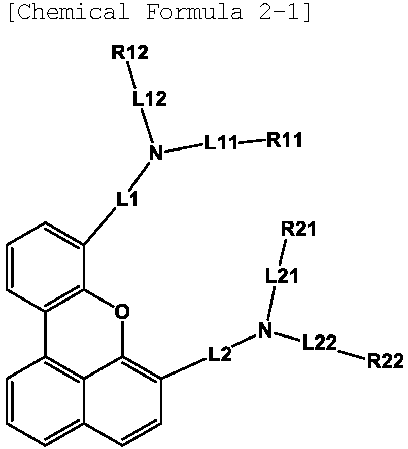

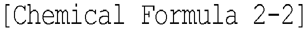

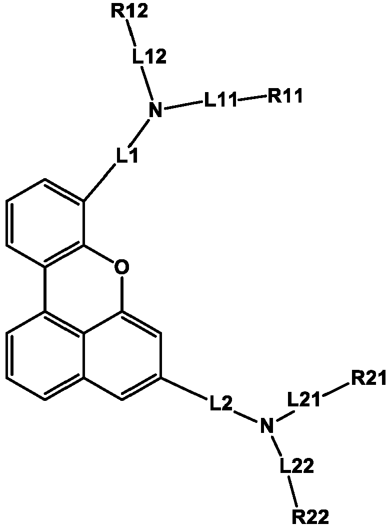

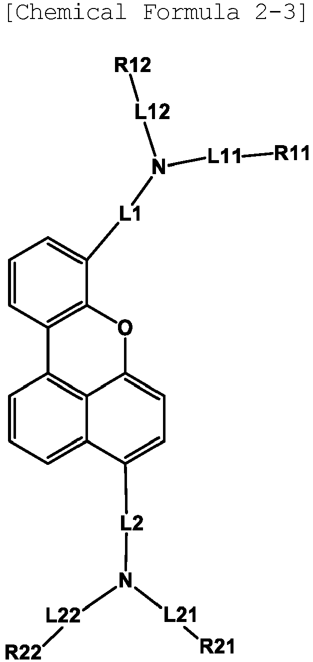

In Chemical Formulae 2-1 to 2-3,
each substituent has the same definition as in Chemical Formula 1.

In one embodiment of the present specification, Chemical Formula 1 may be represented by Chemical Formula 2-1 or 2-2.

In one embodiment of the present specification, Chemical Formula 1 may be represented by any one of the following Chemical Formulae 3-1 to 3-3.

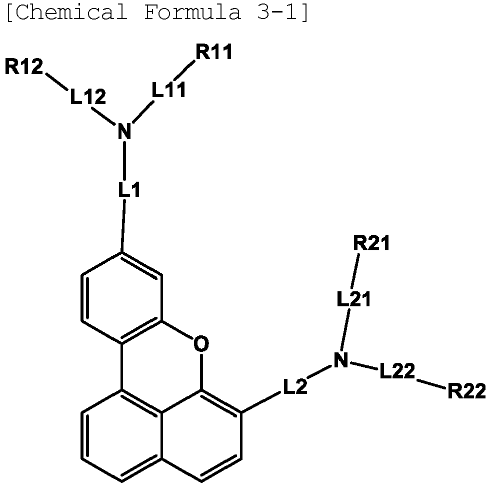

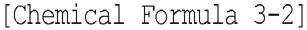

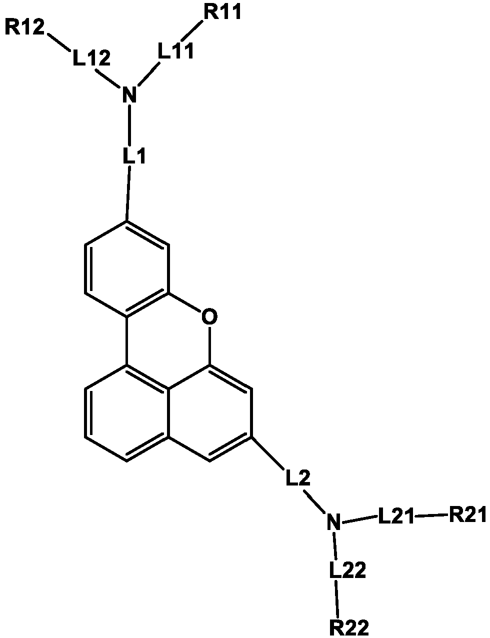

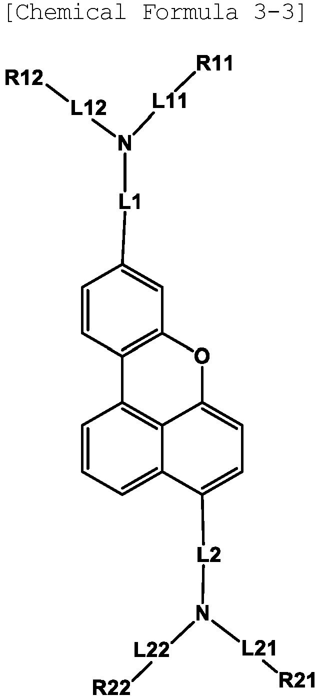

In Chemical Formulae 3-1 to 3-3,
each substituent has the same definition as in Chemical Formula 1.

In one embodiment of the present specification, Chemical Formula 1 may be represented by Chemical Formula 3-1 or 3-2.

In one embodiment of the present specification, Chemical Formula 1 may be represented by any one of the following Chemical Formulae 4-1 to 4-3.

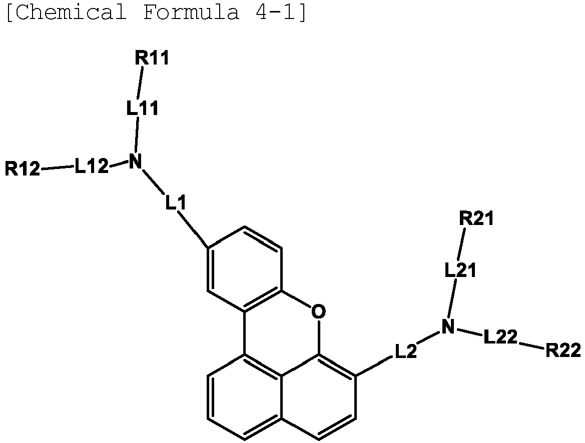

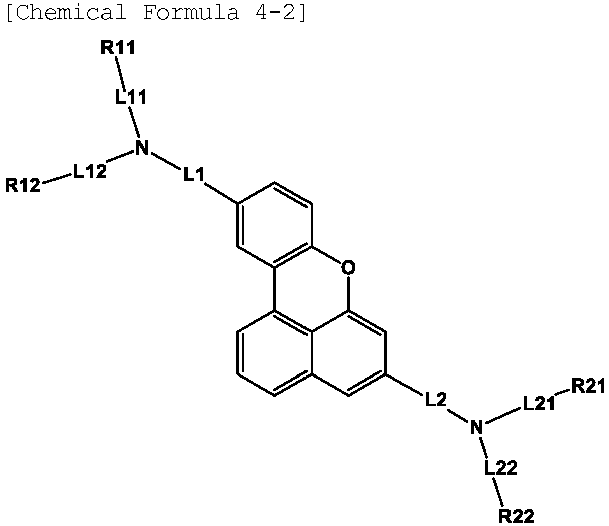

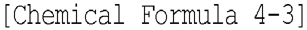

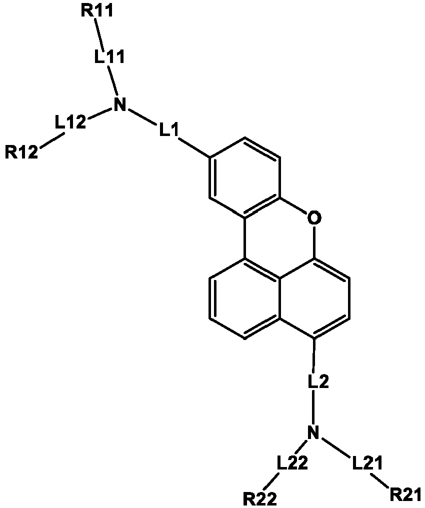

In Chemical Formulae 4-1 to 4-3,
each substituent has the same definition as in Chemical Formula 1.

In one embodiment of the present specification, Chemical Formula 1 may be represented by Chemical Formula 4-1 or 4-2.

In one embodiment of the present specification, Chemical Formula 1 may be represented by any one of the following Chemical Formulae 5-1 to 5-3.

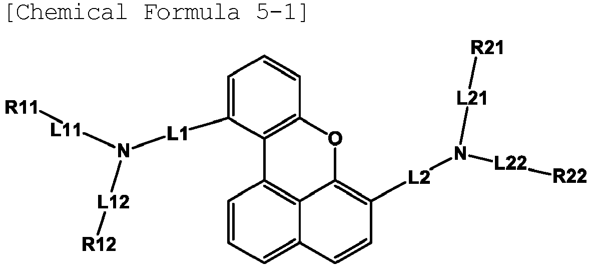

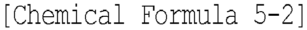

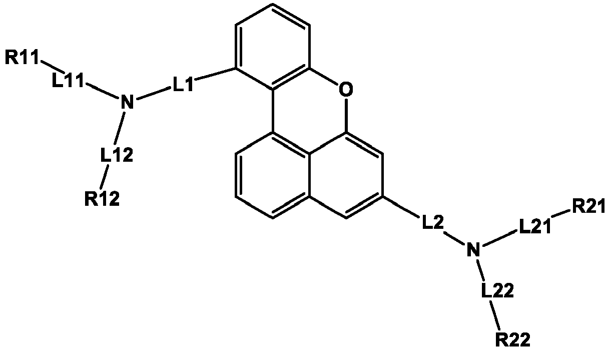

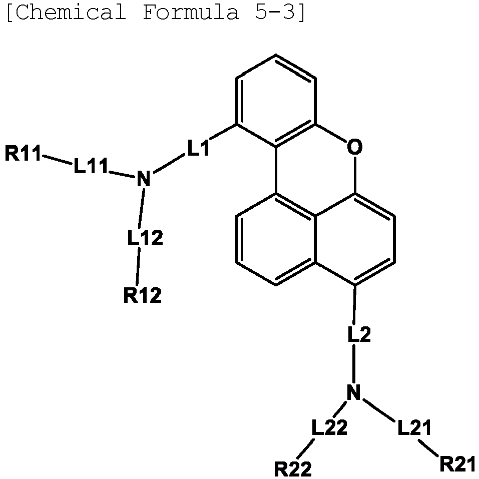

In Chemical Formulae 5-1 to 5-3,
each substituent has the same definition as in Chemical Formula 1.

In one embodiment of the present specification, Chemical Formula 1 may be represented by Chemical Formula 5-1 or 5-2.

In one embodiment of the present specification, Chemical Formula 1 may be represented by any one of the following compounds, but is not limited thereto.

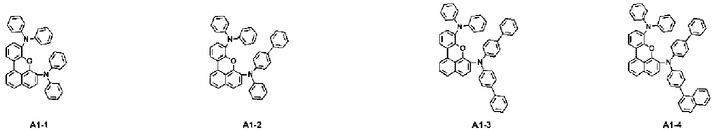

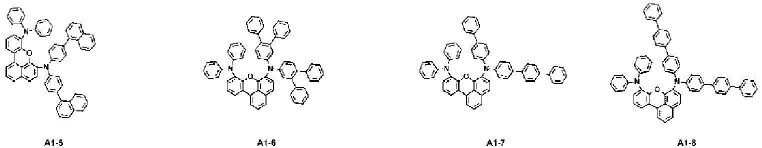

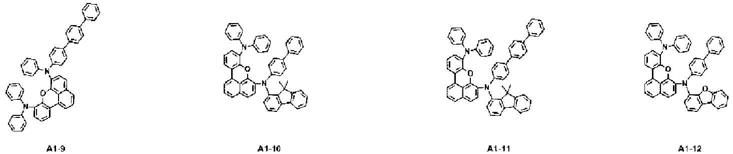

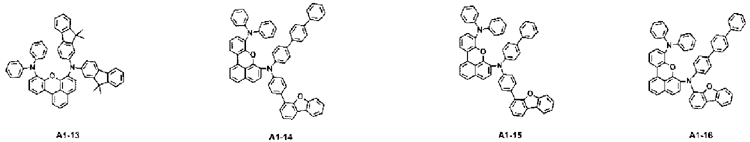

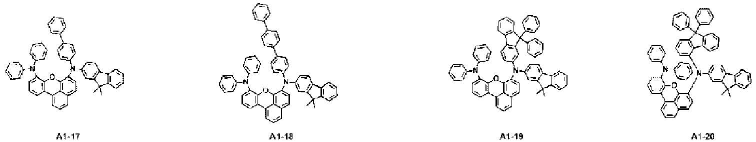

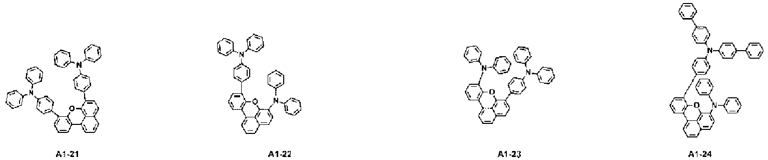

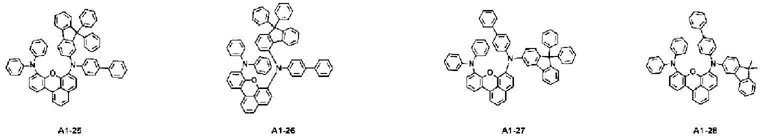

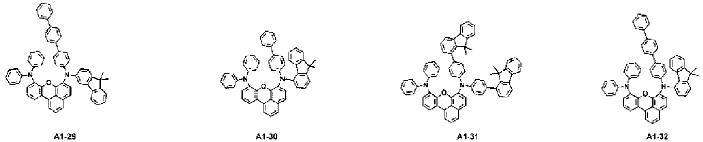

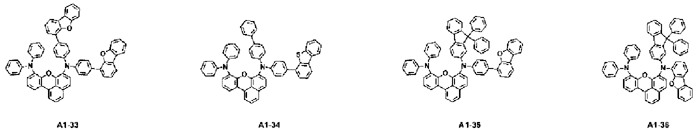

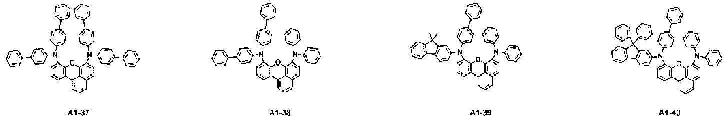

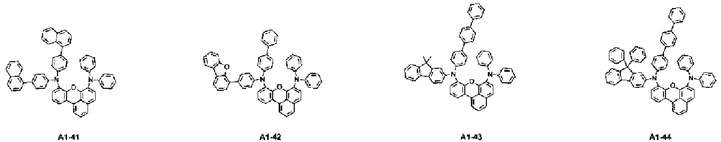

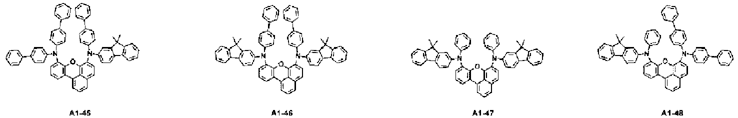

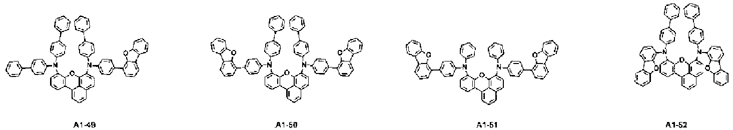

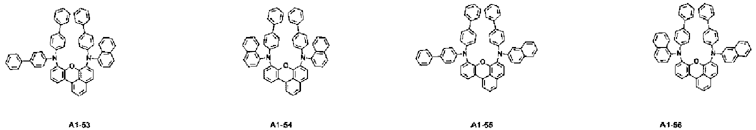

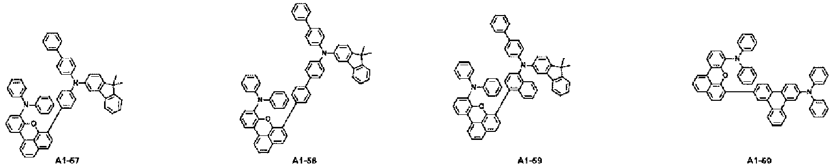

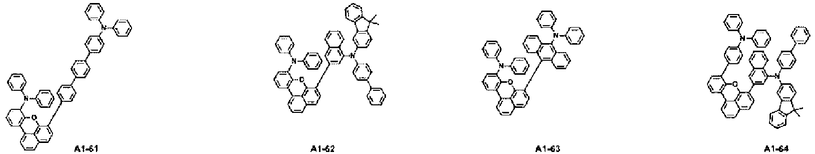

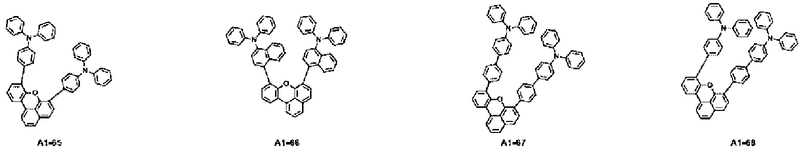

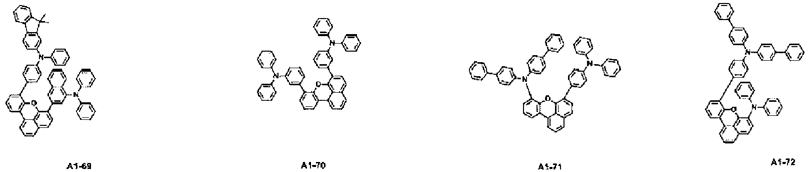

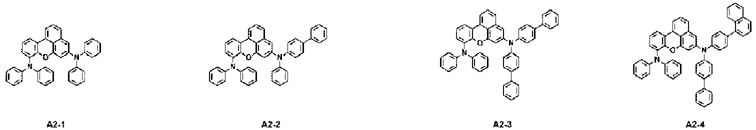

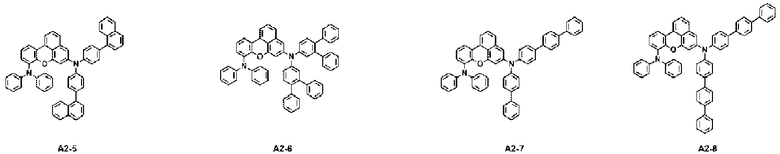

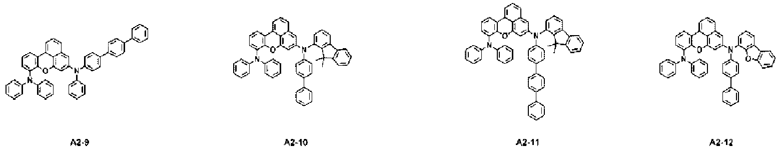

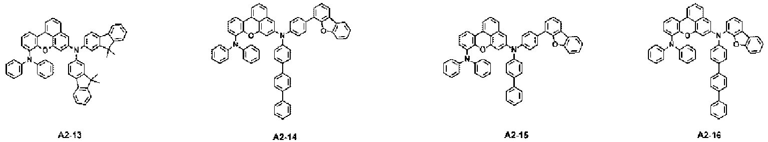

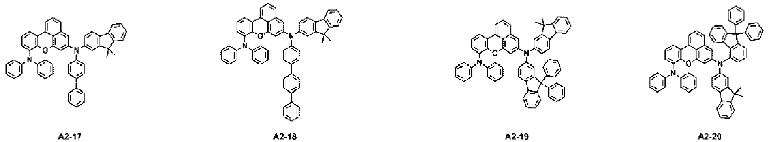

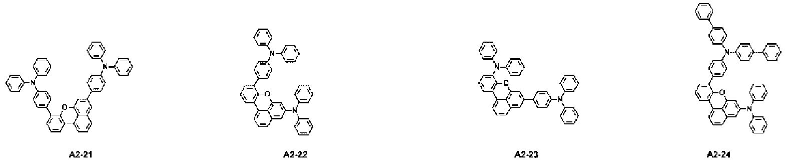

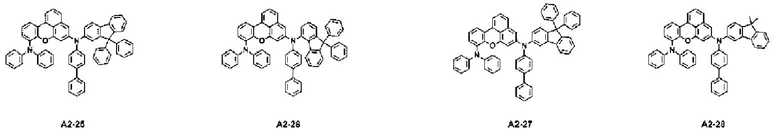

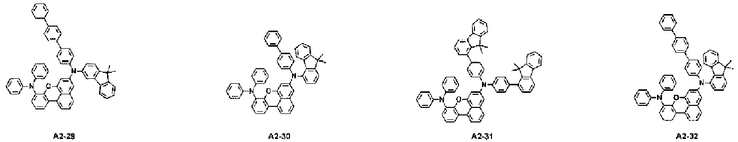

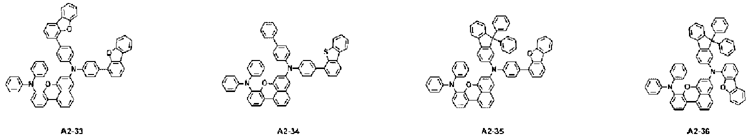

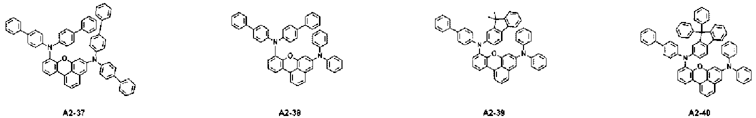

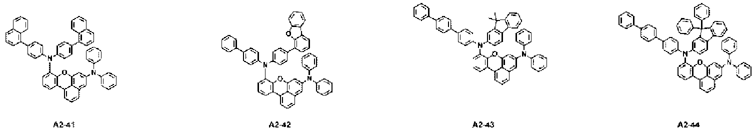

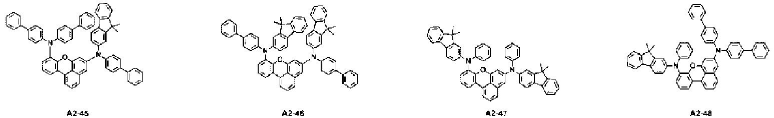

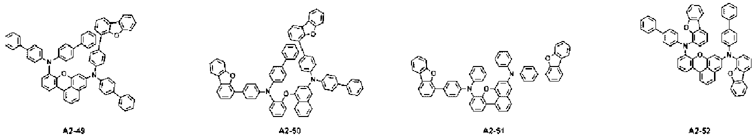

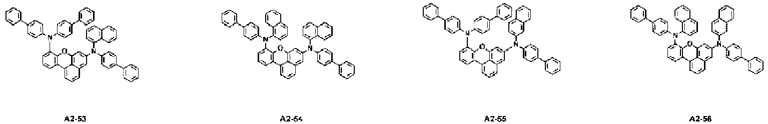

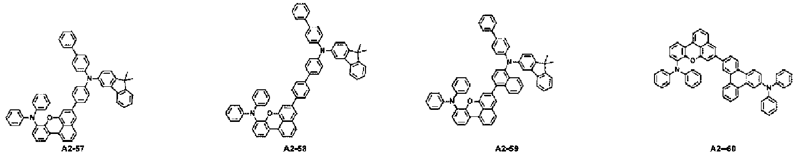

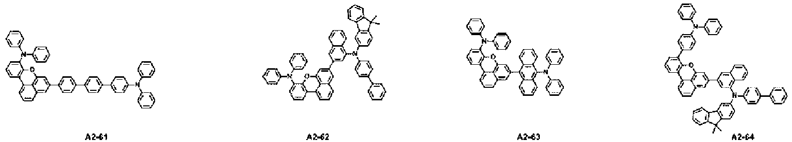

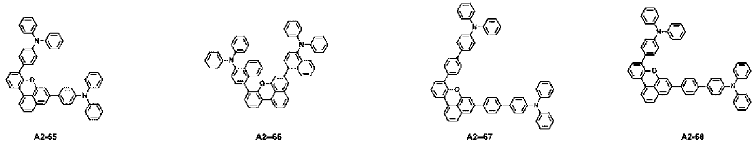

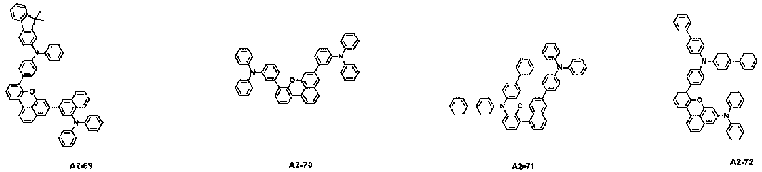

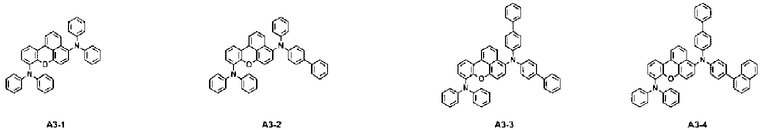

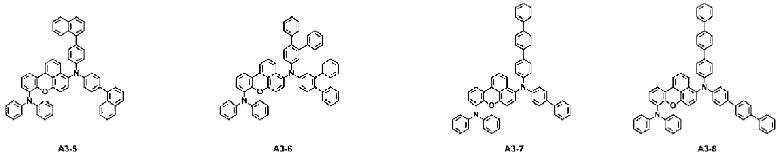

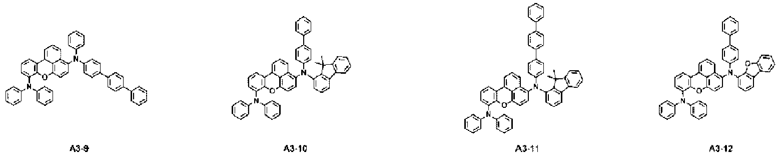

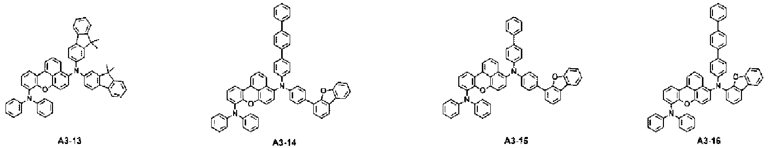

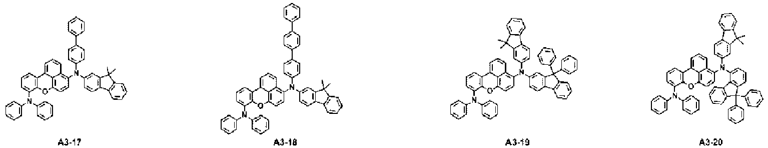

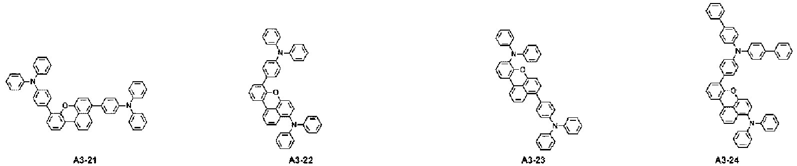

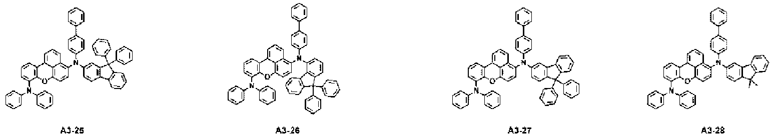

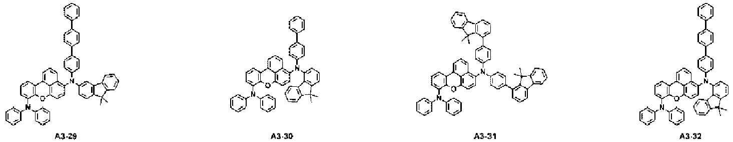

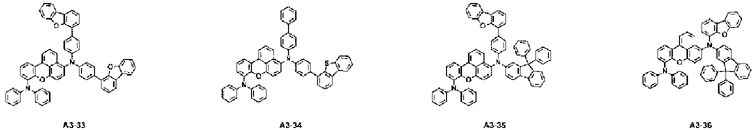

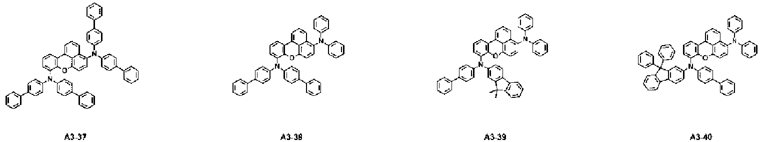

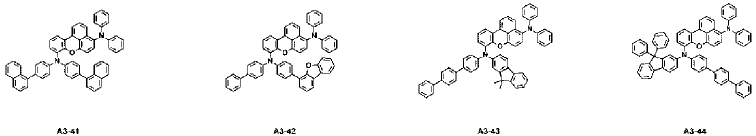

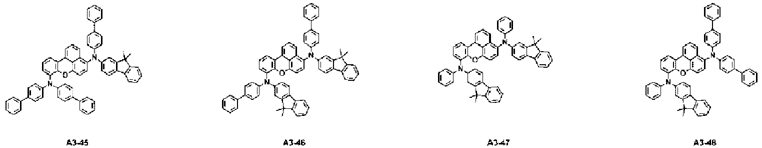

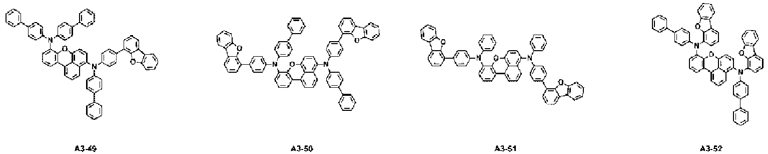

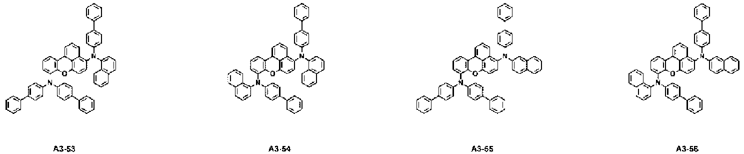

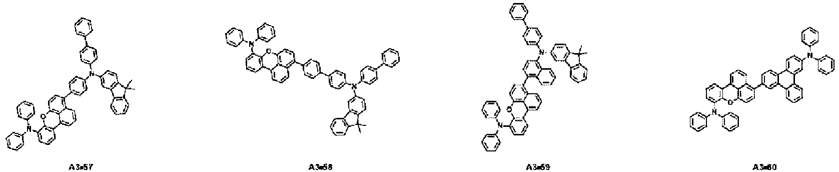

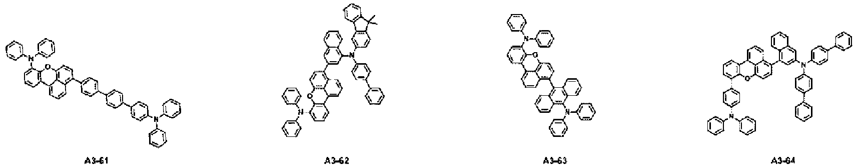

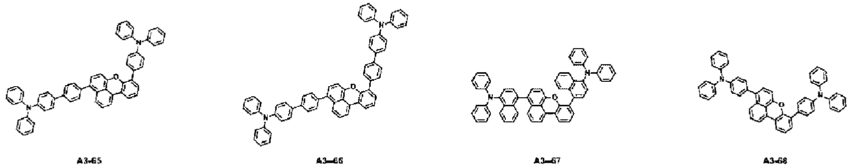

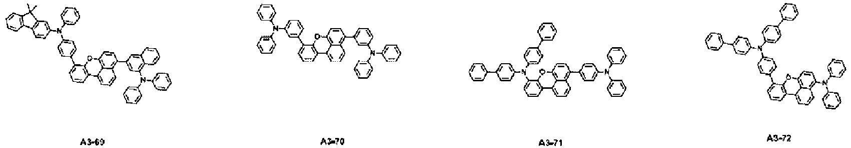

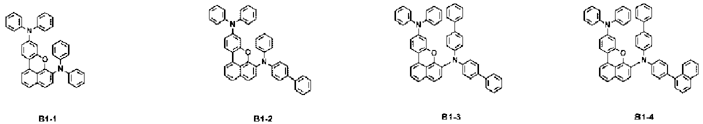

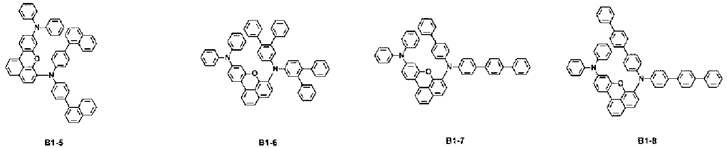

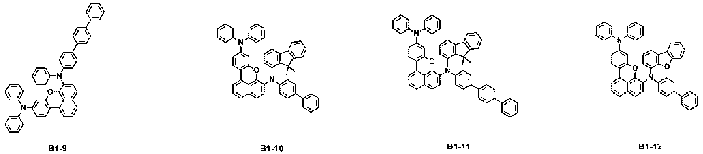

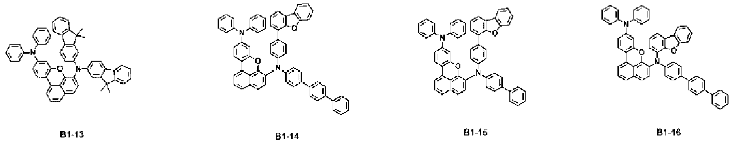

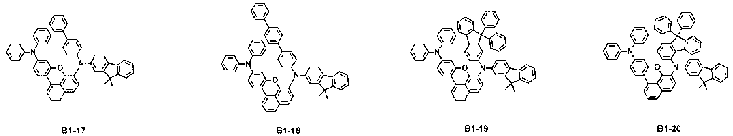

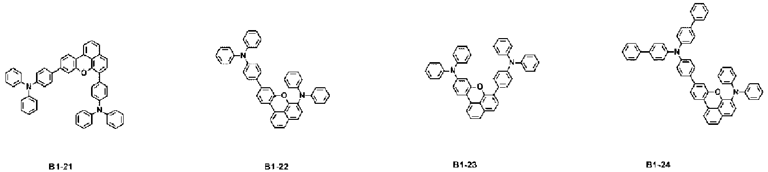

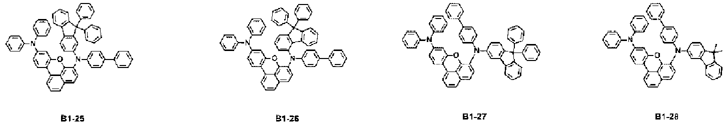

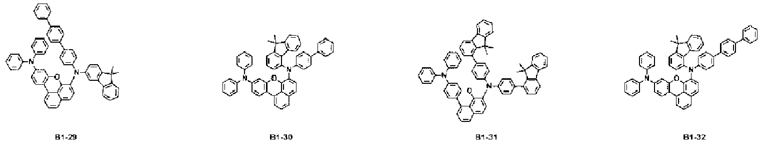

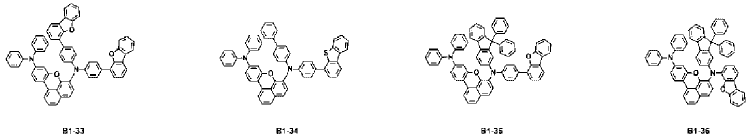

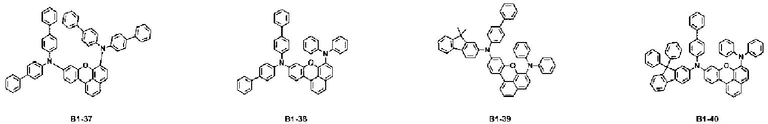

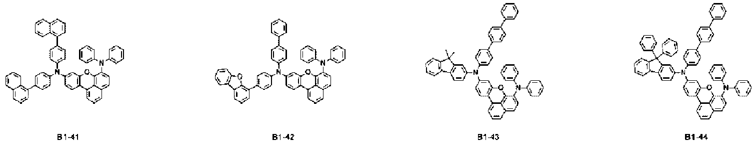

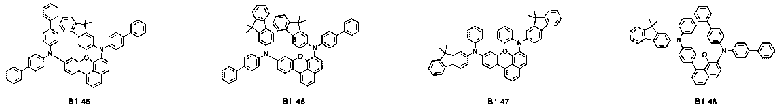

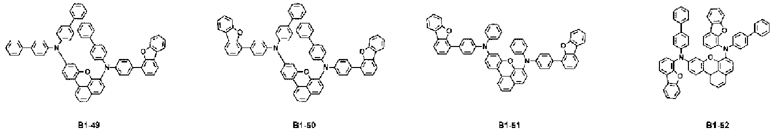

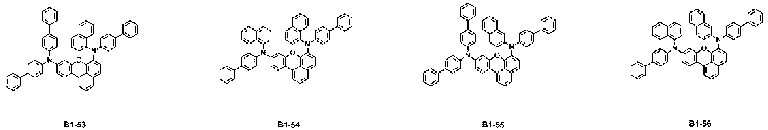

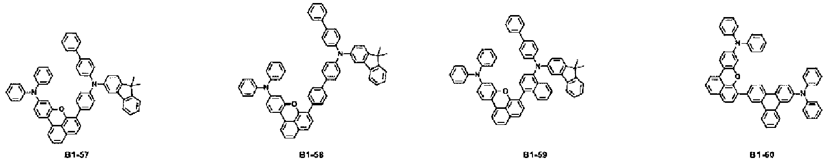

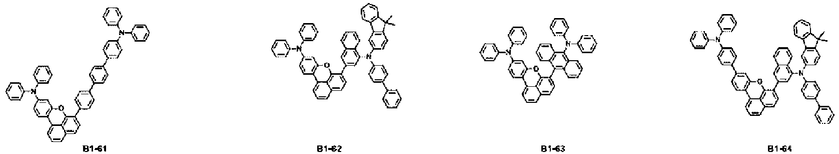

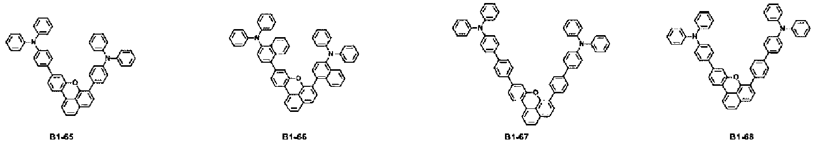

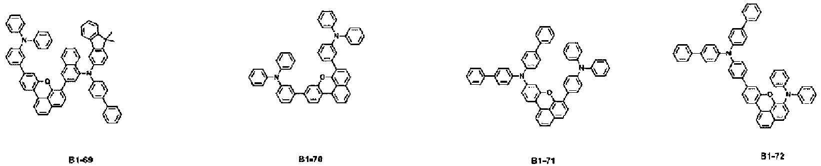

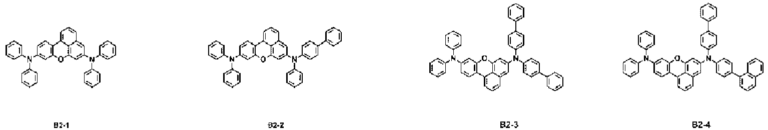

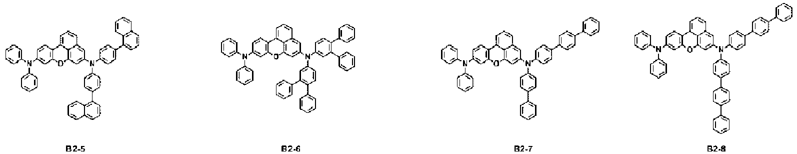

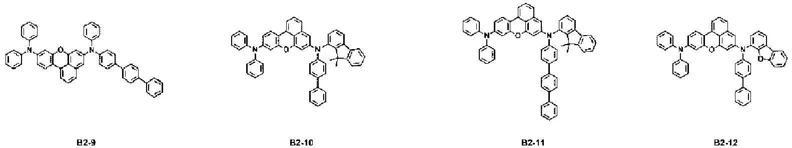

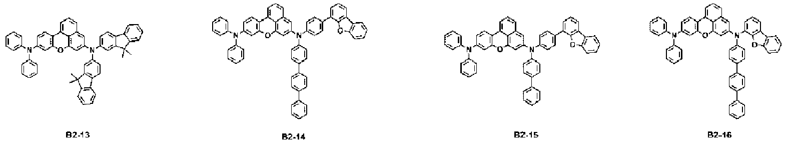

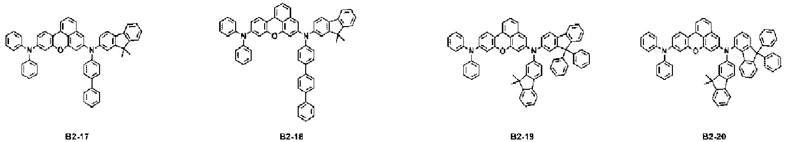

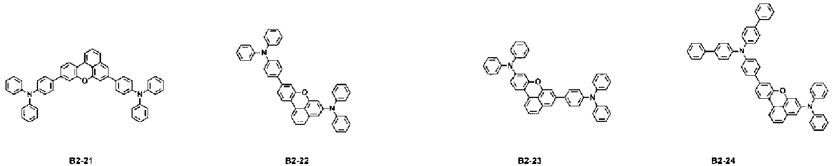

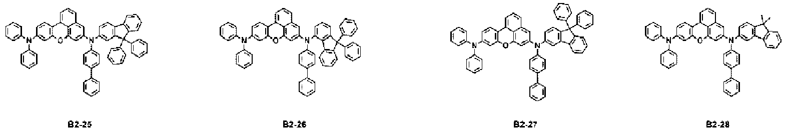

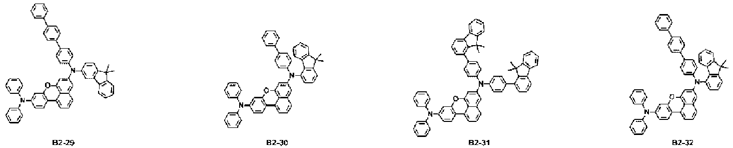

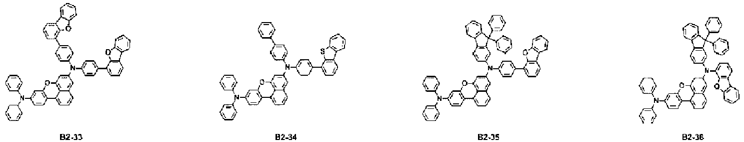

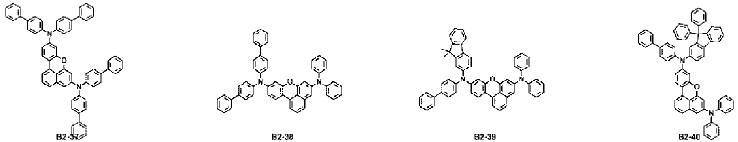

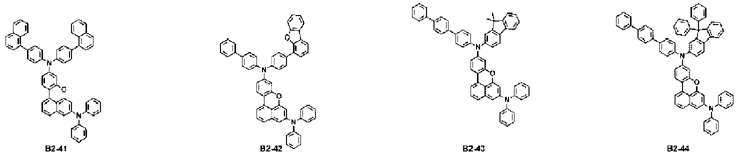

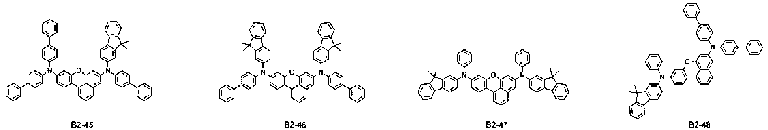

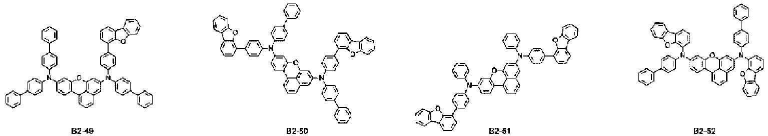

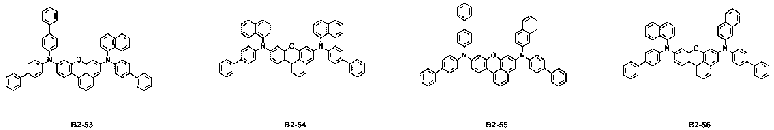

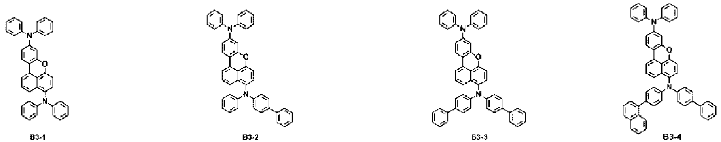

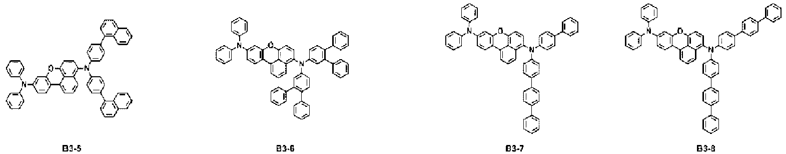

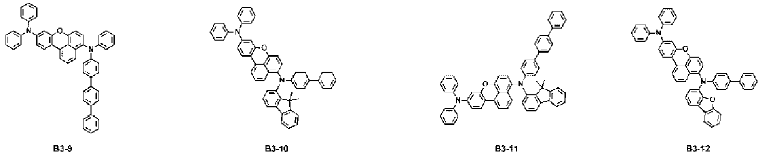

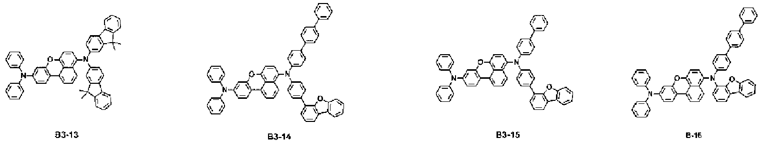

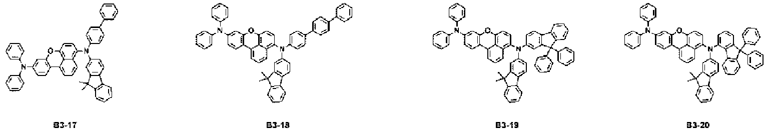

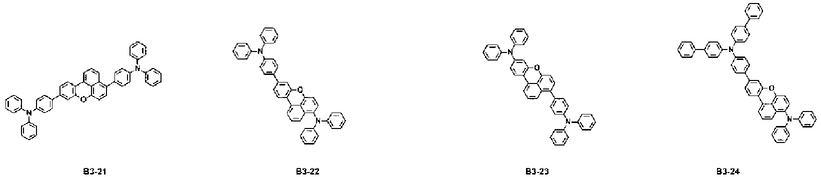

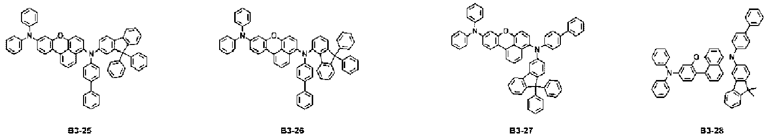

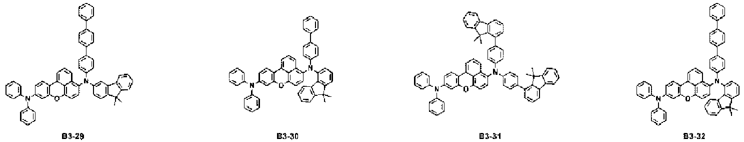

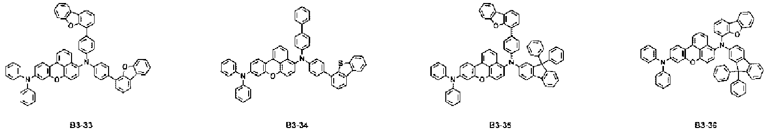

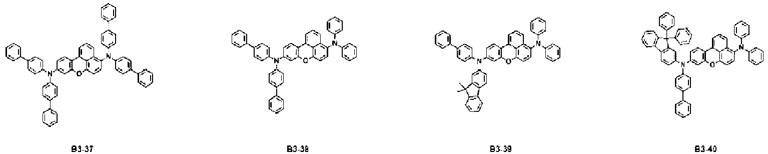

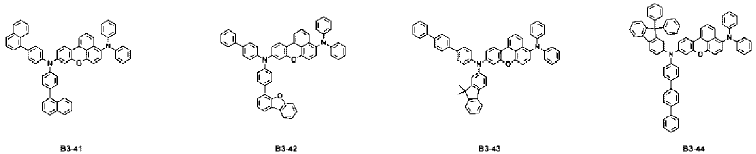

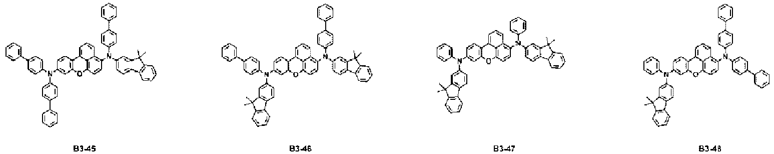

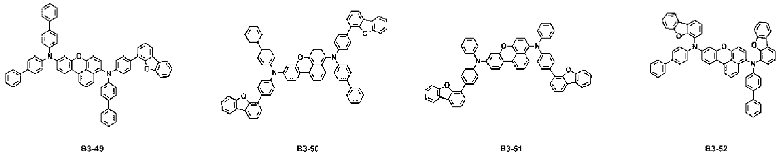

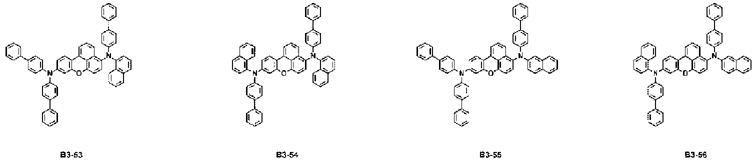

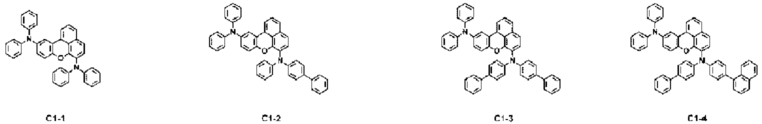

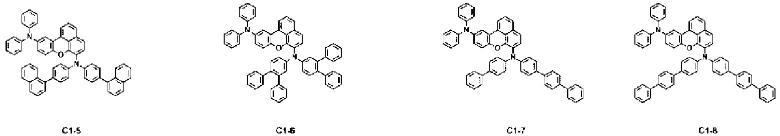

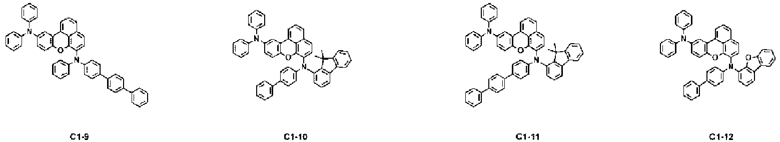

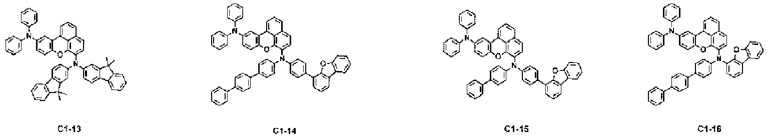

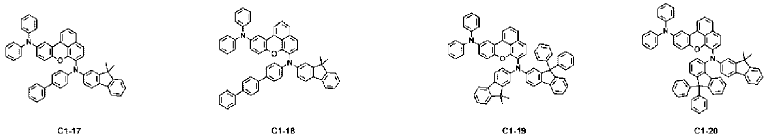

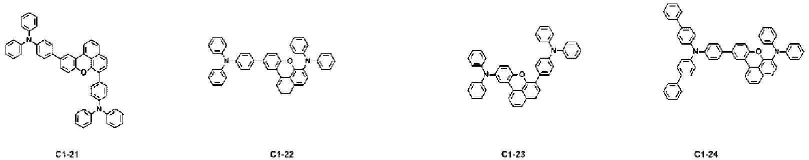

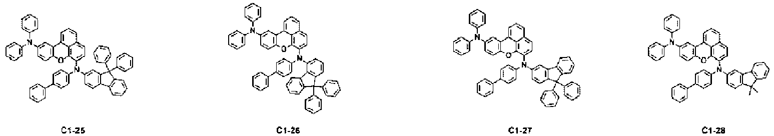

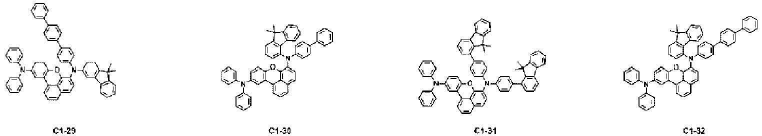

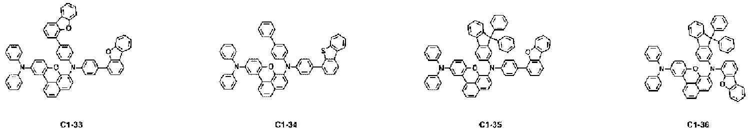

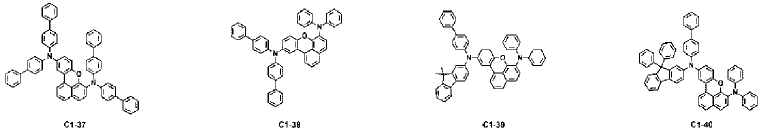

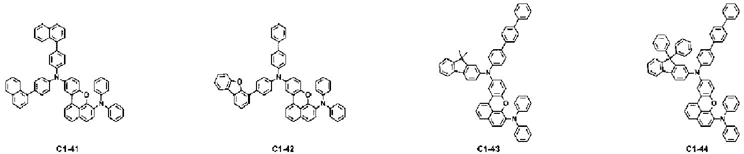

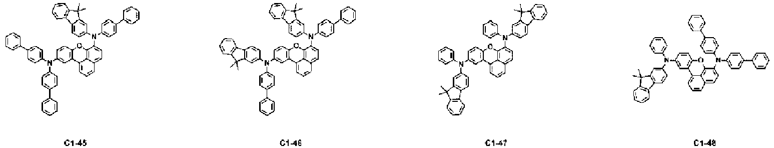

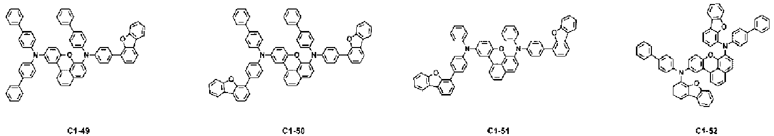

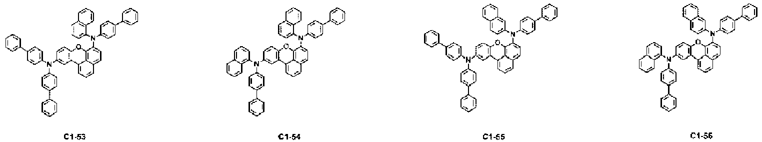

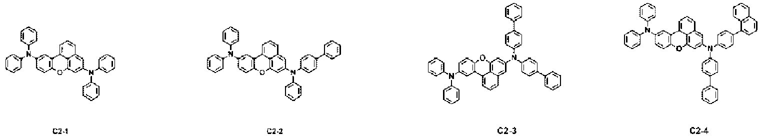

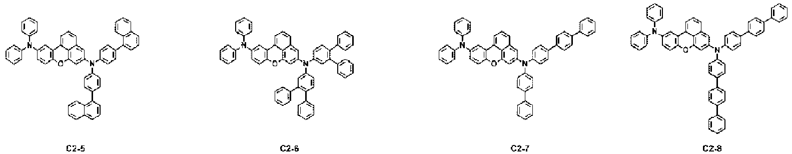

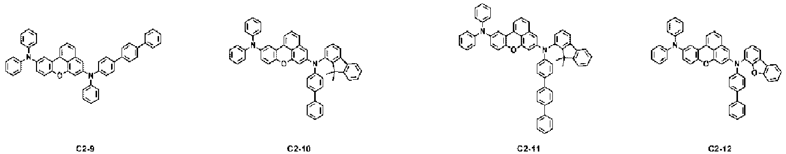

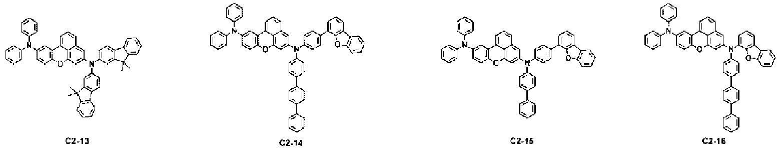

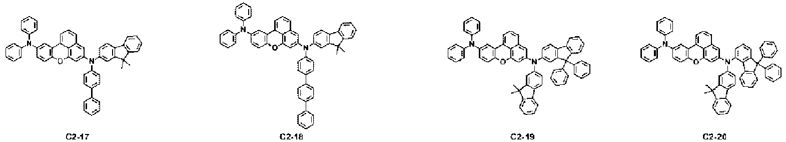

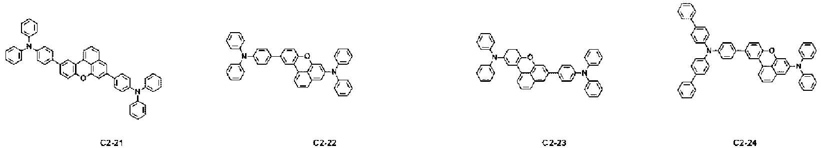

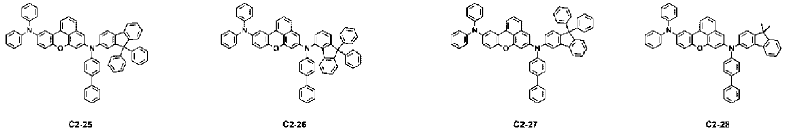

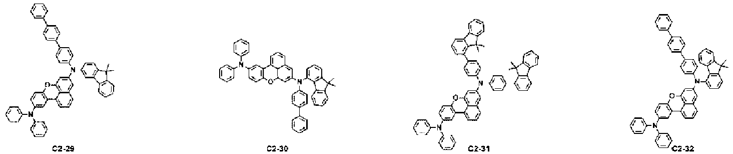

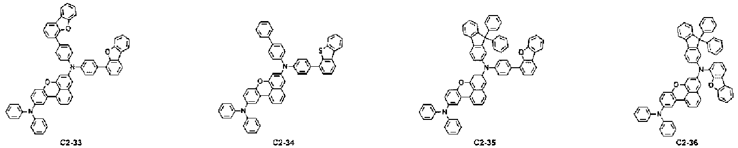

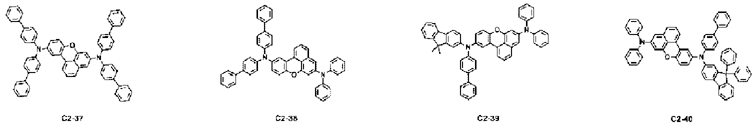

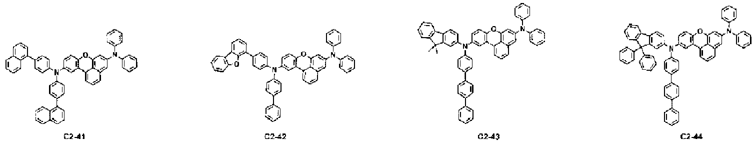

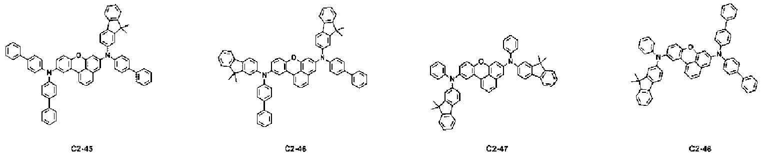

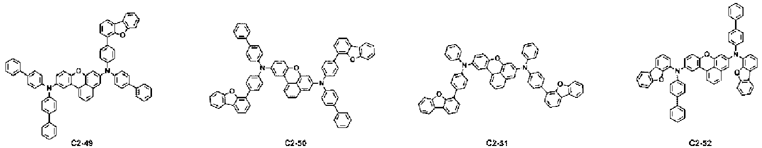

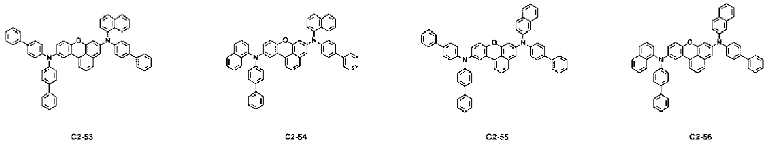

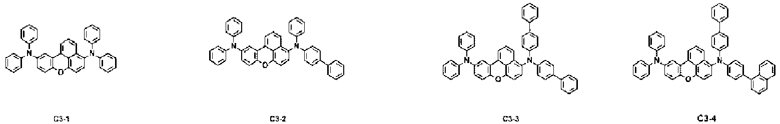

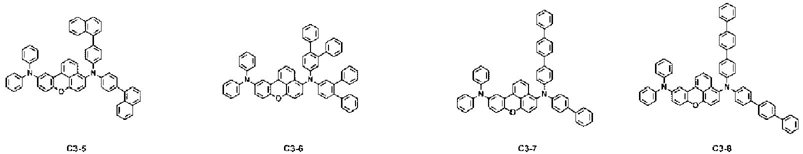

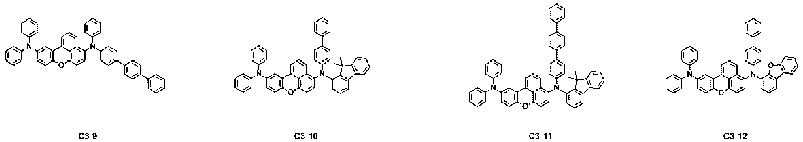

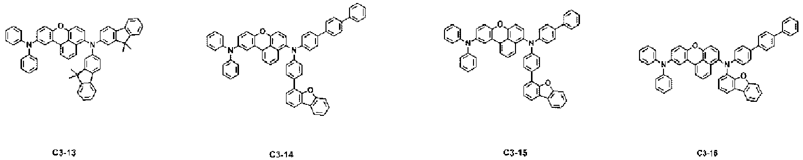

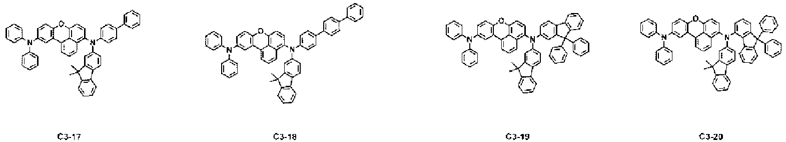

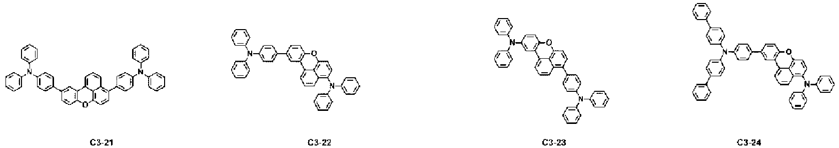

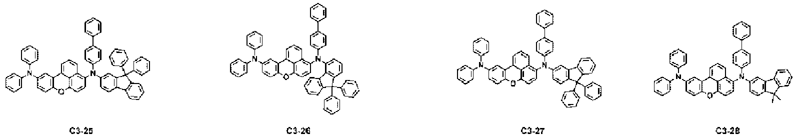

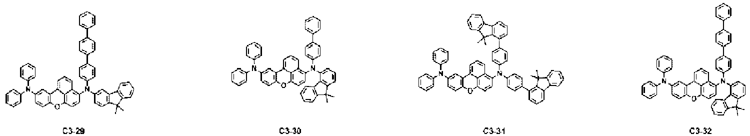

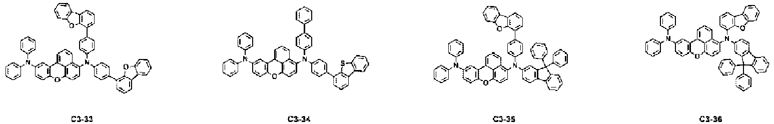

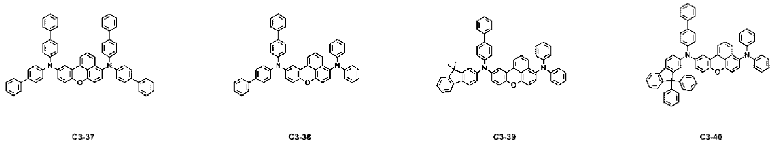

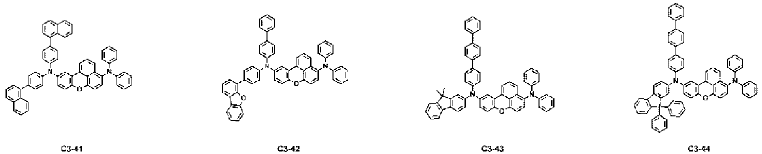

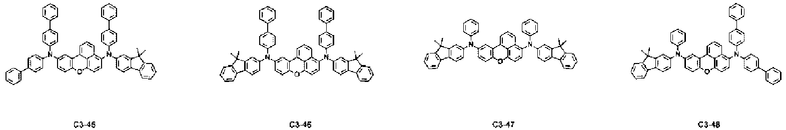

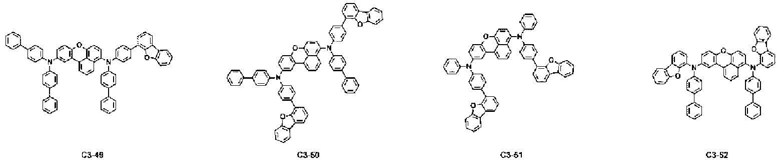

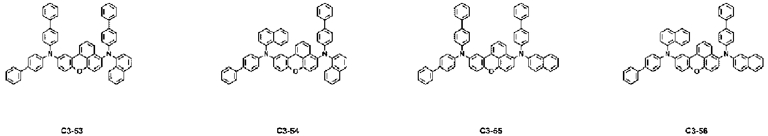

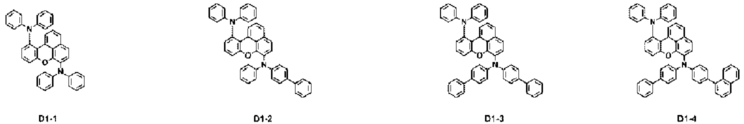

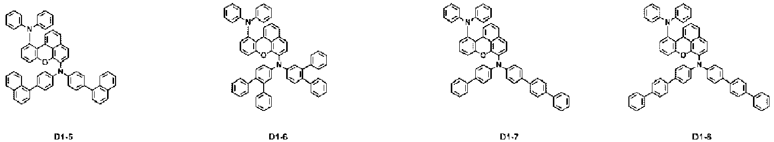

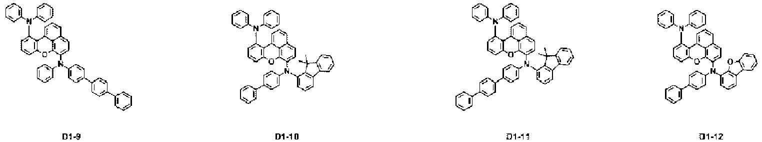

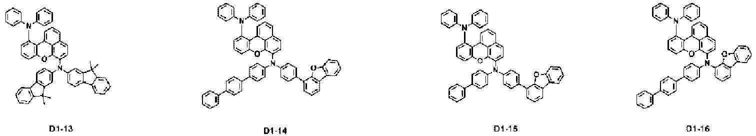

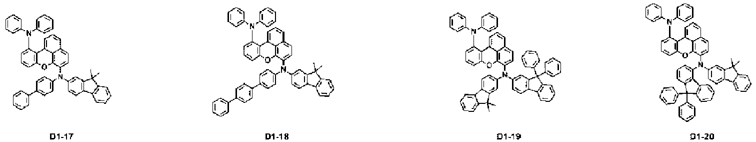

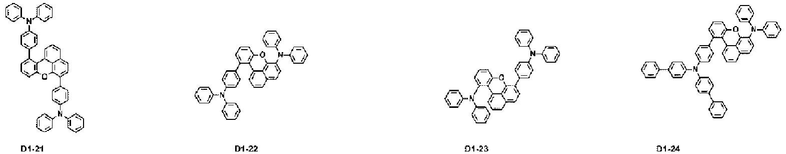

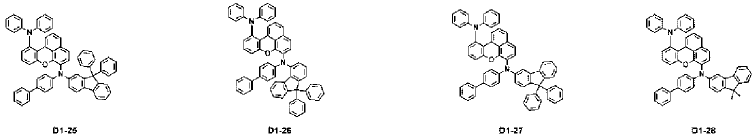

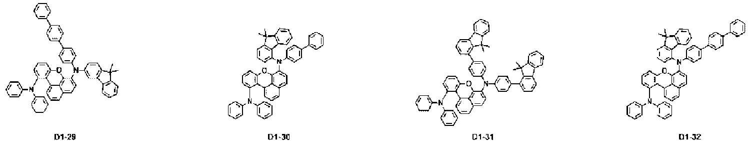

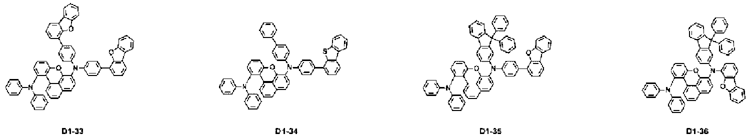

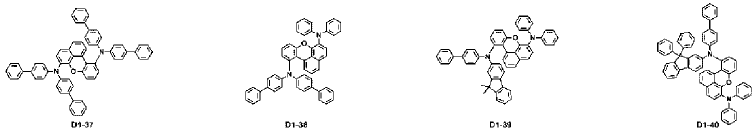

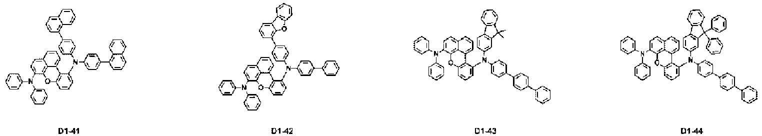

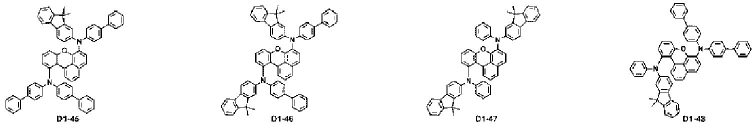

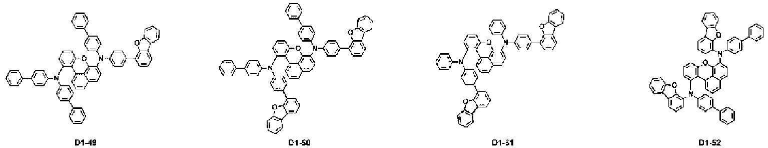

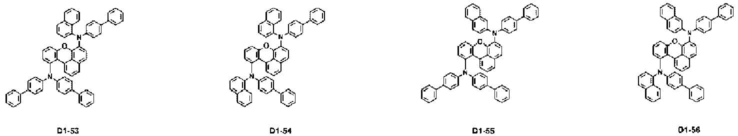

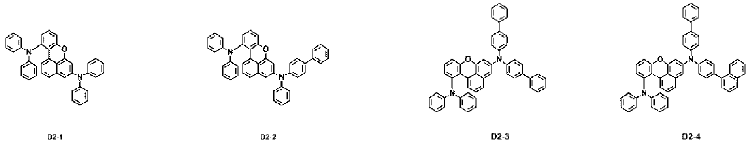

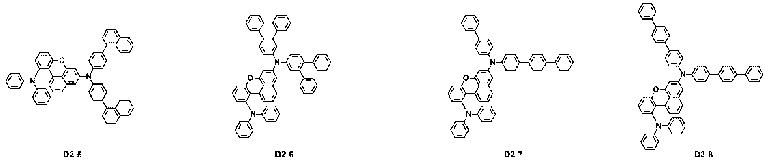

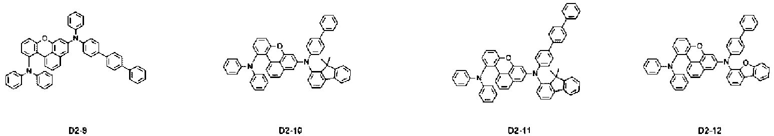

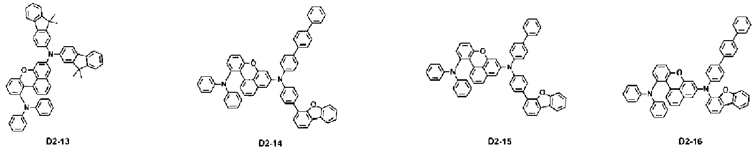

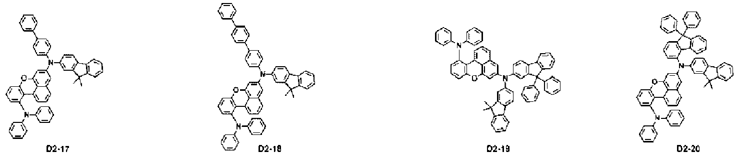

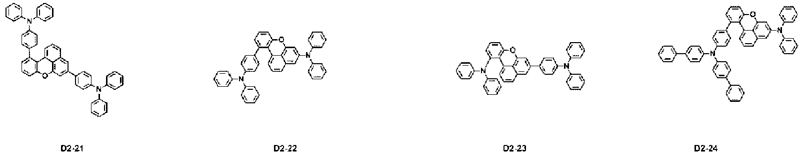

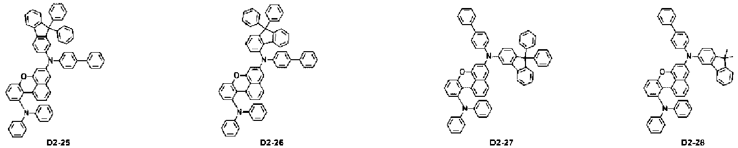

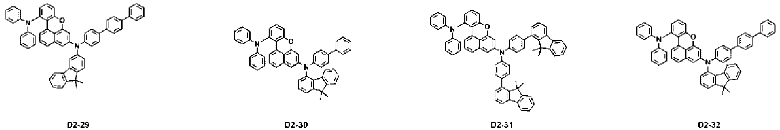

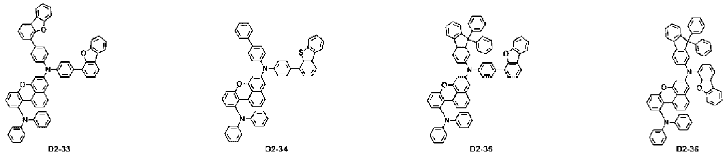

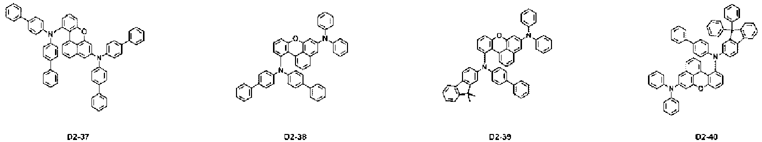

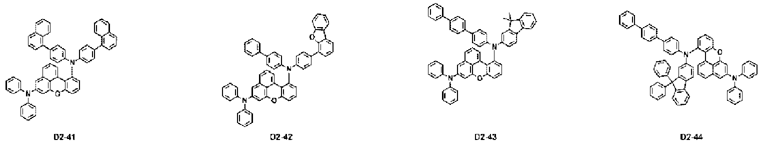

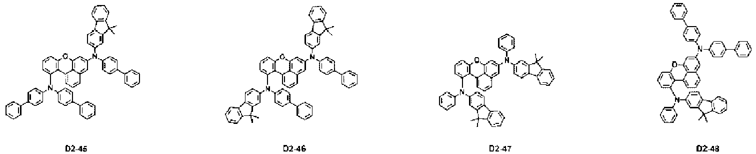

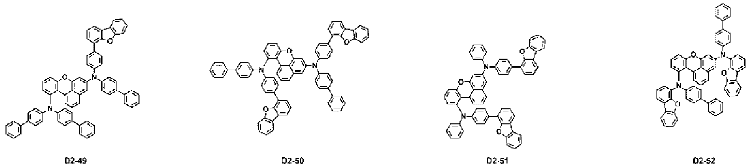

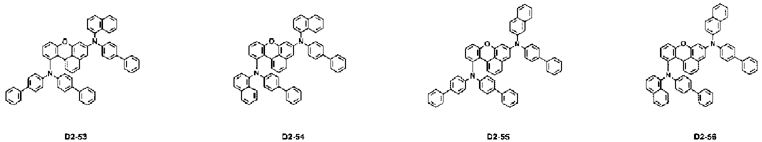

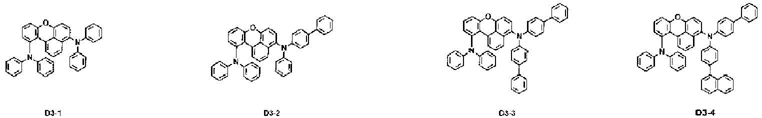

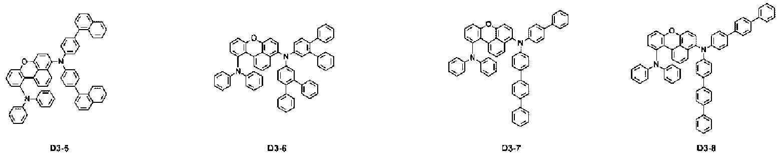

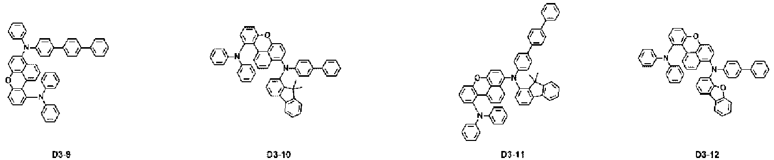

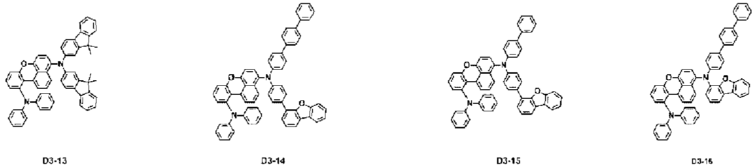

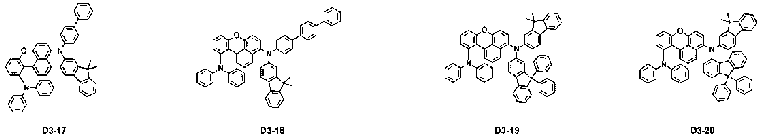

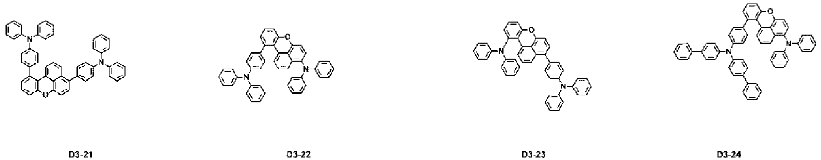

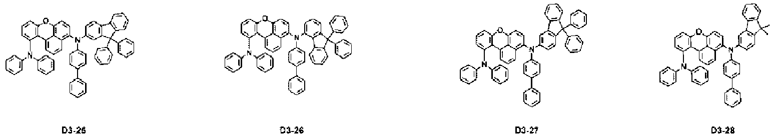

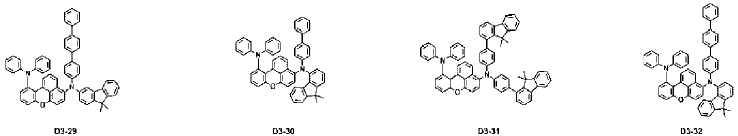

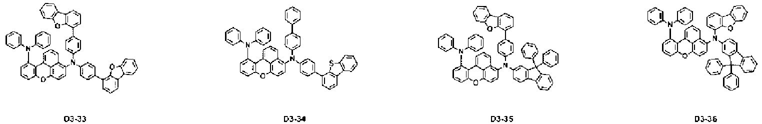

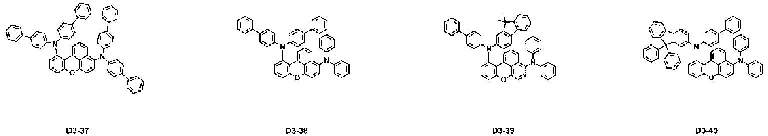

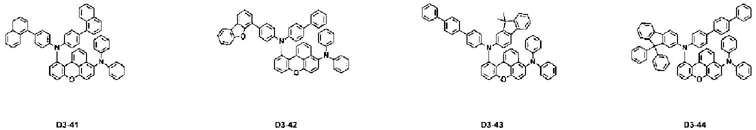

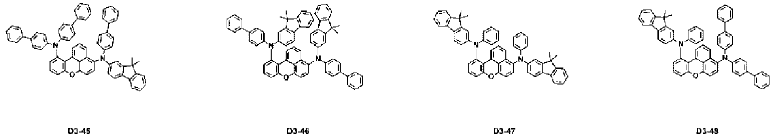

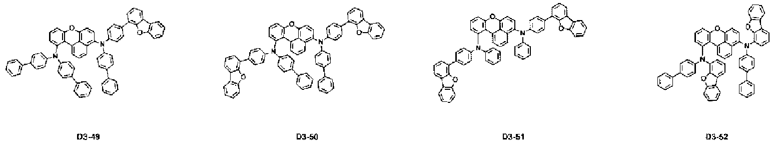

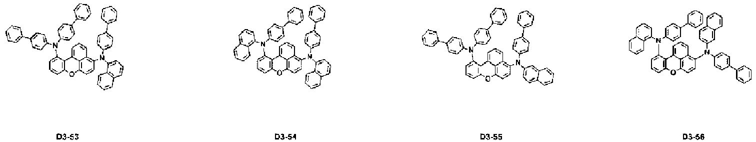

In addition, by introducing various substituents to the structure of Chemical Formula 1, compounds having unique properties of the introduced substituents may be synthesized. For example, by introducing substituents normally used as hole injection layer materials, hole transfer layer materials, light emitting layer materials, electron transfer layer materials and charge generation layer materials used for manufacturing an organic light emitting device to the core structure, materials satisfying conditions required for each organic material layer may be synthesized.

In addition, by introducing various substituents to the structure of Chemical Formula 1, the energy band gap may be finely controlled, and meanwhile, properties at interfaces between organic materials are enhanced, and material applications may become diverse.

One embodiment of the present specification provides an organic light emitting device comprising a first electrode; a second electrode; and one or more organic material layers provided between the first electrode and the second electrode, wherein one or more layers of the organic material layers comprise one or more types of the heterocyclic compound represented by Chemical Formula 1.

In one embodiment of the present specification, one or more layers of the organic material layers comprise one type of the heterocyclic compound represented by Chemical Formula 1.

In one embodiment of the present specification, the first electrode may be an anode, and the second electrode may be a cathode.

In another embodiment of the present specification, the first electrode may be a cathode, and the second electrode may be an anode.

In one embodiment of the present specification, the organic light emitting device may be a blue organic light emitting device, and the heterocyclic compound represented by Chemical Formula 1 may be used as a material of the blue organic light emitting device. For example, the heterocyclic compound represented by Chemical Formula 1 may be included in an electron transfer layer or a hole blocking layer of the blue organic light emitting device.

In one embodiment of the present specification, the organic light emitting device may be a green organic light emitting device, and the heterocyclic compound represented by Chemical Formula 1 may be used as a material of the green organic light emitting device. For example, the heterocyclic compound represented by Chemical Formula 1 may be included in an electron transfer layer or a hole blocking layer of the green organic light emitting device.

In one embodiment of the present specification, the organic light emitting device may be a red organic light emitting device, and the heterocyclic compound represented by Chemical Formula 1 may be used as a material of the red organic light emitting device. For example, the heterocyclic compound represented by Chemical Formula 1 may be included in an electron transfer layer or a hole blocking layer of the red organic light emitting device.

The organic light emitting device of the present specification may be manufactured using common organic light emitting device manufacturing methods and materials except that one or more organic material layers are formed using the compound described above.

The compound may be formed into an organic material layer using a solution coating method as well as a vacuum deposition method when manufacturing the organic light emitting device. Herein, the solution coating method means spin coating, dip coating, inkjet printing, screen printing, a spray method, roll coating and the like, but is not limited thereto.

The organic material layer of the organic light emitting device of the present specification may be formed in a single layer structure, but may be formed in a multilayer structure in which two or more organic material layers are laminated. For example, the organic light emitting device of the present disclosure may have a structure comprising a hole injection layer, a hole transfer layer, a light emitting layer, an electron transfer layer, an electron injection layer and the like as the organic material layer. However, the structure of the organic light emitting device is not limited thereto, and may comprise a smaller number of organic material layers.

In the organic light emitting device of the present specification, the organic material layer comprises a hole transfer layer, and the hole transfer layer may comprise the heterocyclic compound represented by Chemical Formula 1.

In the organic light emitting device of the present specification, the organic material layer comprises an electron blocking layer, and the electron blocking layer may comprise the heterocyclic compound represented by Chemical Formula 1.

The organic light emitting device of the present disclosure may further comprise one, two or more layers selected from the group consisting of a light emitting layer, a hole injection layer, a hole transfer layer, an electron injection layer, an electron transfer layer, an electron blocking layer and a hole blocking layer.

FIG. 1 to FIG. 4 illustrate a lamination order of electrodes and organic material layers of the organic light emitting device according to one embodiment of the present specification. However, the scope of the present application is not limited to these diagrams, and structures of organic light emitting devices known in the art may also be used in the present application.

FIG. 1 illustrates an organic light emitting device in which an anode (200), an organic material layer (300) and a cathode (400) are consecutively laminated on a substrate (100). However, the structure is not limited to such a structure, and as illustrated in FIG. 2, an organic light emitting device in which a cathode, an organic material layer and an anode are consecutively laminated on a substrate may also be obtained.

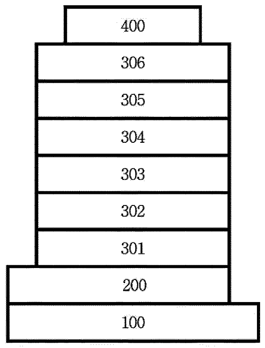

FIG. 3 and FIG. 4 illustrate cases of the organic material layer being a multilayer. The organic light emitting device according to FIG. 3 includes a hole injection layer (301), a hole transfer layer (302), a light emitting layer (304), an electron transfer layer (305) and an electron injection layer (306), and the organic light emitting device according to FIG. 4 includes a hole injection layer (301), a hole transfer layer (302), an electron blocking layer (303), a light emitting layer (304), an electron transfer layer (305) and an electron injection layer (306). However, the scope of the present application is not limited to such a lamination structure, and as necessary, layers other than the light emitting layer may not be included, and other necessary functional layers may be further added.

The organic material layer comprising the heterocyclic compound represented by Chemical Formula 1 may further comprise other materials as necessary.

In the organic light emitting device according to one embodiment of the present specification, materials other than the heterocyclic compound represented by Chemical Formula 1 are illustrated below, however, these are for illustrative purposes only and not for limiting the scope of the present application, and may be replaced by materials known in the art.

As the anode material, materials having relatively large work function may be used, and transparent conductive oxides, metals, conductive polymers or the like may be used. Specific examples of the anode material include metals such as vanadium, chromium, copper, zinc and gold, or alloys thereof; metal oxides such as zinc oxide, indium oxide, indium tin oxide (ITO) and indium zinc oxide (IZO); combinations of metals and oxides such as ZnO:Al or SnO₂:Sb; conductive polymers such as poly(3-methylthiophene), poly[3,4-(ethylene-1,2-dioxy)thiophene] (PEDOT), polypyrrole and polyaniline, and the like, but are not limited thereto.

As the cathode material, materials having relatively small work function may be used, and metals, metal oxides, conductive polymers or the like may be used. Specific examples of the cathode material include metals such as magnesium, calcium, sodium, potassium, titanium, indium, yttrium, lithium, gadolinium, aluminum, silver, tin and lead, or alloys thereof; multilayer structure materials such as LiF/Al or LiO₂/Al, and the like, but are not limited thereto.

As the hole injection material, known hole injection materials may be used, and for example, phthalocyanine compounds such as copper phthalocyanine disclosed in US Patent No. 4,356,429, or starburst-type amine derivatives such as tris(4-carbazoyl-9-ylphenyl)amine (TCTA), 4,4',4"-tri[phenyl(m-tolyl)amino]triphenylamine (m-MTDATA) or 1,3,5-tris[4-(3-methylphenylphenylamino)phenyl]benzene (m-MTDAPB) described in the literature [Advanced Material, 6, p.677 (1994)], polyaniline/dodecylbenzene sulfonic acid, poly(3,4-ethylenedioxythiophene)/poly(4-styrenesulfonate), polyaniline/camphor sulfonic acid or polyaniline/poly(4-styrenesulfonate) that are conductive polymers having solubility, and the like, may be used.

In one embodiment of the present specification, the hole injection material may be 4,4',4"-tris(N,N-(2-naphthyl)-phenylamino)triphenyl amine (2-TNATA), but is not limited thereto.

As the hole transfer material, pyrazoline derivatives, arylamine-based derivatives, stilbene derivatives, triphenyldiamine derivatives and the like may be used, and low molecular or high molecular materials may also be used.

In one embodiment of the present specification, the the hole transfer material may be an arylamine-based derivative, for example, N,N'-bis(α-naphthyl)-N,N'-diphenyl-4,4'-diamine (NPB), but is not limited thereto.

As the electron transfer material, metal complexes of oxadiazole derivatives, anthraquinodimethane and derivatives thereof, benzoquinone and derivatives thereof, naphthoquinone and derivatives thereof, anthraquinone and derivatives thereof, tetracyanoanthraquinodimethane and derivatives thereof, fluorenone derivatives, diphenyldicyanoethylene and derivatives thereof, diphenoquinone derivatives, 8-hydroxyquinoline and derivatives thereof, and the like, may be used, and high molecular materials may also be used as well as low molecular materials.

In one embodiment of the present specification, the electron transfer material may be a benzimidazole derivative, for example, 2-(4-(9,10-di(naphthalen-2-yl)anthracen-2-yl)phenyl)-1-phenyl-1H-benzo[d]imidazole, but is not limited thereto.

As examples of the electron injection material, LiF is typically used in the art, however, the present application is not limited thereto.

As the light emitting material, red, green or blue light emitting materials may be used, and as necessary, two or more light emitting materials may be mixed and used. Herein, two or more light emitting materials may be used by being deposited as individual sources of supply or by being premixed and deposited as one source of supply. In addition, fluorescent materials may also be used as the light emitting material, however, phosphorescent materials may also be used. As the light emitting material, materials emitting light by bonding electrons and holes injected from an anode and a cathode, respectively, may be used alone, however, materials having a host material and a dopant material involving in light emission together may also be used.

When mixing light emitting material hosts, same series hosts may be mixed, or different series hosts may be mixed. For example, any two or more types of materials among n-type host materials or p-type host materials may be selected and used as a host material of a light emitting layer.

The organic light emitting device according to one embodiment of the present specification may be a top-emission type, a bottom-emission type or a dual-emission type depending on the materials used.

The compound according to one embodiment of the present specification may also be used in an organic electronic device comprising an organic solar cell, an organic photo conductor, an organic transistor and the like under a similar principle used in the organic light emitting device.

Hereinafter, the present specification will be described in more detail with reference to examples, however, these are for illustrative purposes only, and the scope of the present application is not limited thereto.

### [Preparation Example] Preparation of Compound A2-2

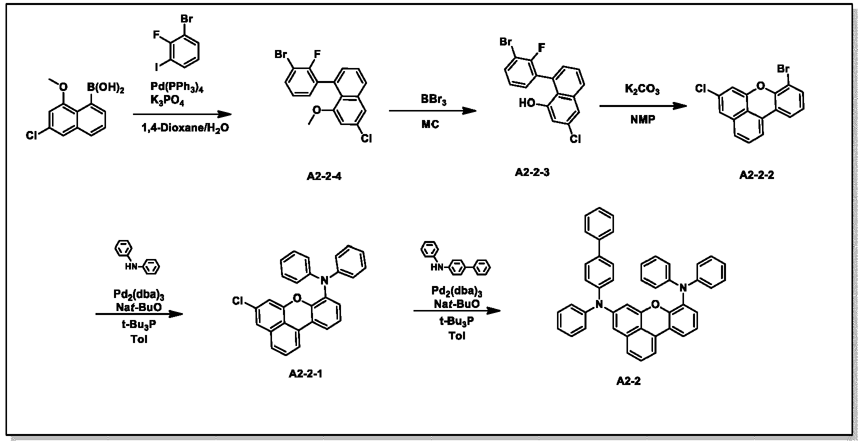

### 1) Preparation of Compound A2-2-4

Toluene (Tol) (750 ml), H₂O and ethanol (EtOH) (150 ml) were introduced to (6-chloro-8-methoxynaphthalen-1-yl)boronic acid (A) (50 g, 0.21 mol, 1.0 eq.), 1-bromo-2-fluoro-3-iodobenzene (B) (70 g, 0.23 mol, 1.1 eq.), Pd(PPh₃)₄ (tetrakis(triphenylphosphine)palladium(0)) (12 g, 0.0105 mol, 0.05 eq.) and K₃PO₄ (89 g, 0.42 mol, 2 eq.), and the mixture was stirred for 5 hours at 110°C. The reaction was terminated by introducing water thereto, and the result was extracted using methylene chloride (MC) and water. After that, moisture was removed with anhydrous MgSO₄. The result was separated using a silica gel column to obtain Compound A2-2-4 (61 g) in a 78% yield.

### 2) Preparation Compound A2-2-3

Compound A2-2-4 (50 g, 0.14 mol, 1 eq.) was introduced to MC (600 ml), and, after substituted with N₂, the mixture was stirred at 0°C. BBr₃ (43 ml, 0.17 mol, 1.2 eq.) was slowly added dropwise thereto, and the result was stirred for 12 hours at RT (room temperature). The reaction was terminated by introducing water thereto, and the result was extracted using MC and water. After that, moisture was removed with anhydrous MgSO₄. The result was separated using a silica gel column to obtain Compound A2-2-3 (42 g) in a 85% yield.

### 3) Preparation of Compound A2-2-2

Compound A2-2-3 (40 g, 0.11 mol, 1 eq.) and K₂CO₃ (48 g, 0.22 mol, 2 eq.) were introduced to N-methyl-2-pyrrolidone (NMP) (600 ml), and the mixture was stirred for 3 hours at 200°C. After the reaction was finished, the temperature was lowered to room temperature, and after terminating the reaction by introducing water thereto, the result was extracted using MC and water. After that, moisture was removed with anhydrous MgSO₄. The result was separated using a silica gel column to obtain Compound A2-2-2 (23 g) in a 63% yield.

### 4) Preparation of Compound A2-2-1

Compound A2-2-2 (20 g, 0.06 mol, 1 eq.), diphenylamine (C) (11.2 g, 0.066 mol, 1.1 eq.), Na*t*-BuO (12 g, 0.12 mol, 2 eq.), Pd₂(dba)₃ (tris(dibenzylideneacetone)dipalladium(0)) (2.7 g, 0.003 mol, 0.05 eq.) and t-Bu₃P (2.7 ml 0.006 mol, 0.1 eq.) were introduced to toluene (200 ml), and the mixture was stirred for 6 hours at 110°C. The reaction was terminated by introducing water thereto, and the result was extracted using MC and water. After that, moisture was removed with anhydrous MgSO₄. The result was separated using a silica gel column to obtain Compound A2-2-1 (18 g) in a 71% yield.

### 5) Preparation of Compound A2-2

Compound A2-2-1 (10 g, 0.023 mol, 1 eq.), N-phenyl-[1,1'-biphenyl]-4-amine (D) (6.44 g, 0.026 mol, 1.1 eq.), Nat-BuO (4.59 g, 0.047 mol, 2 eq.), Pd₂(dba)₃ (1.1 g, 0.001 mol, 0.05 eq.) and t-Bu₃P (1.1 ml, 0.002 mol, 0.1 eq.) were introduced to toluene (100 ml), and the mixture was stirred for 6 hours at 110°C. The reaction was terminated by introducing water thereto, and the result was extracted using MC and water. After that, moisture was removed with anhydrous MgSO₄. The result was separated using a silica gel column to obtain Compound A2-2 (12 g) in a 80% yield.

Compounds were synthesized in the same manner as in the preparation example except that Intermediate A of the following Table 1 was used instead of (6-chloro-8-methoxynaphthalen-1-yl)boronic acid (A), Intermediate B of the following Table 1 was used instead of 1-bromo-2-fluoro-3-iodobenzene (B), Intermediate C of the following Table 1 was used instead of diphenylamine (C), and Intermediate D of the following Table 1 was used instead of N-phenyl-[1,1'-biphenyl]-4-amine (D).

**[Table 1]**

| Compo und No. | Intermediat e A | Intermedi ate B | Intermediate C | Intermediate D | Yie ld |
|---|---|---|---|---|---|
| A1-1 | 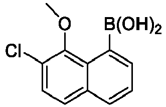 | 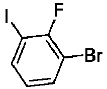 | 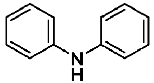 | 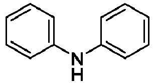 | 80% |
| A1-2 | 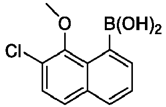 | 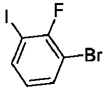 | 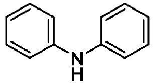 | 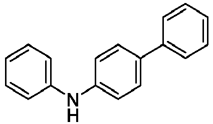 | 77% |
| A1-17 | 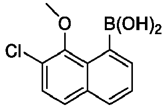 | 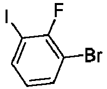 | 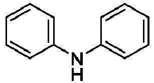 | 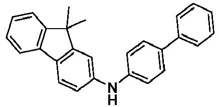 | 69% |
| A1-39 | 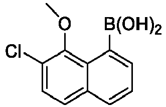 | 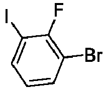 | 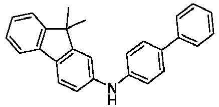 | 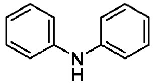 | 71% |
| A2-1 | 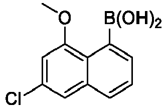 | 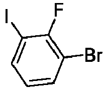 | 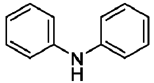 | 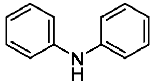 | 72% |
| A2-17 | 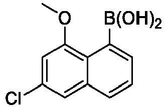 | 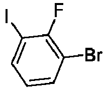 | 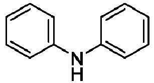 | 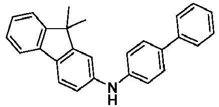 | 70% |
| A2-39 | 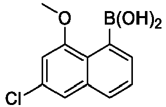 | 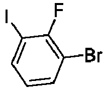 | 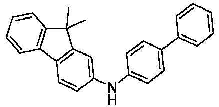 | 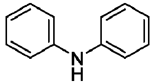 | 65% |
| B1-1 | 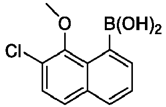 | 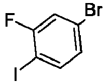 | 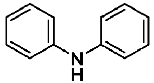 | 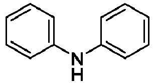 | 85% |
| B1-2 | 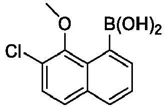 | 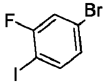 | 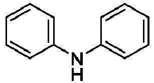 | 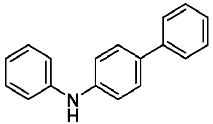 | 85% |
| B1-17 | 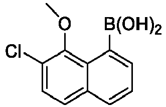 | 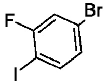 | 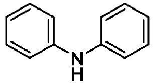 | 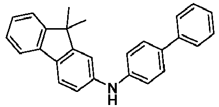 | 71% |
| B1-39 | 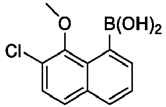 | 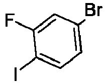 | 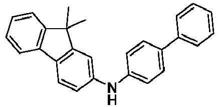 | 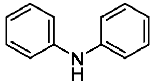 | 78% |
| B2-1 | 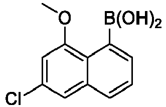 | 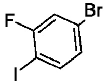 | 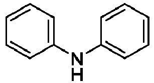 | 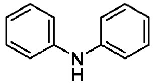 | 80% |
| B2-2 | 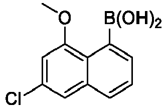 | 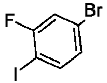 | 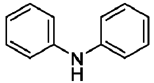 | 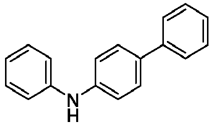 | 76% |
| B2-17 | 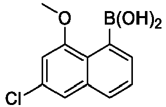 | 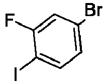 | 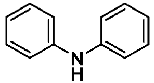 | 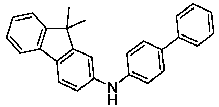 | 70% |
| B2-39 | 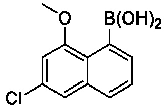 | 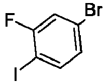 | 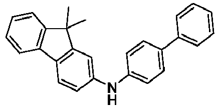 | 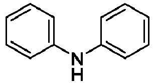 | 72% |
| C1-1 | 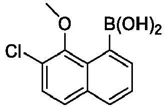 | 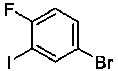 | 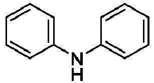 | 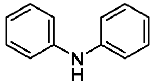 | 66% |
| C1-2 | 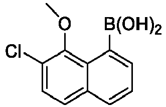 | 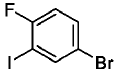 | 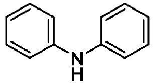 | 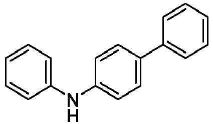 | 65% |
| C1-17 | 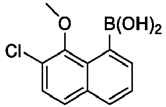 | 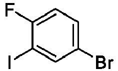 | 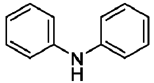 | 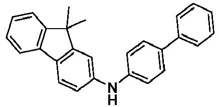 | 71% |
| C1-39 | 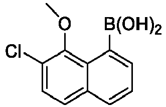 | 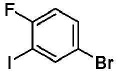 | 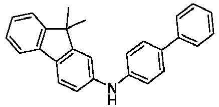 | 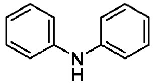 | 77% |
| C2-1 | 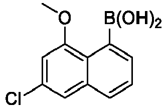 | 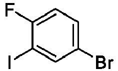 | 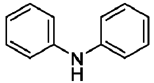 | 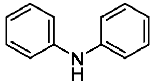 | 69% |
| C2-2 | 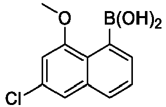 | 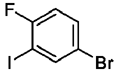 | 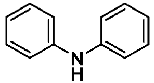 | 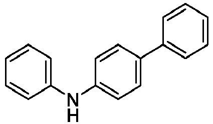 | 65% |
| C2-17 | 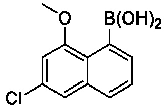 | 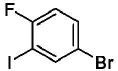 | 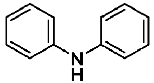 | 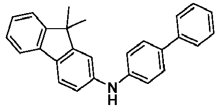 | 85% |
| C2-39 | 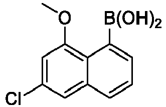 | 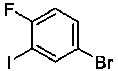 | 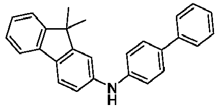 | 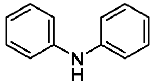 | 70% |
| D1-1 | 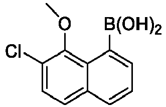 | 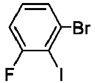 | 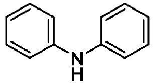 | 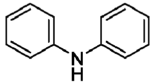 | 80% |
| D1-2 | 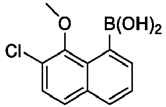 | 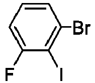 | 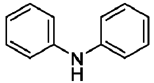 | 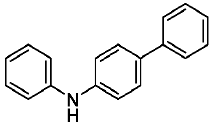 | 82% |
| D1-17 | 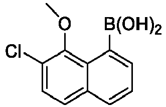 | 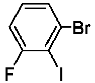 | 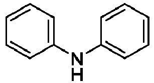 | 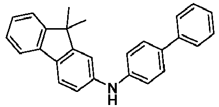 | 85% |
| D1-39 | 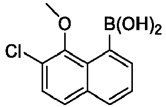 | 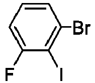 | 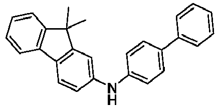 | 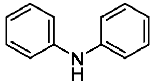 | 67% |
| D2-1 | 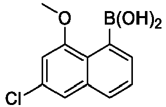 | 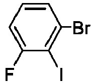 | 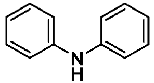 | 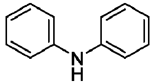 | 69% |
| D2-2 | 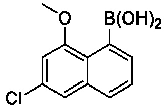 | 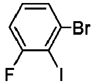 | 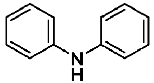 | 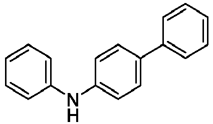 | 65% |
| D2-17 | 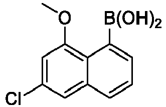 | 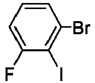 | 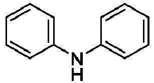 | 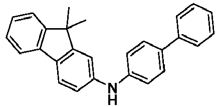 | 85% |
| D2-39 | 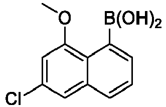 | 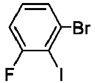 | 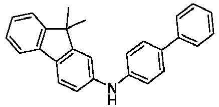 | 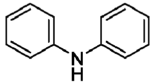 | 77% |

The compounds were prepared in the same manner as in the preparation example, and the synthesis identification results are shown in Table 2 and Table 3. Table 2 shows measurement values of ¹H NMR (CDCl₃, 200 Mz), and Table 3 shows measurement values of FD-mass spectrometry (FD-MS: field desorption mass spectrometry).

**[Table 2]**

| Compou nd | ¹H NMR (CDCl₃, 200 Mz) |
|---|---|
| A1-1 | δ=8.23(1H, d), 7.89(1H, d), 7.78(1H, d), 7.62∼7.57(3H, m), 7.24∼7.27(10H, m), 7.08∼7.00(12H, m) |
| A1-2 | δ=8.23(1H, d), 7.89(1H, d), 7.78∼7.75(3H, m), 7.62∼7.37(10H, m), 7.24∼7.27(8H, m), 7.08∼7.00(9H, m) |
| A1-17 | δ=8.23(1H, d), 7.90∼7.89(2H, m), 7.78∼7.75(3H, m), 7.62∼7.37(14H, m), 7.24∼7.28(7H, m), 7.08∼7.00(7H, m) |
| A1-39 | δ=8.23(1H, d), 7.90∼7.85(3H, m), 7.78∼7.00(22H, m) |
| A2-1 | δ=8.31 (1H, d), 7.97 (1H, d), 7.61~7.54 (2H, m), 7.24∼7.27(10H, m), 7.08∼7.00(12H, m), 6.78(1H, s), 6.66 (1H, d) |
| A2-17 | δ=8.31(1H, d), 7.97(1H, d), 7.90(1H, d), 7.75(2H, m), 7.24∼7.55(20H, m), 7.08∼7.00(7H, m), 6.88(1H, d), 6.66 (1H, d) |
| A2-39 | δ=8.31(1H, d), 7.97(1H, d), 7.90(1H, d), 7.75(2H, m), 7.24∼7.55(20H, m), 7.08∼7.00(7H, m), 6.88(1H, d), 6.66 (1H, d) |
| B1-1 | δ=8.23(1H, d), 7.89(1H, d), 7.78(1H, d), 7.69∼7.57(3H, m), 7.24∼7.28(12H, m), 7.08∼7.00(14H, m) |
| B1-2 | δ=8.23(1H, d), 7.89(1H, d), 7.78∼7.27(13H, m), 7.24 (6H, m), 7.08∼7.00(14H, m) |
| B1-17 | δ=8.23(1H, d), 7.89(1H, d), 7.69-7.38(16H, m), 7.24-7.28(4H, m), 7.08-7.00(9H, m) |
| B1-39 | δ=8.23(1H, d), 7.90-7.89(3H, m), 7.78-7.00(22H, m) |
| B2-1 | δ=8.31 (1H, d), 7.97(1H, d), 7.61~7.54(2H, m), 7.24∼7.27(10H, m), 7.08∼7.00(12H, m), 6.78(1H, s), 6.66 (1H, d) |
| B2-2 | δ=8.31 (1H, d), 7.97(1H, d), 7.75∼7.69(3H, m), 7.55∼7.49(8H, m), 7.24 (4H, m) 7.08∼7.00(11H, m), 6.78(1H, s), 6.66 (1H, d) |
| B2-17 | δ=8.31(1H, d), 7.97(1H, d), 7.90(1H, d), 7.75(2H, m), 7.24∼7.55(20H, m), 7.08∼7.00(7H, m), 6.88(1H, d), 6.66 (1H, d) |
| B2-39 | δ=8.31(1H, d), 7.97(1H, d), 7.90(1H, d), 7.75(2H, m), 7.24∼7.55(20H, m), 7.08∼7.00(7H, m), 6.88(1H, d), 6.66 (1H, d) |
| C1-1 | δ=8.23(1H, d), 7.89(1H, d), 7.78(1H, d), 7.69∼7.57(3H, m), 7.24∼7.28(12H, m), 7.08∼7.00(14H, m) |
| C1-2 | δ=8.23(1H, d), 7.89(1H, d), 7.78∼7.26(13H, m), 7.24 (6H, m), 7.06∼7.00(14H, m) |
| C1-17 | δ=8.23(1H, d), 7.89(1H, d), 7.65∼7.38(16H, m), 7.29∼7.24(4H, m), 7.08∼7.00(9H, m) |
| C1-39 | δ=8.23(1H, d), 7.90∼7.89(3H, m), 7.78∼7.00(22H, m) |
| C2-1 | δ=8.31 (1H, d), 7.97 (1H, d), 7.61~7.54(2H, m), 7.24∼7.27(10H, m), 7.08∼7.00(12H, m), 6.78(1H, s), 6.66 (1H, d) |
| C2-2 | δ=8.31 (1H, d), 7.97(1H, d), 7.75∼7.69(3H, m), 7.55∼7.49(8H, m), 7.24 (4H, m) 7.08∼7.00(11H, m), 6.78(1H, s), 6.66 (1H, d) |
| C2-17 | δ=8.31 (1H, d), 7.97(1H, d), 7.78(1H, d), 7.74∼7.75 (2H, m), 7.24∼7.55(20H, m), 7.08∼7.00(7H, m), 6.78(1H, d), 6.66 (1H, d) |
| C2-39 | δ=8.31(1H, d), 7.97(1H, d), 7.79(1H, d), 7.75(2H, m), 7.24∼7.55(20H, m), 7.08∼7.00(7H, m), 6.88(1H, d), 6.66 (1H, d) |
| D1-1 | δ=8.23(1H, d), 7.89(1H, d), 7.78(1H, d), 7.69∼7.57(2H, m), 7.24∼7.28(9H, m), 7.08∼7.00(13H, m), 6.85(1H, d) |
| D1-2 | δ=8.23(1H, d), 7.89(1H, d), 7.78∼7.75(3H, m), 7.24∼7.55(9H, m), 7.08∼7.00(10H, m), 6.85(1H, d) |
| D1-17 | δ=8.23(1H, d), 7.89(1H, d), 7.78∼7.75(2H, q), 7.55∼7.37(13H, m), 7.24∼7.28(6H, m), 7.08∼7.00(8H, m), 6.85(1H, d) |
| D1-39 | δ=8.23(1H, d), 7.89(1H, d), 7.78∼7.75(2H, q), 7.69∼7.24(19H, m), 7.08∼7.00(8H, m), 6.85(1H, d) |
| D2-1 | δ=8.31 (1H, d), 7.97(1H, d), 7.61~7.54(2H, m), 7.24∼7.27(10H, m), 7.08∼7.00(12H, m), 6.78(1H, s), 6.66 (1H, d) |
| D2-2 | δ=8.31 (1H, d), 7.97(1H, d), 7.75∼7.69(3H, m), 7.55∼7.49(8H, m), 7.24 (4H, m) 7.08∼7.00(11H, m), 6.78(1H, s), 6.66 (1H, d) |
| D2-17 | δ=8.31 (1H, d), 7.97(1H, d), 7.78(1H, d), 7.74∼7.75 (2H, m), 7.24∼7.55(20H, m), 7.08∼7.00(7H, m), 6.78(1H, d), 6.66 (1H, d) |
| D2-39 | δ=8.31(1H, d), 7.97(1H, d), 7.79(1H, d), 7.75(2H, m), 7.24∼7.55(20H, m), 7.08∼7.00(7H, m), 6.88(1H, d), 6.66 (1H, d) |

**[Table 3]**

| Compoun d | FD-MS | Compoun d | FD-MS |
|---|---|---|---|
| A1-1 | m/z=552.68 | C1-1 | m/z=552.68 |
| | (C₄₀H₂₈N₂O=552.68) | | (C₄₀H₂₈N₂O=552.68) |
| A1-2 | m/z=628.77 | C1-2 | m/z=628.77 |
| | (C₄₆H₃₂N₂O=628.77) | | (C₄₆H₃₂N₂O=628.77) |
| A1-17 | m/z=744.94 | C1-17 | m/z=744.94 |
| | (C₅₅H₄₀N₂O=744.94) | | (C₅₅H₄₀N₂O=744.94) |
| A1-39 | m/z=744.94 | C1-39 | m/z=744.94 |
| | (C₅₅H₄₀N₂O=744.94) | | (C₅₅H₄₀N₂O=744.94) |
| A2-1 | m/z=552.68 | C2-1 | m/z=552.68 |
| | (C₄₀H₂₈N₂O=552.68) | | (C₄₀H₂₈N₂O=552.68) |
| A2-17 | m/z=744.94 | C2-2 | m/z=628.77 |
| | (C₅₅H₄₀N₂O=744.94) | | (C₄₆H₃₂N₂O=628.77) |
| A2-39 | m/z=744.94 | C2-17 | m/z=744.94 |
| | (C₅₅H₄₀N₂O=744.94) | | (C₅₅H₄₀N₂O=744.94) |
| B1-1 | m/z=552.68 | C2-39 | m/z=744.94 |
| | (C₄₀H₂₈N₂O=552.68) | | (C₅₅H₄₀N₂O=744.94) |
| B1-2 | m/z=628.77 | D1-1 | m/z=552.68 |
| | (C₄₆H₃₂N₂O=628.77) | | (C₄₀H₂₈N₂O=552.68) |
| B1-17 | m/z=744.94 | D1-2 | m/z=628.77 |
| | (C₅₅H₄₀N₂O=744.94) | | (C₄₆H₃₂N₂O=628.77) |
| B1-39 | m/z=744.94 | D1-17 | m/z=744.94 |
| | (C₅₅H₄₀N₂O=744.94) | | (C₅₅H₄₀N₂O=744.94) |
| B2-1 | m/z=552.68 | D1-39 | m/z=744.94 |
| | (C₄₀H₂₈N₂O=552.68) | | (C₅₅H₄₀N₂O=744.94) |
| B2-2 | m/z=628.77 | D2-1 | m/z=552.68 |
| | (C₄₆H₃₂N₂O=628.77) | | (C₄₀H₂₈N₂O=552.68) |
| B2-17 | m/z=744.94 | D2-2 | m/z=628.77 |
| | (C₅₅H₄₀N₂O=744.94) | | (C₄₆H₃₂N₂O=628.77) |
| B2-39 | m/z=744.94 | D2-17 | m/z=744.94 |
| | (C₅₅H₄₀N₂O=744.94) | | (C₅₅H₄₀N₂O=744.94) |
| | | D2-39 | m/z=744.94 |
| | | | (C₅₅H₄₀N₂O=744.94) |

### [Experimental Example]

### <Experimental Example 1>

### 1) Manufacture of Organic Light Emitting Device

### -Comparative Example 1

A transparent ITO (indium tin oxide) electrode thin film obtained from glass for an OLED (manufactured by Samsung-Corning Co., Ltd.) was ultrasonic cleaned using trichloroethylene, acetone, ethanol and distilled water consecutively for 5 minutes each, stored in isopropanol, and used. Next, the ITO substrate was installed in a substrate folder of a vacuum deposition apparatus, and the following 4,4',4"-tris(N,N-(2-naphthyl)-phenylamino)triphenylamine (2-TNATA) was introduced to a cell in the vacuum deposition apparatus.

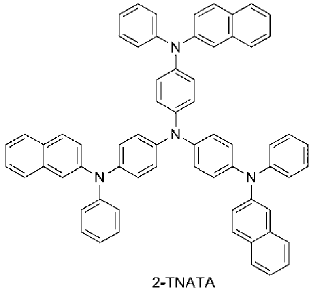

Subsequently, the chamber was evacuated until the degree of vacuum therein reached 10⁻⁶ torr, and then 2-TNATA was evaporated by applying a current to the cell to deposit a hole injection layer having a thickness of 600 Å on the ITO substrate. To another cell in the vacuum deposition apparatus, the following N,N'-bis(α-naphthyl)-N,N'-diphenyl-4,4'-diamine (NPB) was introduced, and evaporated by applying a current to the cell to deposit a hole transfer layer having a thickness of 300 Å on the hole injection layer.

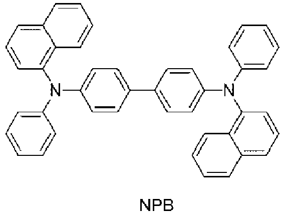

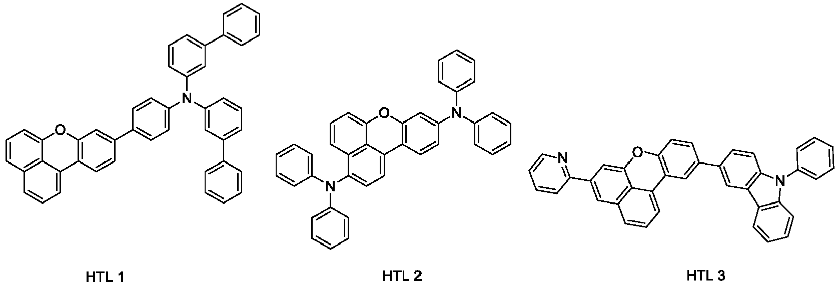

After forming the hole injection layer and the hole transfer layer as above, a blue light emitting material having a structure as below was deposited thereon as a light emitting layer. Specifically, in one side cell in the vacuum deposition apparatus, H1, a blue light emitting host material, was vacuum deposited to a thickness of 200 Å, and D1, a blue light emitting dopant material, was vacuum deposited thereon by 5% with respect to the host material.

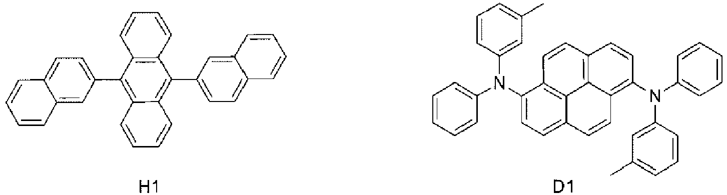

Subsequently, a compound of the following Structural Formula E1 was deposited to a thickness of 300 Å as an electron transfer layer.

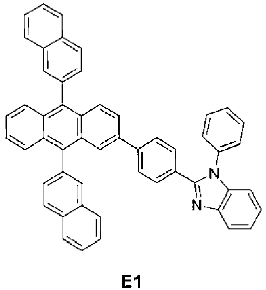

As an electron injection layer, lithium fluoride (LiF) was deposited to a thickness of 10 Å, and an Al cathode was employed to a thickness of 1,000 Å, and as a result, an OLED was manufactured. Meanwhile, all the organic compounds required to manufacture the OLED were vacuum sublimation purified under 10⁻⁸ torr to 10⁻⁶ torr by each material to be used in the OLED manufacture.

### -Comparative Examples 2 to 4 and Examples 1 to 31

Organic electroluminescent devices were manufactured in the same manner as in Comparative Example 1 except that HTL1, HTL2, HTL3 or compounds shown in Table 4 were used instead of NPB used when forming the hole transfer layer.

### 2) Evaluation on Organic Light Emitting Device

For each of the organic light emitting devices manufactured as above, electroluminescent (EL) properties were measured using M7000 manufactured by McScience Inc., and with the measurement results, T₉₅ was measured when standard luminance was 700 cd/m² through a lifetime measurement system (M6000) manufactured by McScience Inc.. Results of measuring driving voltage, light emission efficiency, color coordinate (CIE) and lifetime of the blue organic light emitting devices manufactured according to the present disclosure are as shown in Table 4.

**[Table 4]**

| | Compound | Driving Voltage (V) | Light Emission Efficiency (cd/A) | Lifetime (T₉₅) |
|---|---|---|---|---|
| Example 1 | A1-1 | 5.05 | 6.11 | 58 |
| Example 2 | A1-2 | 4.89 | 6.23 | 67 |
| Example 3 | A1-17 | 4.77 | 6.30 | 59 |
| Example 4 | A1-39 | 4.72 | 6.98 | 56 |
| Example 5 | A2-1 | 4.80 | 6.89 | 67 |
| Example 6 | A2-17 | 4.75 | 6.95 | 60 |
| Example 7 | A2-39 | 4.68 | 6.93 | 55 |
| Example 8 | B1-1 | 4.87 | 6.84 | 63 |
| Example 9 | B1-2 | 4.75 | 6.91 | 56 |
| Example 10 | B1-17 | 4.98 | 6.35 | 59 |
| Example 11 | B1-39 | 4.11 | 6.12 | 64 |
| Example 12 | B2-1 | 4.96 | 6.10 | 72 |
| Example 13 | B2-2 | 4.17 | 6.20 | 75 |
| Example 14 | B2-17 | 4.65 | 6.43 | 68 |
| Example 15 | B2-39 | 4.82 | 6.84 | 57 |
| Example 16 | C1-1 | 4.84 | 6.97 | 56 |
| Example 17 | C1-2 | 4.90 | 6.81 | 61 |
| Example 18 | C1-17 | 4.88 | 6.82 | 62 |
| Example 19 | C1-39 | 4.74 | 6.75 | 56 |
| Example 20 | C2-1 | 4.81 | 6.82 | 58 |
| Example 21 | C2-2 | 4.26 | 6.44 | 66 |
| Example 22 | C2-17 | 4.99 | 6.38 | 58 |
| Example 23 | C2-39 | 4.16 | 6.20 | 59 |
| Example 24 | D1-1 | 4.95 | 6.42 | 54 |
| Example 25 | D1-2 | 4.31 | 6, 30 | 55 |
| Example 26 | D1-17 | 4.33 | 6.22 | 62 |
| Example 27 | D1-39 | 4.32 | 6.33 | 69 |
| Example 28 | D2-1 | 4.82 | 6.35 | 59 |
| Example 29 | D2-2 | 4.84 | 6.60 | 49 |
| Example 30 | D2-17 | 4.94 | 6.68 | 52 |
| Example 31 | D2-39 | 4.96 | 6.70 | 61 |
| Comparative Example 1 | NPB | 6.01 | 5.43 | 45 |
| Comparative Example 2 | HTL1 | 5.95 | 5.90 | 49 |
| Comparative Example 3 | HTL2 | 5.84 | 6.01 | 48 |
| Comparative Example 4 | HTL3 | 5.26 | 5.83 | 46 |

As seen from the results of Table 4, the organic light emitting device using the hole transfer layer material of the blue organic light emitting device of the present disclosure had lower driving voltage and significantly improved light emission efficiency and lifetime compared to Comparative Examples 1 to 4.

When comparing Comparative Example 1 and the compound of the present disclosure, having an arylamine group is similar, however, the benzoxanthene core is substituted with an amine group and as a result, a property of preventing device properties from declining due to increasing a driving voltage of the organic light emitting device is obtained.

When comparing Comparative Example 2 and the compound of the present disclosure, having an arylamine group in the benzoxanthene is similar, however, Comparative Example 2 is different from the compound of the present disclosure in that the benzoxanthene is substituted with one amine group.

In the compound of the present disclosure, the benzoxanthene is substituted with two bulky amine groups generating steric hindrance and thereby resulting in an increase in the T1 value, and it is considered that this brings excellence in all aspects of driving voltage, efficiency and lifetime. In addition, by the benzoxanthene having two substituents, HOMO electron cloud is expanded, which further strengthens hole injection and hole transfer capability by increasing the HOMO energy level, and as a result, a driving voltage of the device using the same may be lowered.

When comparing Comparative Example 3 and the compound of the present disclosure, having the benzoxanthene substituted with two arylamine groups is similar, however, Comparative Example 3 is different from the present disclosure in the substitution position of the amine group.

When 4, 5 and 6 positions of the benzoxanthene are substituted with an amine group, hole mobility is higher, the T1 value is higher by having a bulky steric structure, and there is more space for the holes to be trapped compared to 1, 2 and 3 positions, which is considered to increase efficiency by improving a charge balance in the light emitting layer.

When comparing Comparative Example 4 and the compound of the present disclosure, the benzoxanthene is substituted with two substituents, and the position of substitution is also similar, however, Comparative Example 4 is different from the present disclosure in that the substituent is not an amine group.

By substituting the benzoxanthene with an amine group, it is considered that hole transfer properties or stability of the amine derivative are enhanced, which brings excellence in all aspects of driving voltage, efficiency and lifetime.

### <Experimental Example 2>

### 1) Manufacture of Organic Light Emitting Device

### -Comparative Example 5

A transparent ITO electrode thin film obtained from glass for an OLED (manufactured by Samsung-Corning Co., Ltd.) was ultrasonic cleaned using trichloroethylene, acetone, ethanol and distilled water consecutively for 5 minutes each, stored in isopropanol, and used. Next, the ITO substrate was installed in a substrate folder of a vacuum deposition apparatus, and the following 4,4',4"-tris(N,N-(2-naphthyl)-phenylamino)triphenylamine (2-TNATA) was introduced to a cell in the vacuum deposition apparatus.

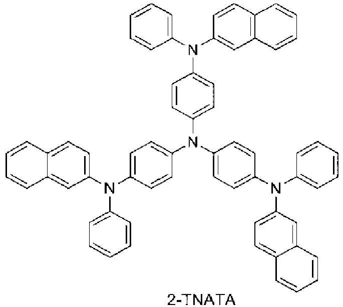

Subsequently, the chamber was evacuated until the degree of vacuum therein reached 10⁻⁶ torr, and then 2-TNATA was evaporated by applying a current to the cell to deposit a hole injection layer having a thickness of 600 Å on the ITO substrate. To another cell in the vacuum deposition apparatus, the following N,N'-bis(α-naphthyl)-N,N'-diphenyl-4,4'-diamine (NPB) was introduced, and evaporated by applying a current to the cell to deposit a hole transfer layer having a thickness of 300 Å on the hole injection layer.

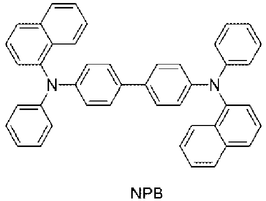

After forming the hole injection layer and the hole transfer layer as above, a blue light emitting material having a structure as below was deposited thereon as a light emitting layer. Specifically, in one side cell in the vacuum deposition apparatus, H1, a blue light emitting host material, was vacuum deposited to a thickness of 200 Å, and D1, a blue light emitting dopant material, was vacuum deposited thereon by 5% with respect to the host material.

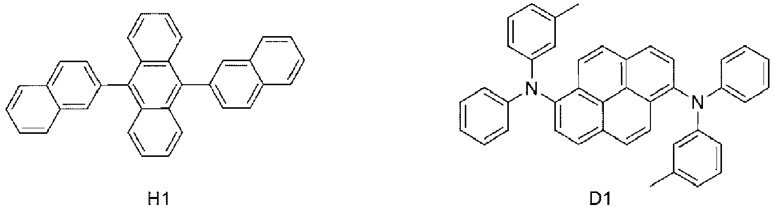

Subsequently, a compound of the following Structural Formula E1 was deposited to a thickness of 300 Å as an electron transfer layer.

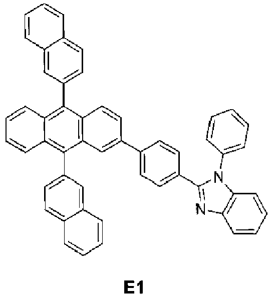

As an electron injection layer, lithium fluoride (LiF) was deposited to a thickness of 10 Å, and an Al cathode was employed to a thickness of 1,000 Å, and as a result, an OLED was manufactured. Meanwhile, all the organic compounds required to manufacture the OLED were vacuum sublimation purified under 10⁻⁸ torr to 10⁻⁶ torr by each material to be used in the OLED manufacture.

### -Comparative Examples 6 to 8 and Examples 32 to 62

Organic electroluminescent devices were manufactured in the same manner as in Comparative Example 5 except that, after forming the hole transfer layer NPB to a thickness of 250 Å, an electron blocking layer was formed to a thickness of 50 Å on the hole transfer layer using compounds shown in Table 5.

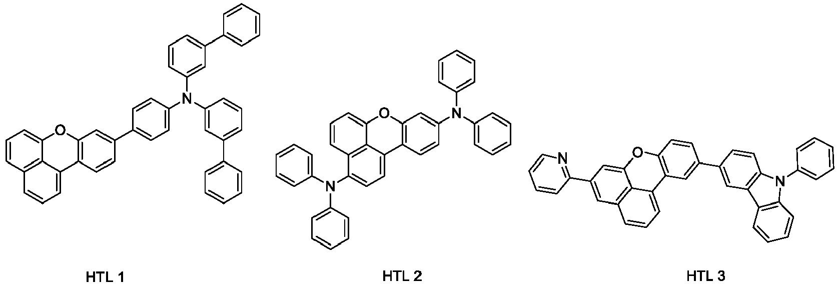

### 2) Evaluation on Organic Light Emitting Device

Results of measuring driving voltage, light emission efficiency and lifetime of the blue organic light emitting devices manufactured according to the present disclosure are as shown in Table 5.

**[Table 5]**

| | Compound | Driving Voltage (V) | Light Emission Efficiency (cd/A) | Lifetime (T₉₅) |
|---|---|---|---|---|
| Example 32 | A1-1 | 5.10 | 6.11 | 60 |
| Example 33 | A1-2 | 4.96 | 6.13 | 65 |
| Example 34 | A1-17 | 4.97 | 6.23 | 63 |
| Example 35 | A1-39 | 4.82 | 5.98 | 59 |
| Example 36 | A2-1 | 5.12 | 6.08 | 66 |
| Example 37 | A2-17 | 5.03 | 5.95 | 61 |
| Example 38 | A2-39 | 5.11 | 6.01 | 57 |
| Example 39 | B1-1 | 4.97 | 5.84 | 65 |
| Example 40 | B1-2 | 4.75 | 6.41 | 55 |
| Example 41 | B1-17 | 4.98 | 6.35 | 60 |
| Example 42 | B1-39 | 5.11 | 6.12 | 59 |
| Example 43 | B2-1 | 4.96 | 6.10 | 71 |
| Example 44 | B2-2 | 5.01 | 6.20 | 64 |
| Example 45 | B2-17 | 5.04 | 6.44 | 62 |
| Example 46 | B2-39 | 4.92 | 6.14 | 59 |
| Example 47 | C1-1 | 4.88 | 6.56 | 57 |
| Example 48 | C1-2 | 4.94 | 6.66 | 61 |
| Example 49 | C1-17 | 4.78 | 5.97 | 63 |
| Example 50 | C1-39 | 5.06 | 6.78 | 57 |
| Example 51 | C2-1 | 4.81 | 6.82 | 55 |
| Example 52 | C2-2 | 5.08 | 6.44 | 59 |
| Example 53 | C2-17 | 4.99 | 6.28 | 54 |
| Example 54 | C2-39 | 4.88 | 6.20 | 61 |
| Example 55 | D1-1 | 4.95 | 6.42 | 63 |
| Example 56 | D1-2 | 4.77 | 6, 33 | 60 |
| Example 57 | D1-17 | 4.68 | 6.28 | 55 |
| Example 58 | D1-39 | 4.56 | 5.95 | 56 |
| Example 59 | D2-1 | 5.10 | 6.35 | 59 |
| Example 60 | D2-2 | 5.12 | 6.60 | 52 |
| Example 61 | D2-17 | 4.94 | 6.68 | 61 |
| Example 62 | D2-39 | 4.96 | 6.70 | 51 |
| Comparative Example 5 | NPB | 6.42 | 5.46 | 51 |
| Comparative Example 6 | HTL1 | 5.88 | 5.95 | 46 |
| Comparative Example 7 | HTL2 | 5.78 | 5.43 | 49 |
| Comparative Example 8 | HTL3 | 5.26 | 5.13 | 48 |

As seen from the results of Table 5, the organic light emitting device using the electron blocking layer material of the blue organic light emitting device of the present disclosure had lower driving voltage and significantly improved light emission efficiency and lifetime compared to Comparative Examples 5 to 8. When electrons pass through a hole transfer layer and migrate to an anode without binding in a light emitting layer, efficiency and lifetime of an OLED are reduced. When using a compound having a high LUMO level as an electron blocking layer in order to prevent this phenomenon, electrons to migrate to an anode after passing through a light emitting layer are blocked by an energy barrier of the electron blocking layer. Accordingly, holes and electrons are more likely to form excitons, which increases possibility of being emitted as light in the light emitting layer, and it is considered that the compound of the present disclosure brings excellence in all aspects of driving voltage, efficiency and lifetime due to a higher LUMO level compared to Comparative Examples 5 to 8.

## Claims

1. A heterocyclic compound represented by the following Chemical Formula 1: 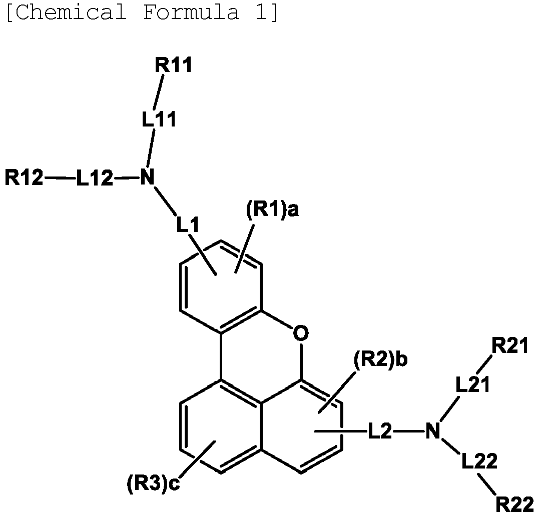 wherein, in Chemical Formula 1,
L1, L2, L11, L12, L21 and L22 are each independently a direct bond; a substituted or unsubstituted C6 to C60 arylene group; or a substituted or unsubstituted C2 to C60 heteroarylene group,
R1 to R3, R11, R12, R21 and R22 are each independently hydrogen; deuterium; a halogen group; a cyano group; a substituted or unsubstituted C1 to C60 alkyl group; a substituted or unsubstituted C6 to C60 aryl group; or a substituted or unsubstituted C2 to C60 heteroaryl group,
a and c are each an integer of 1 to 3,
b is 1 or 2,
when a is 2 or greater, R1s are the same as or different from each other,
when b is 2, R2s are the same as or different from each other, and
when c is 2 or greater, R3s are the same as or different from each other.

2. The heterocyclic compound of Claim 1, wherein Chemical Formula 1 is represented by the following Chemical Formula 1-1: 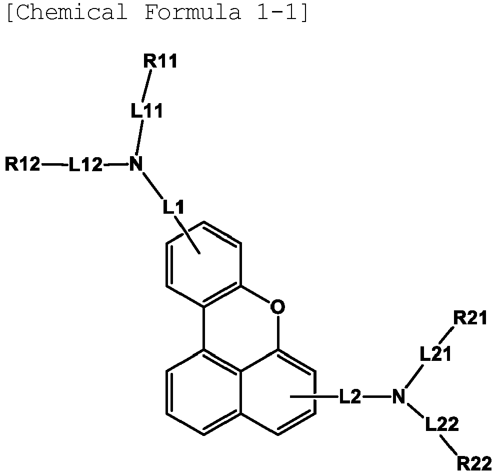 in Chemical Formula 1-1,
each substituent has the same definition as in Chemical Formula 1.

3. The heterocyclic compound of Claim 1, wherein L1 and L2 are each independently a direct bond; or a substituted or unsubstituted C6 to C20 arylene group.

4. The heterocyclic compound of Claim 1, wherein R11, R12, R21 and R22 are each independently a substituted or unsubstituted C6 to C30 aryl group; or a substituted or unsubstituted C2 to C30 heteroaryl group.

5. The heterocyclic compound of Claim 1, wherein R1 to R3 are hydrogen; or deuterium.

6. The heterocyclic compound of Claim 1, wherein Chemical Formula 1 is represented by any one of the following compounds:

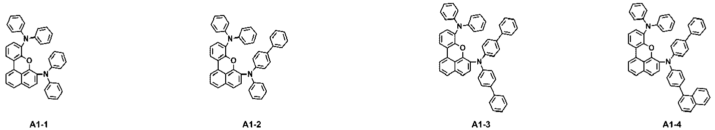

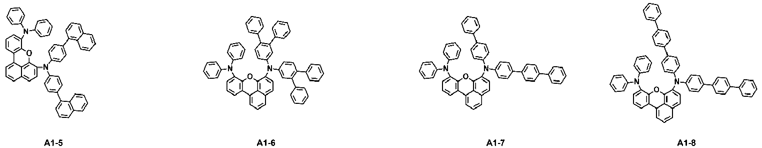

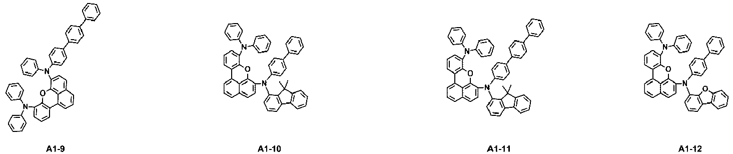

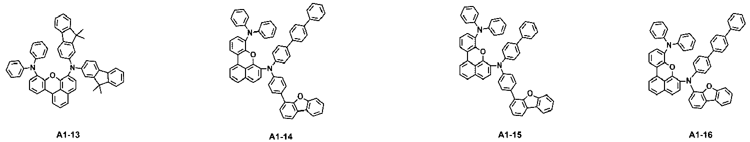

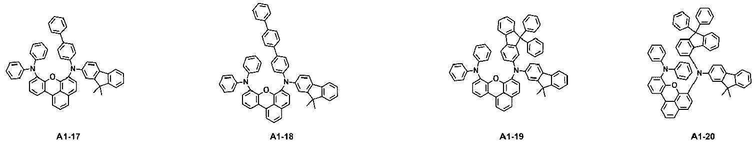

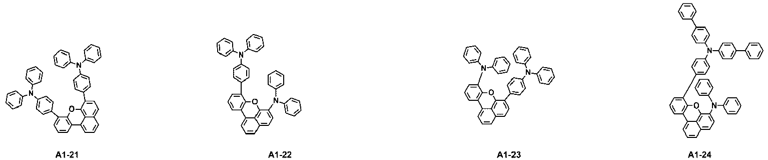

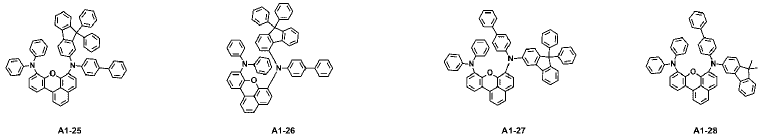

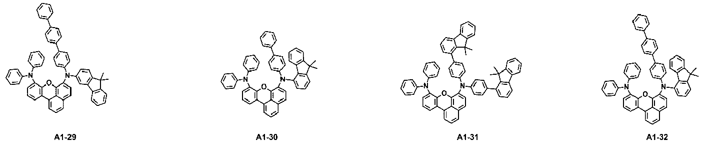

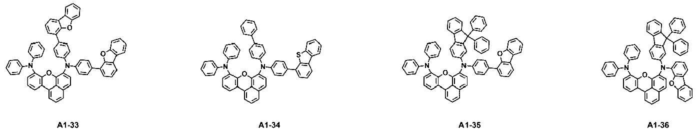

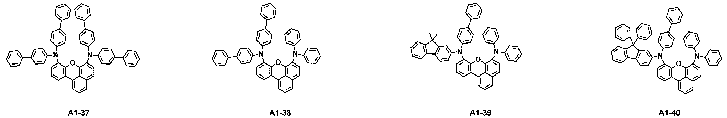

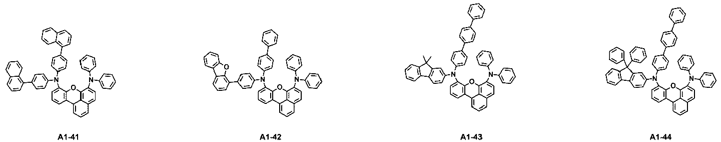

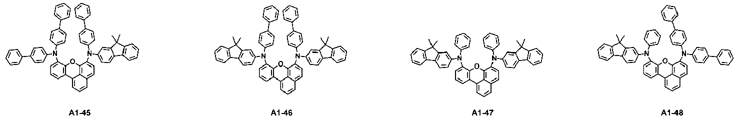

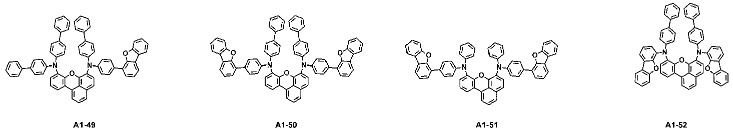

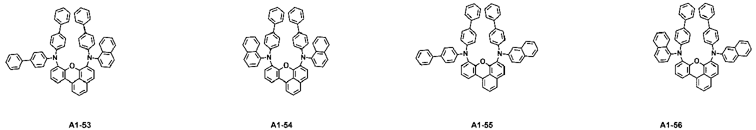

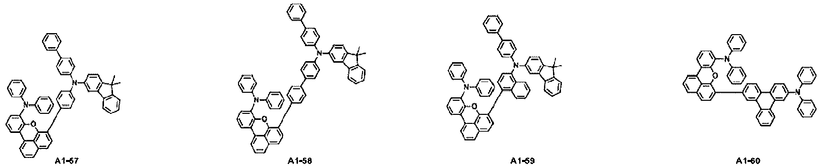

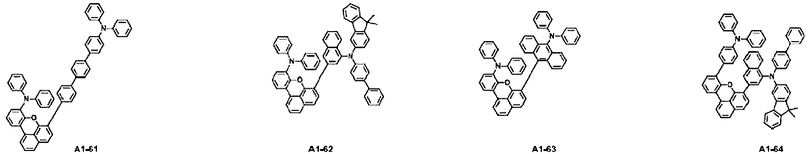

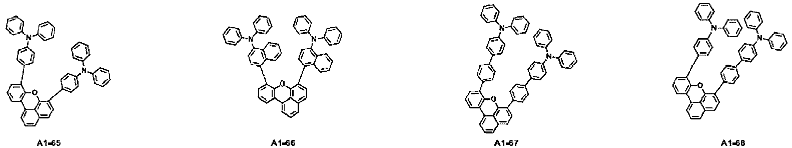

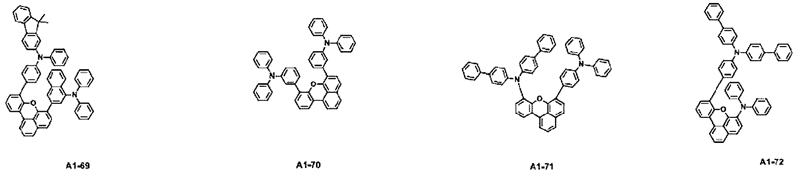

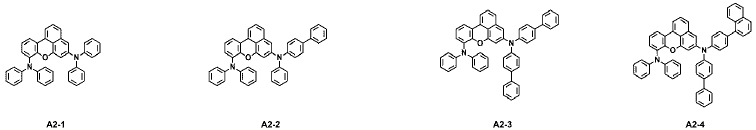

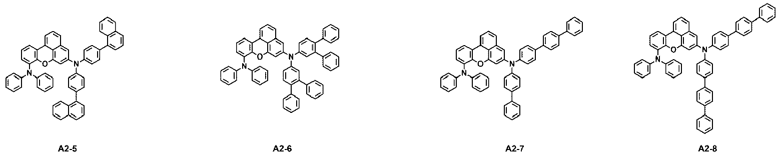

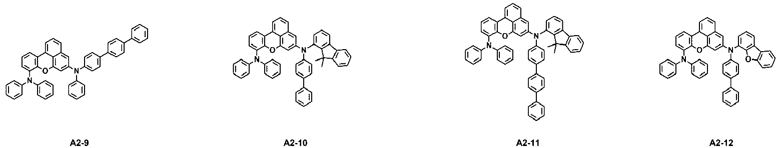

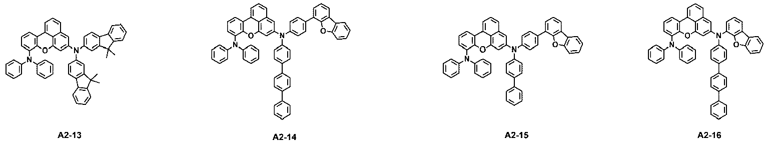

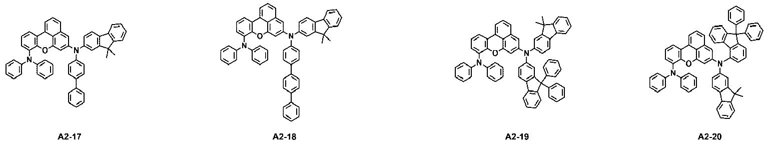

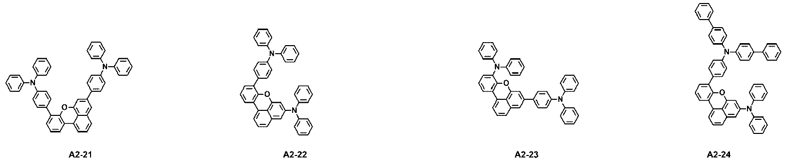

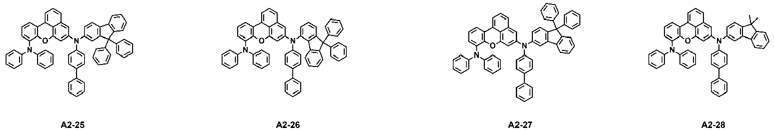

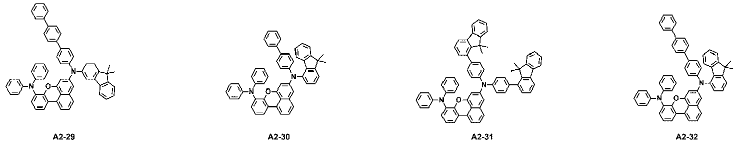

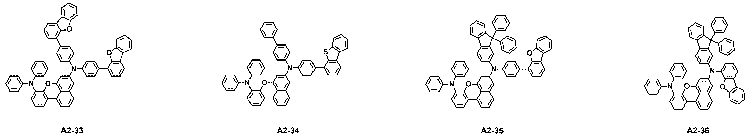

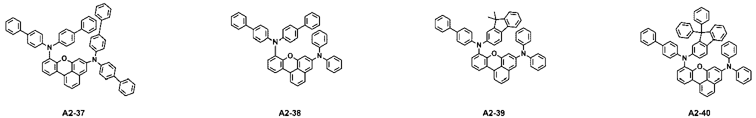

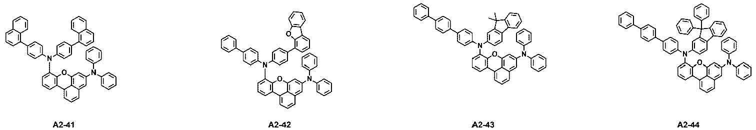

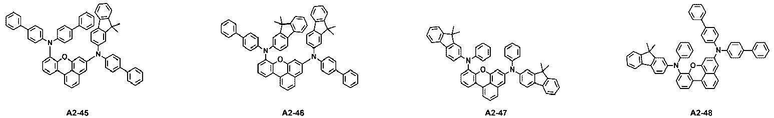

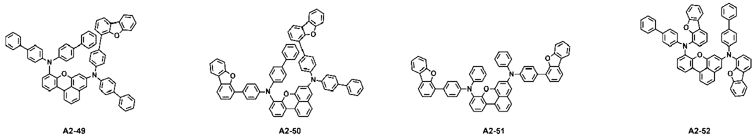

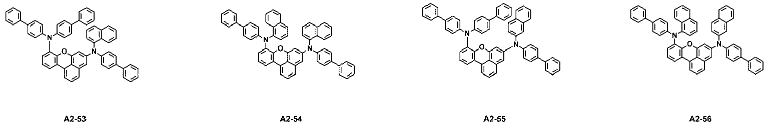

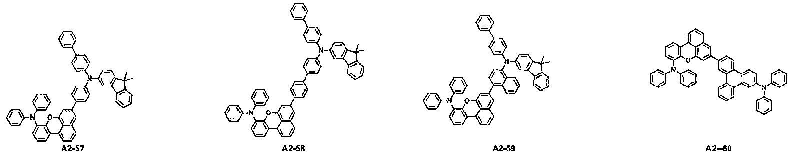

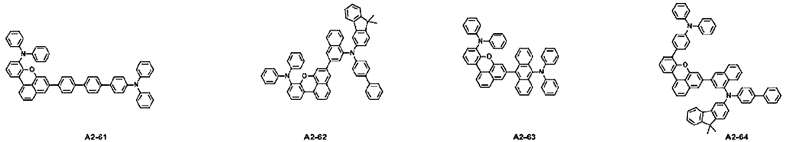

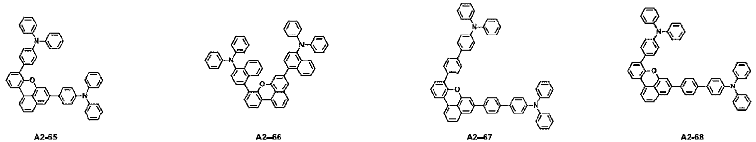

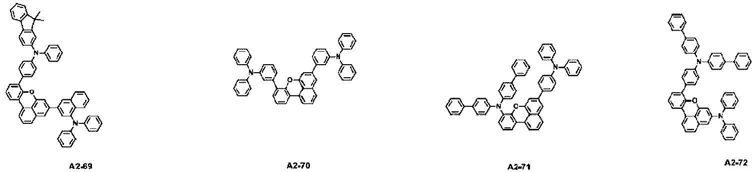

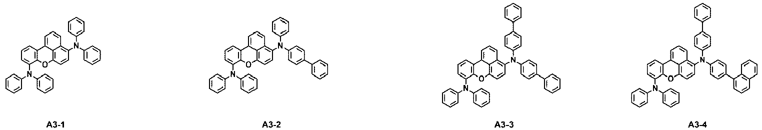

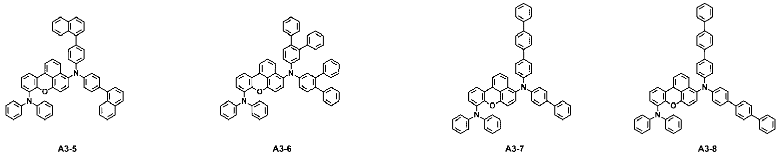

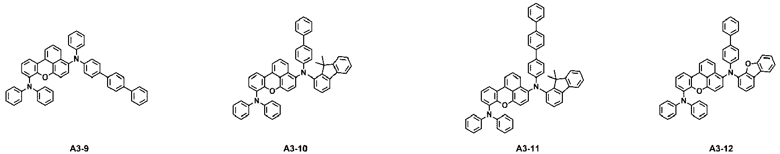

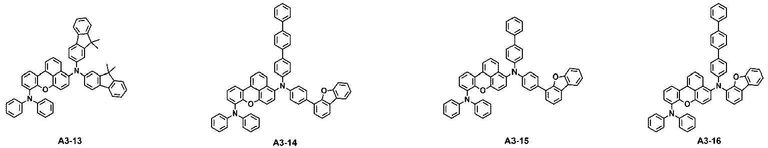

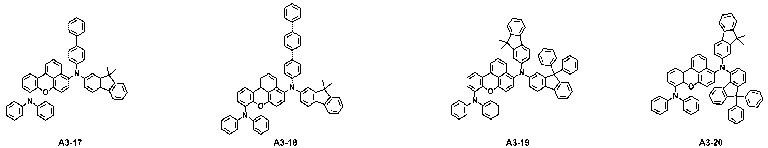

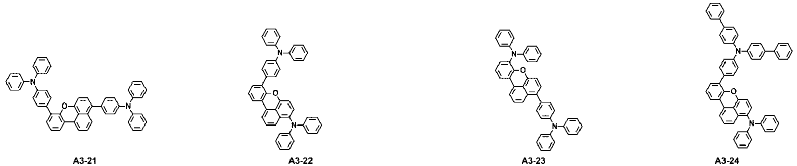

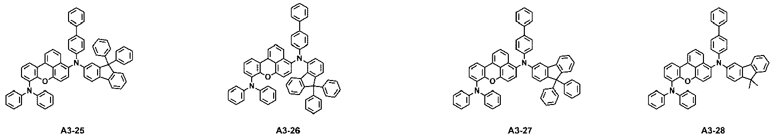

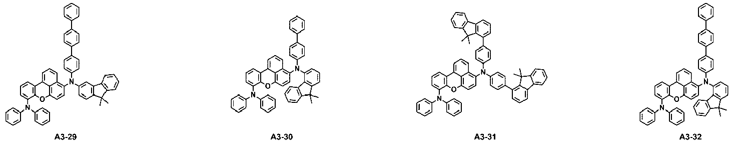

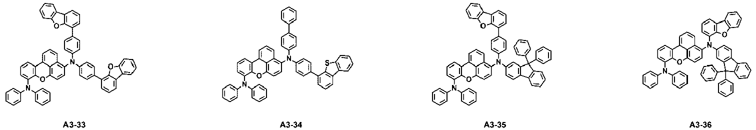

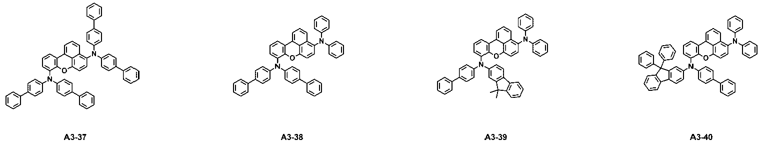

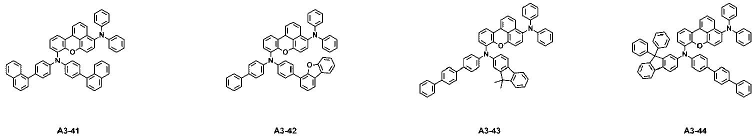

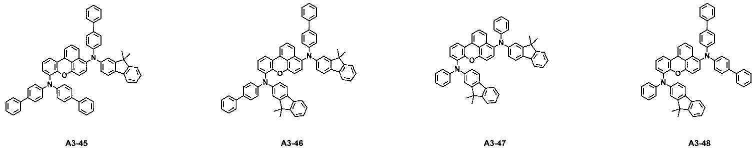

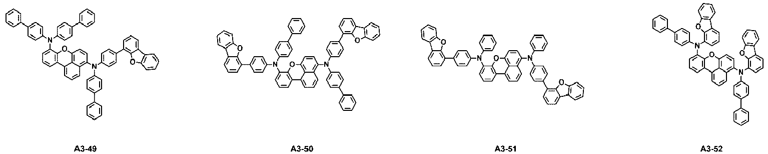

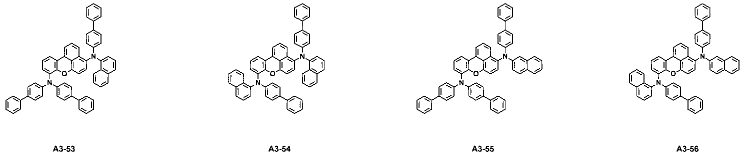

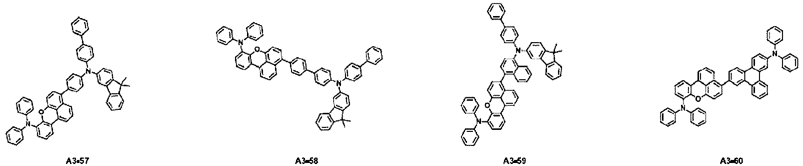

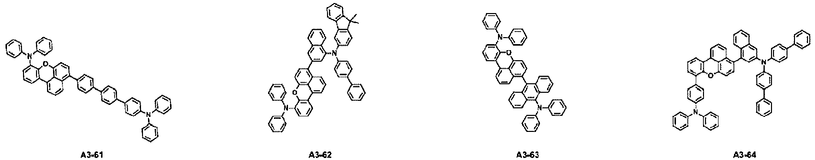

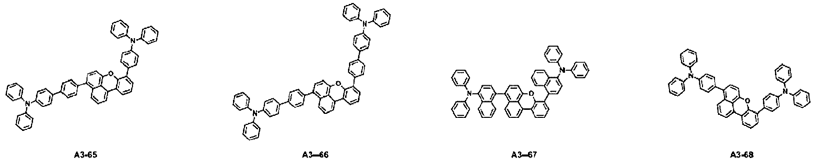

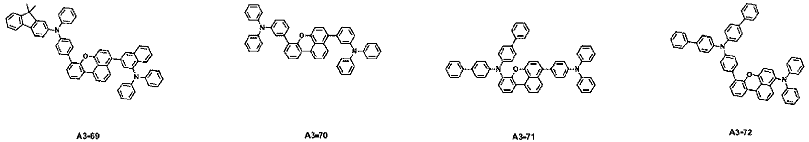

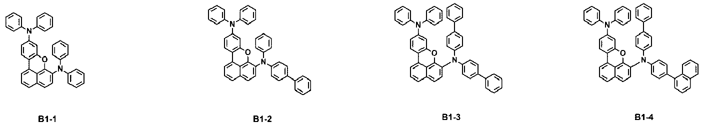

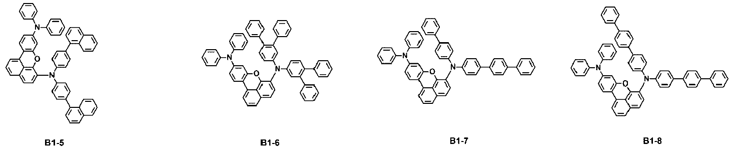

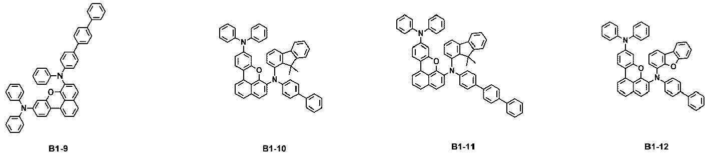

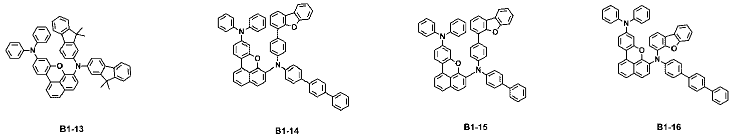

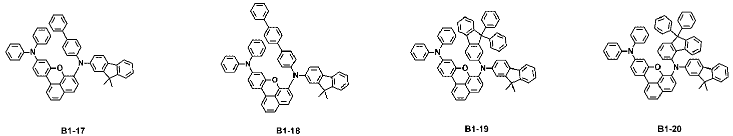

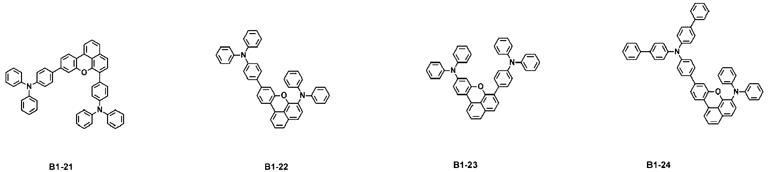

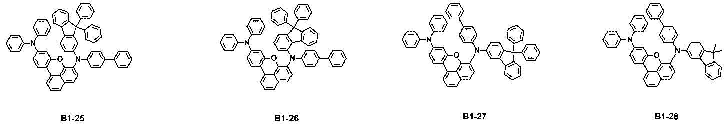

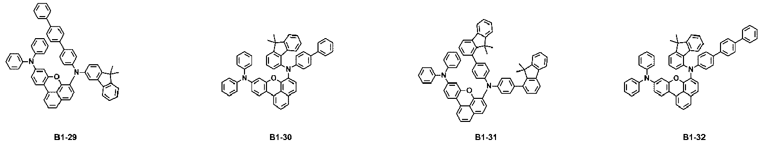

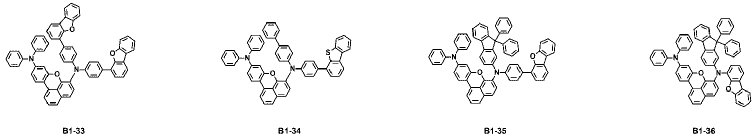

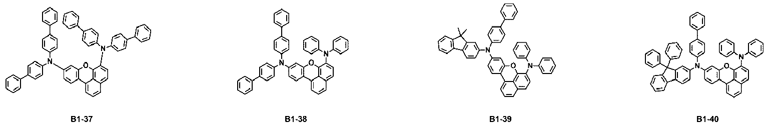

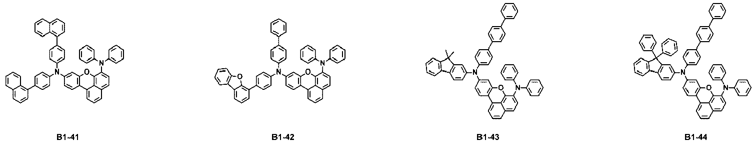

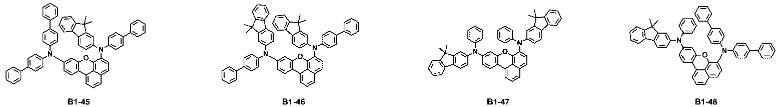

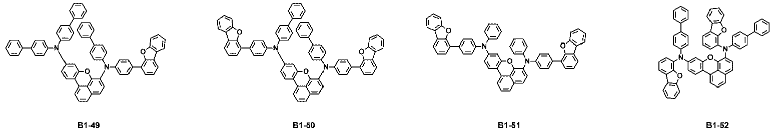

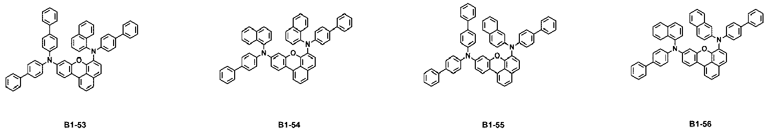

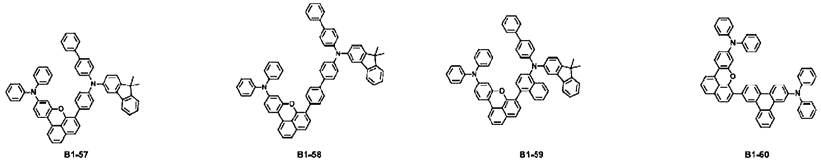

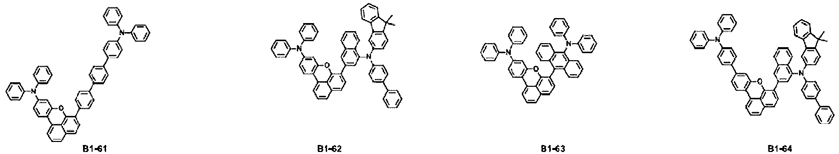

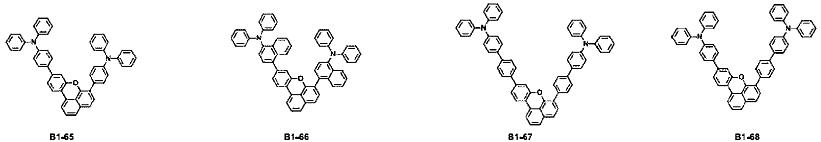

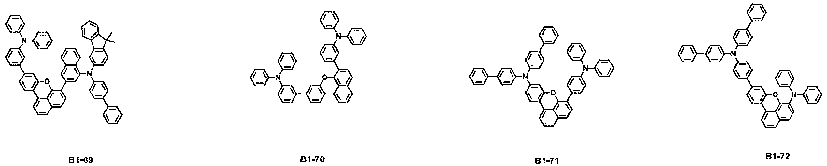

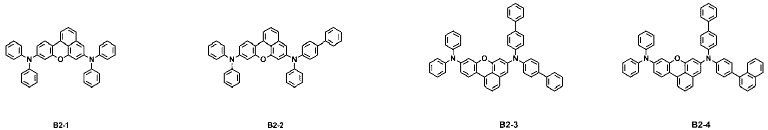

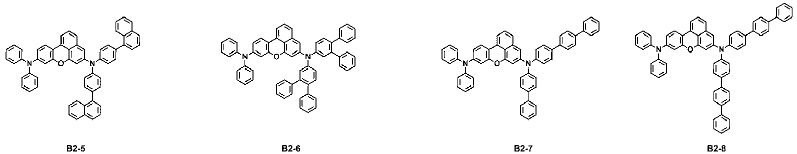

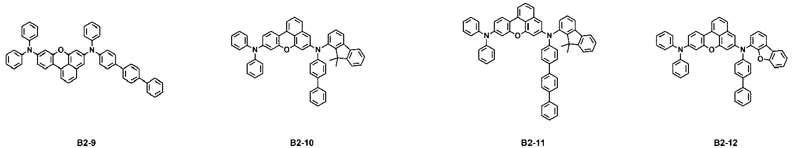

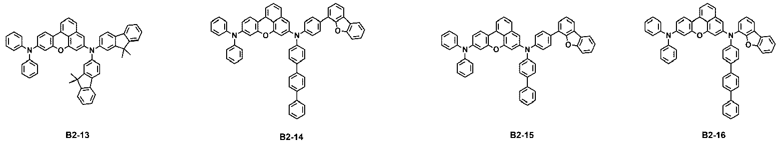

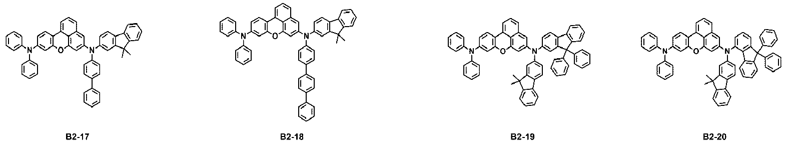

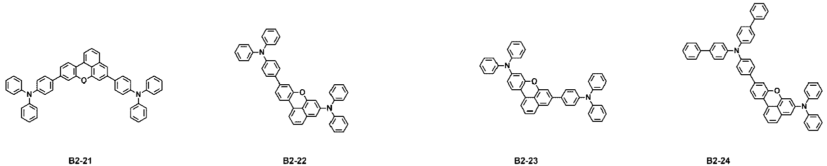

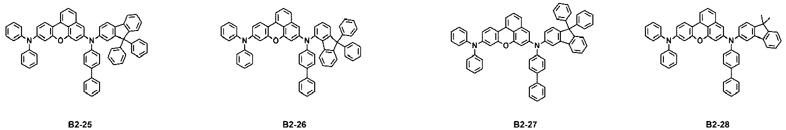

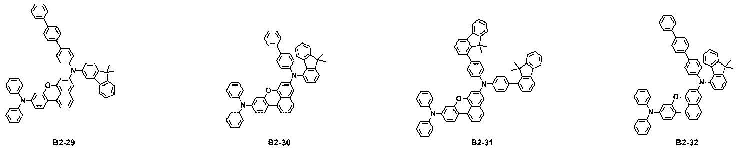

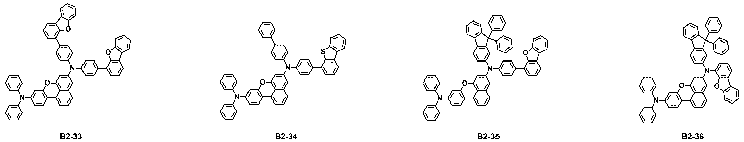

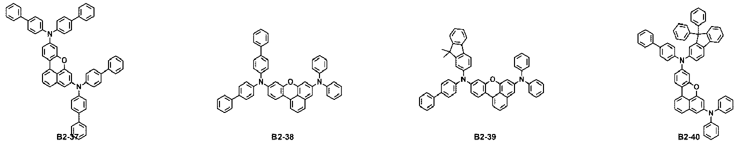

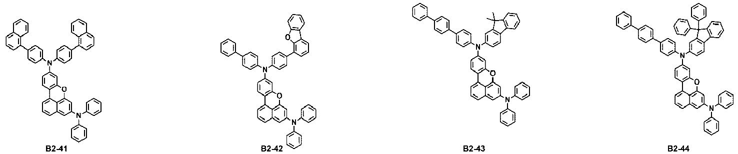

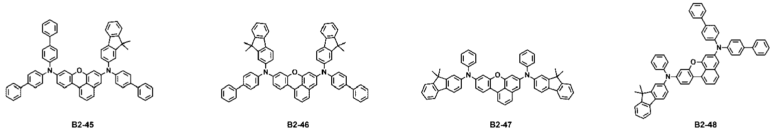

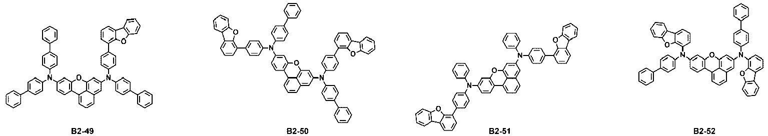

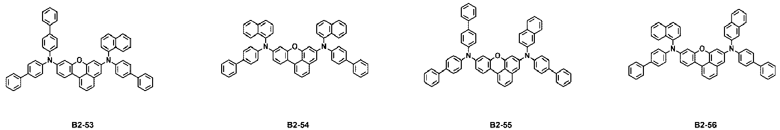

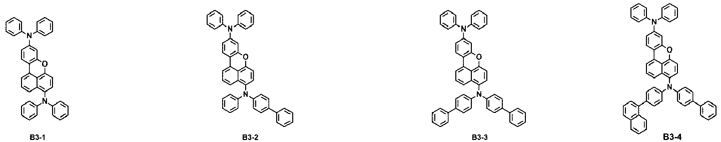

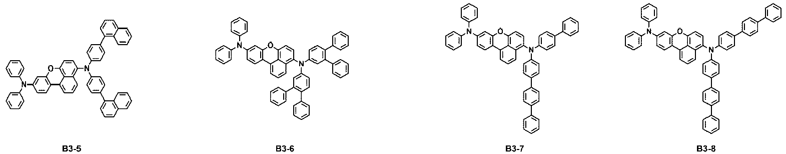

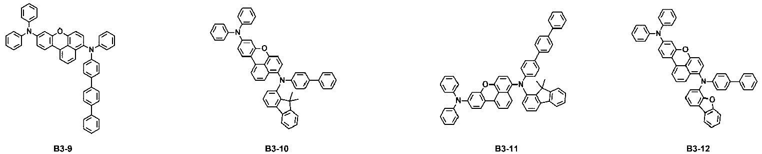

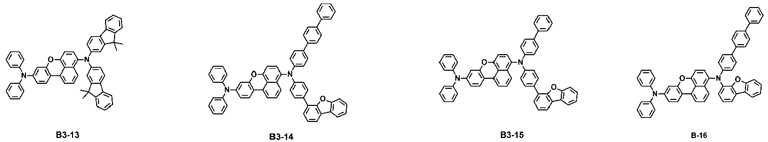

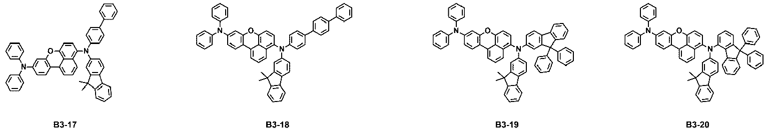

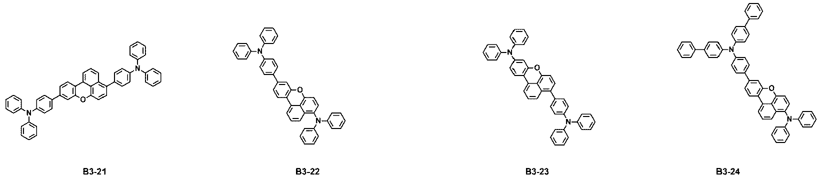

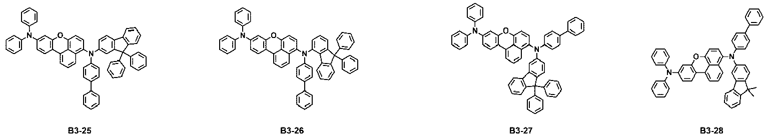

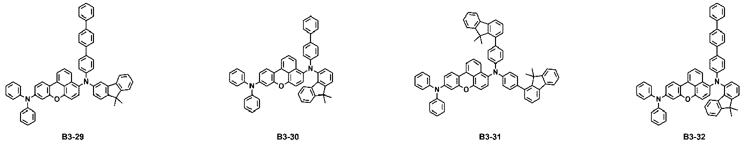

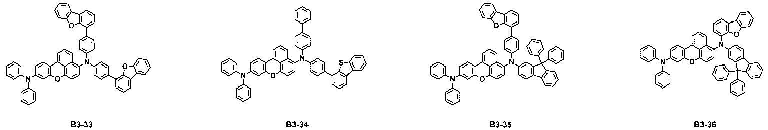

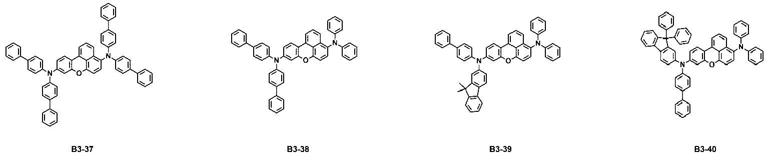

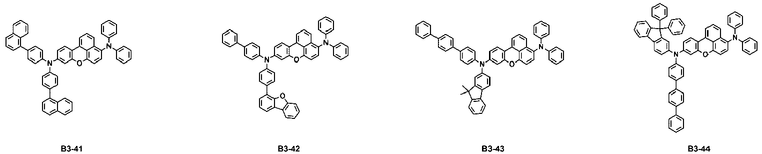

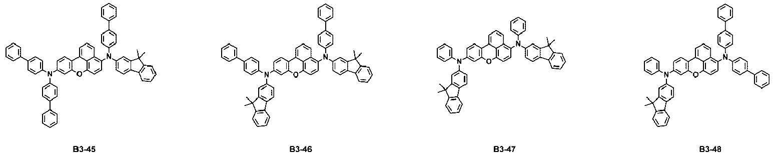

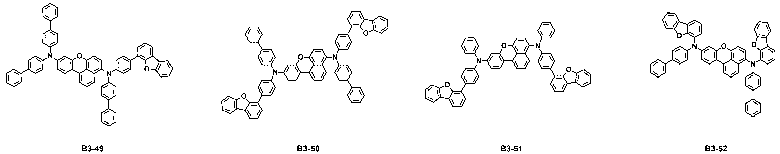

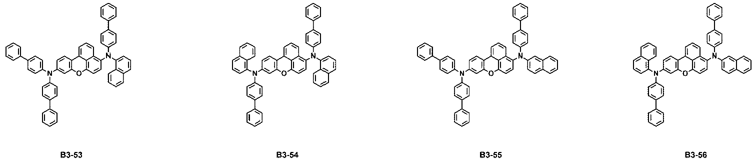

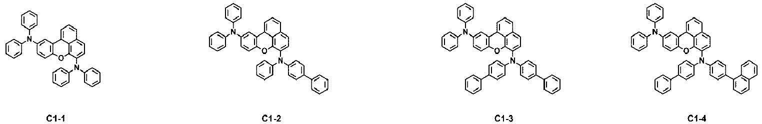

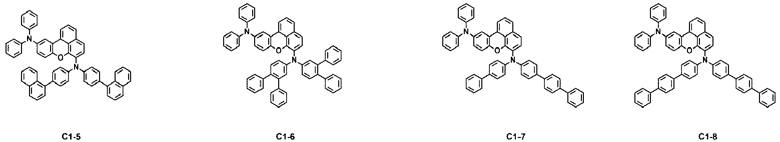

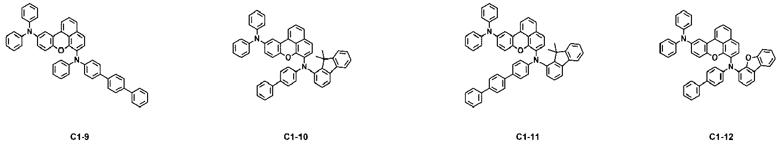

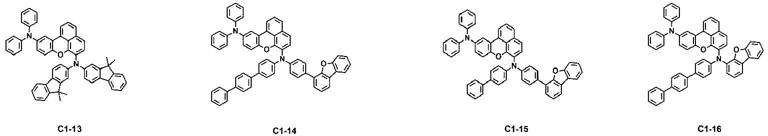

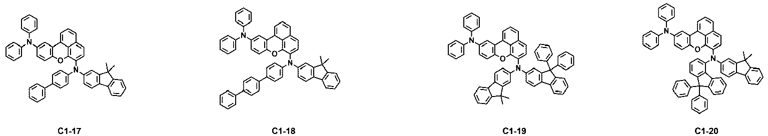

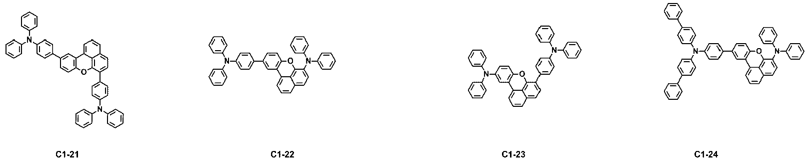

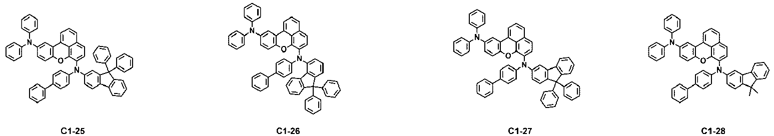

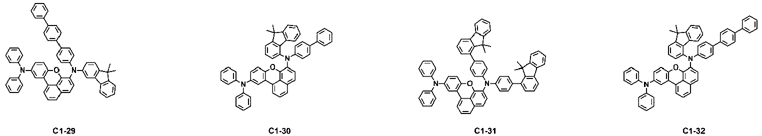

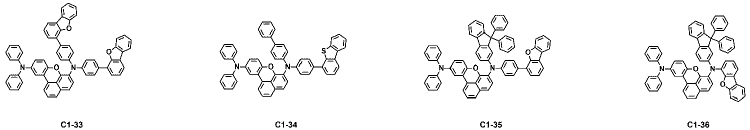

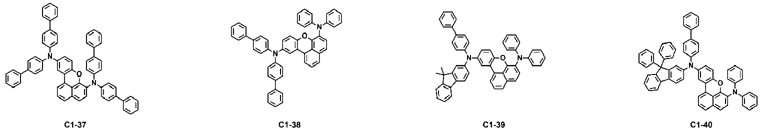

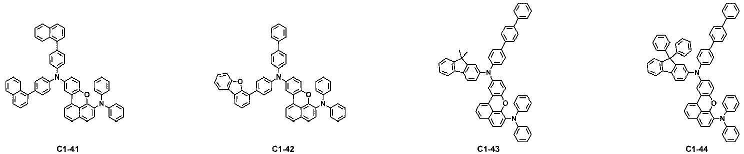

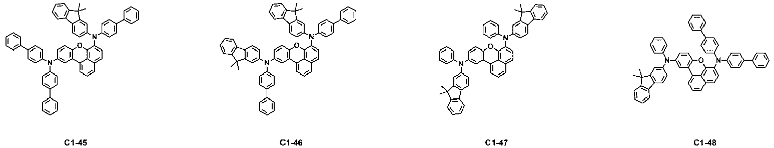

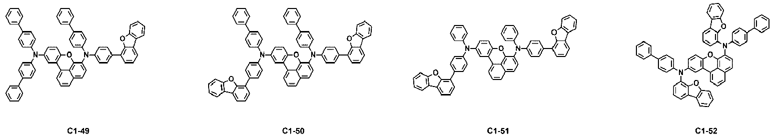

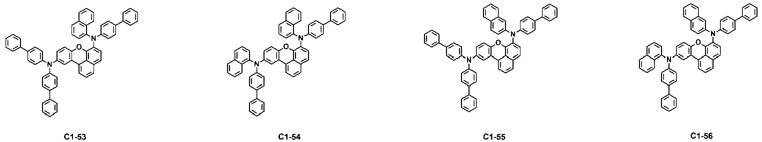

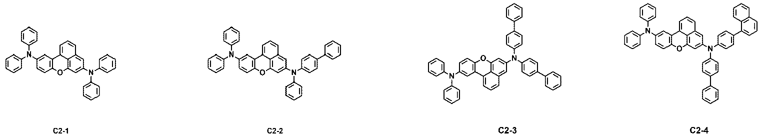

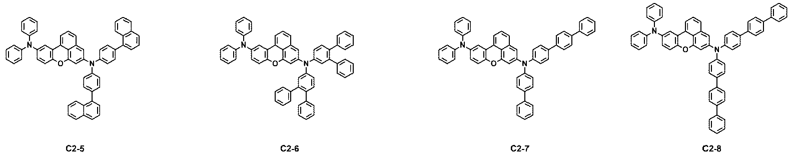

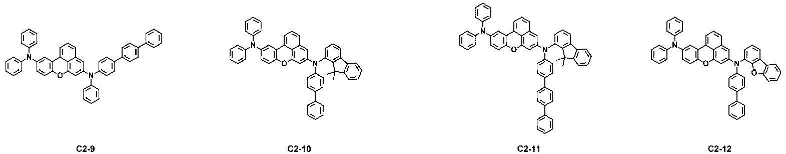

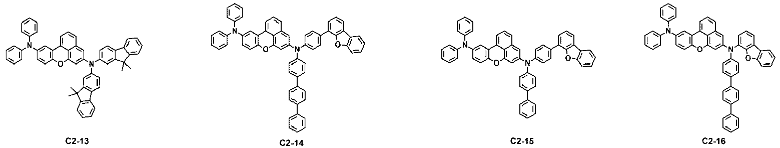

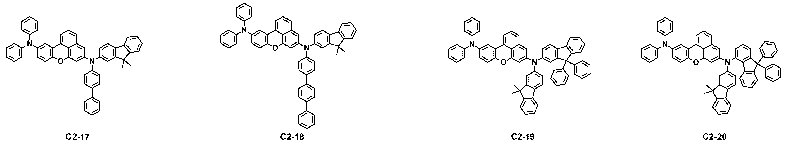

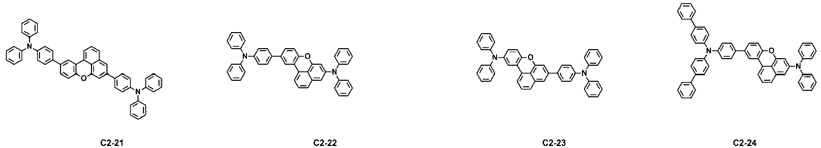

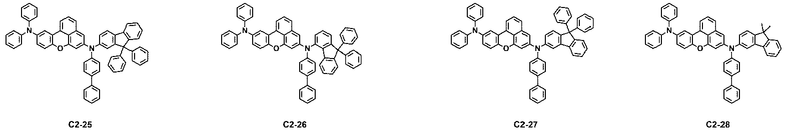

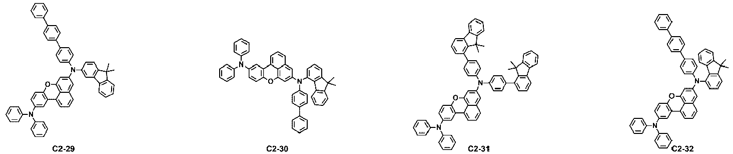

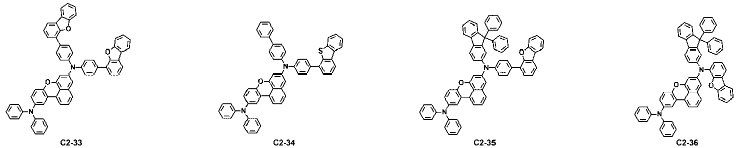

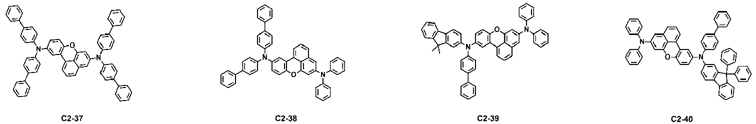

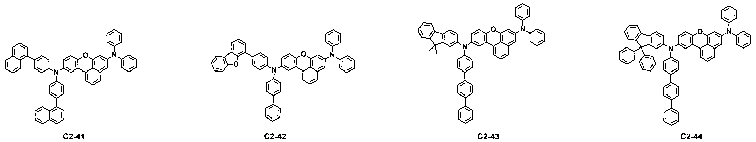

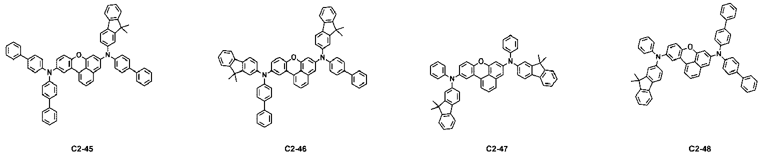

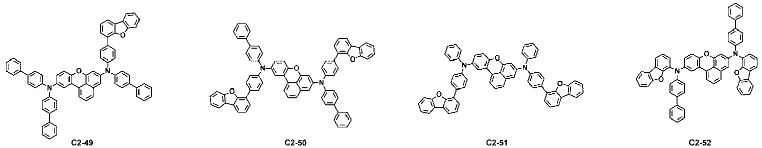

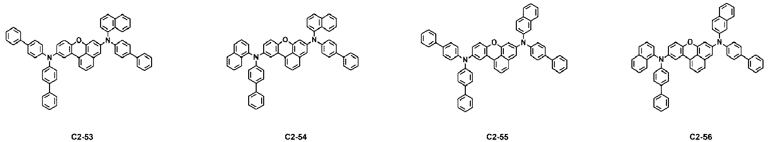

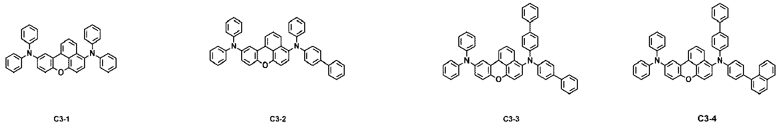

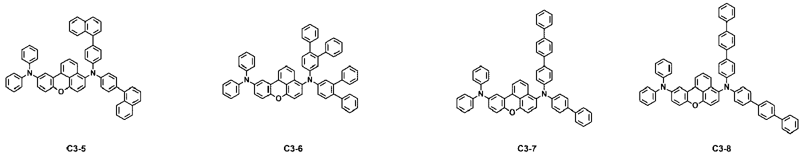

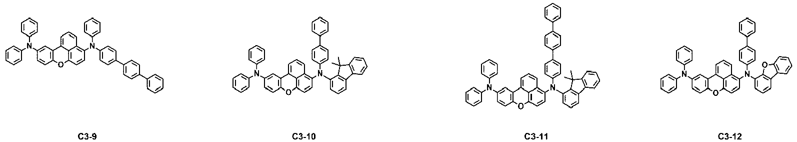

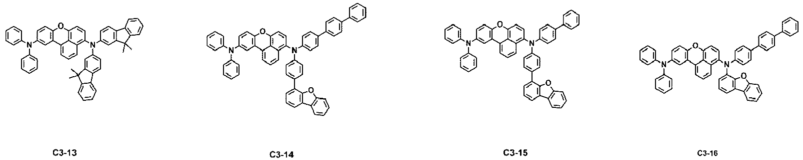

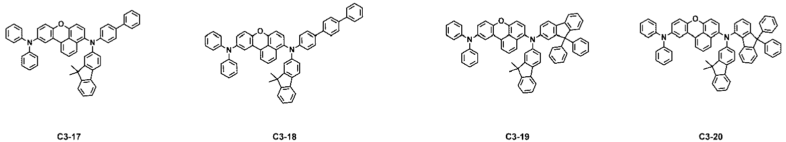

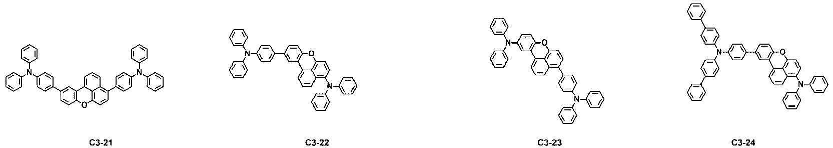

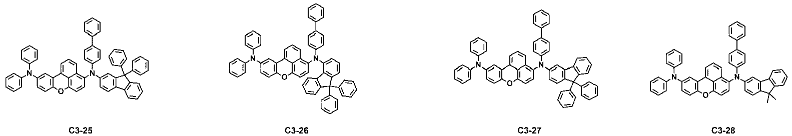

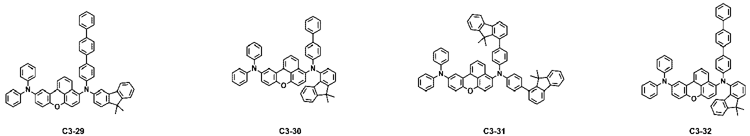

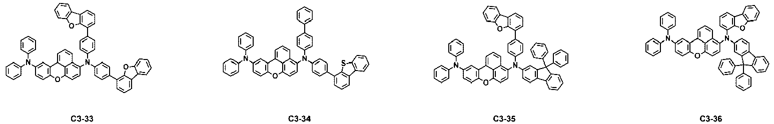

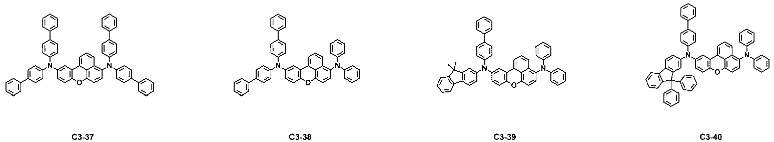

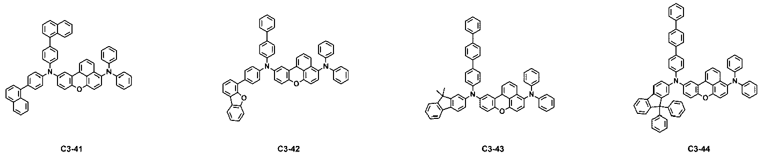

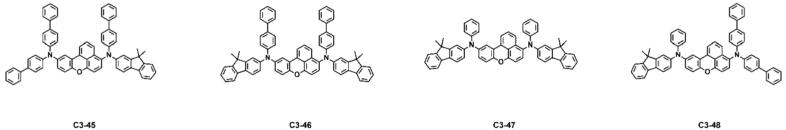

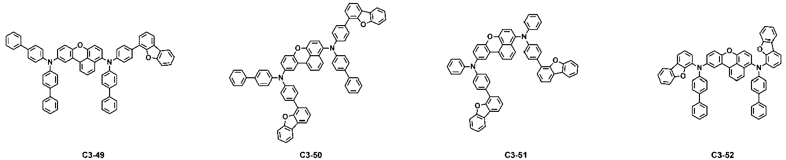

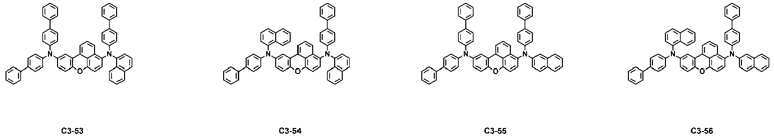

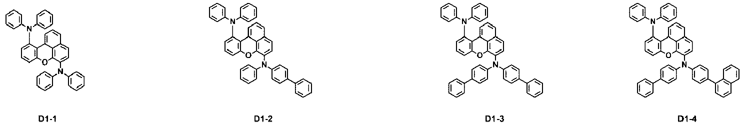

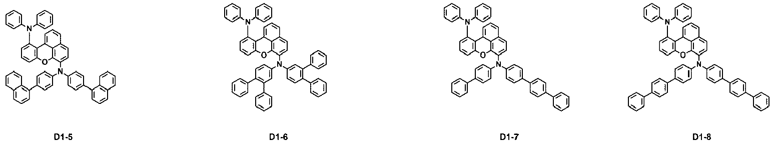

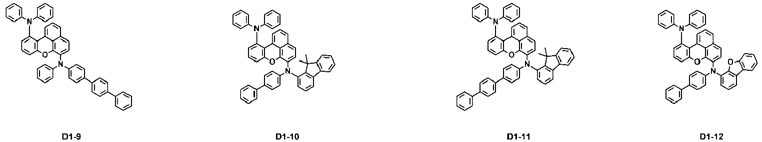

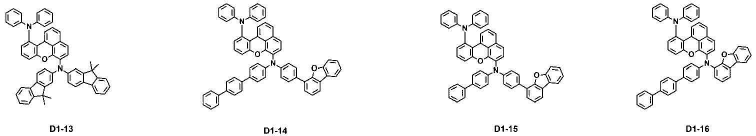

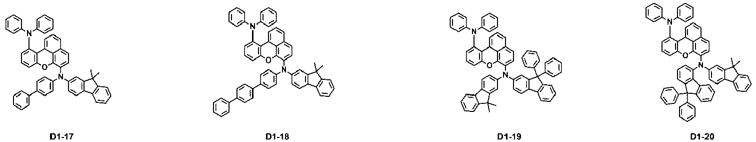

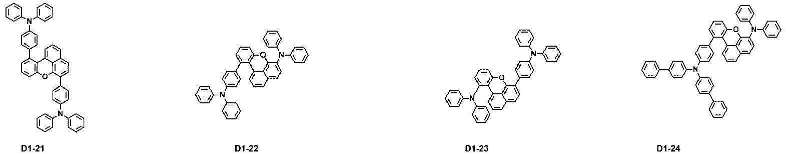

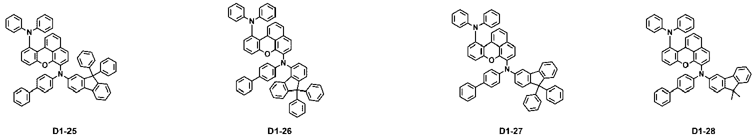

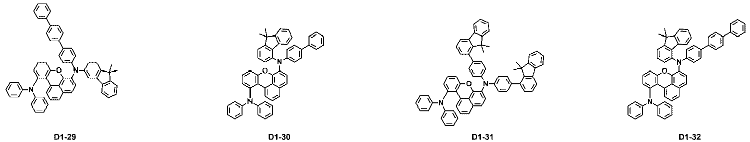

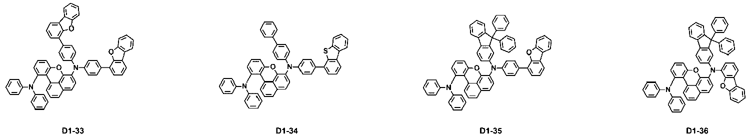

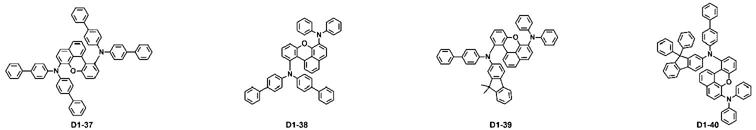

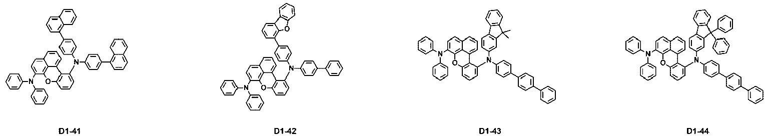

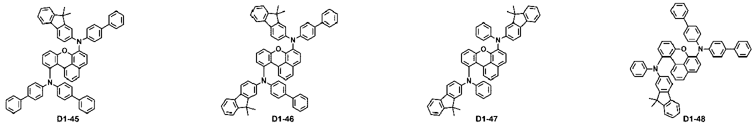

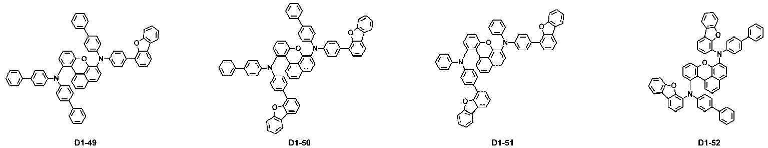

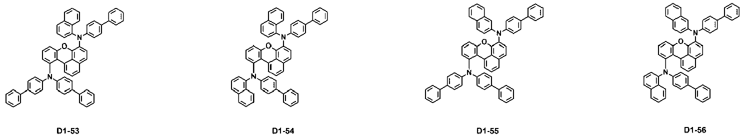

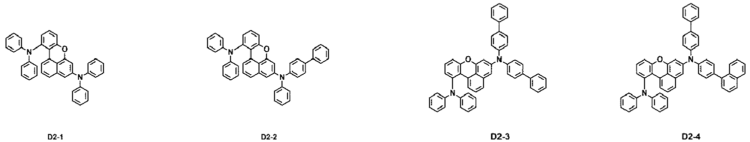

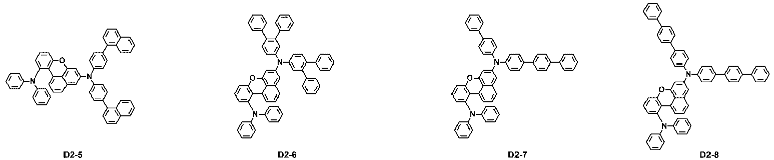

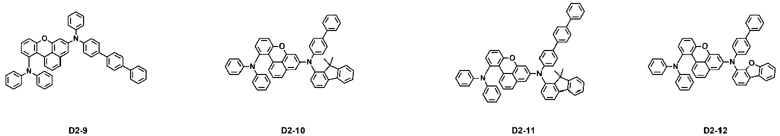

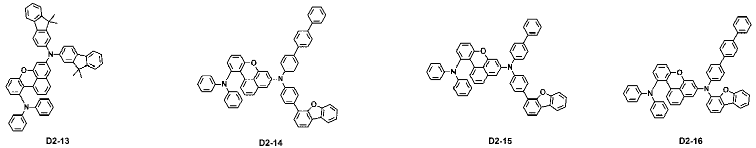

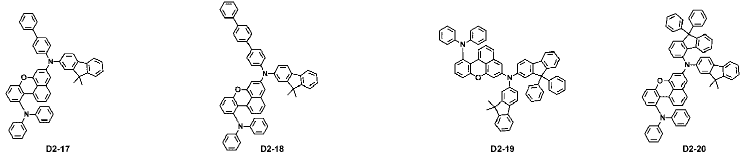

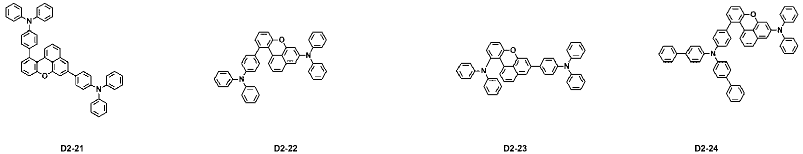

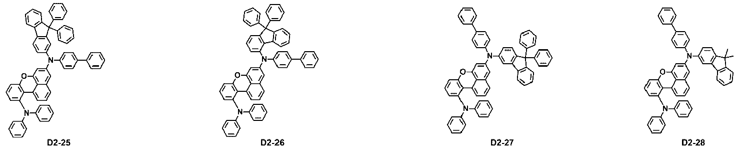

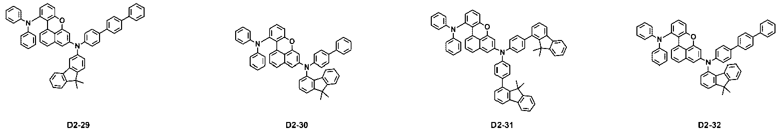

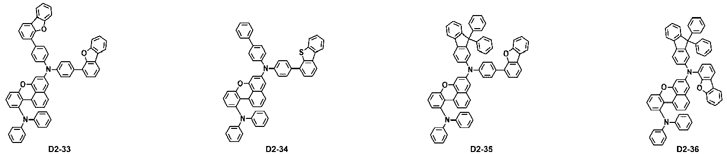

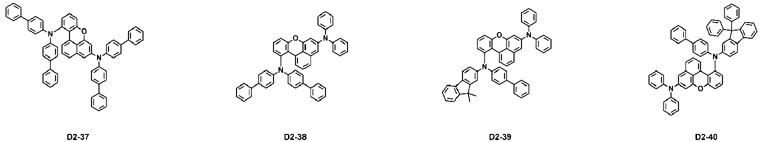

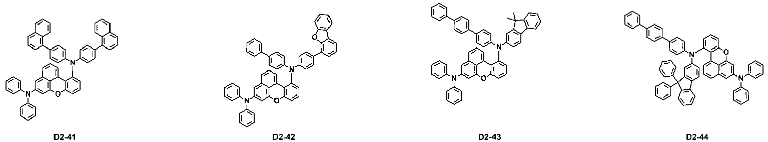

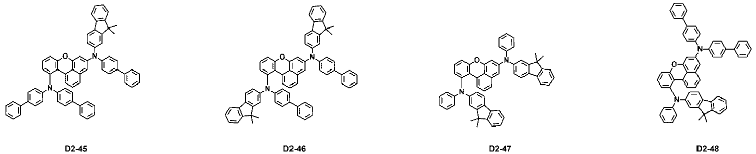

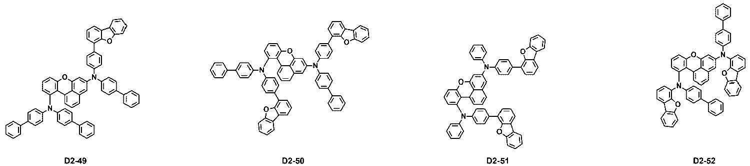

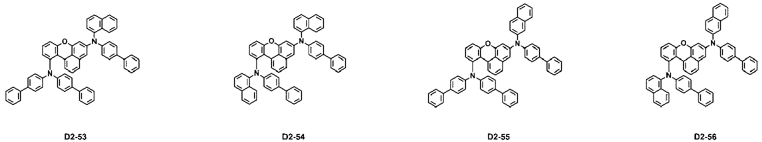

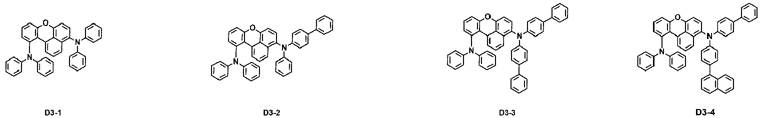

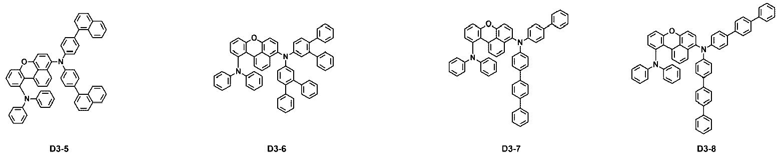

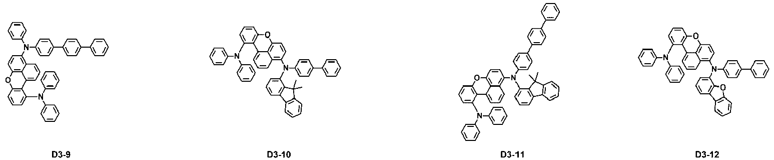

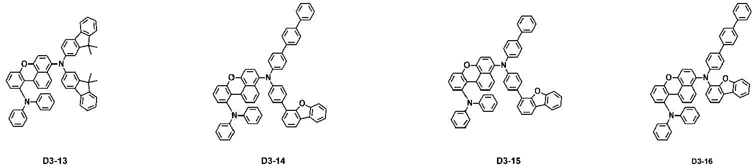

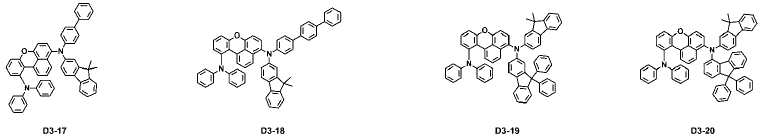

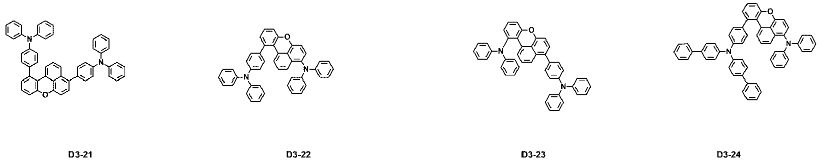

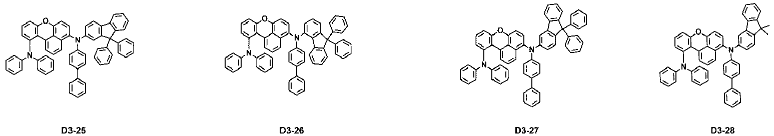

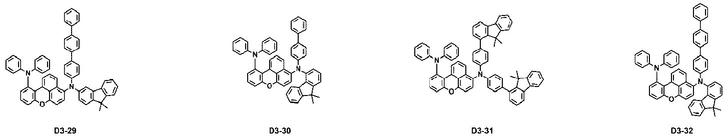

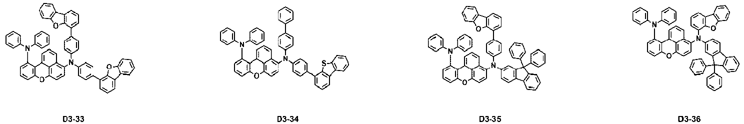

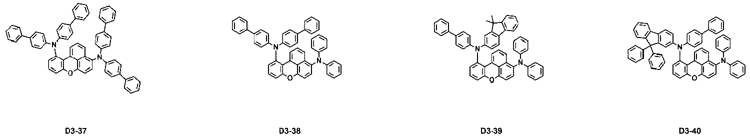

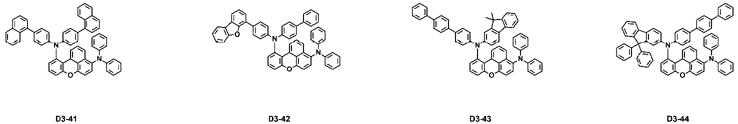

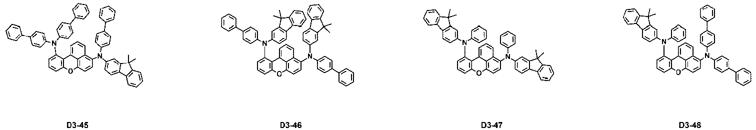

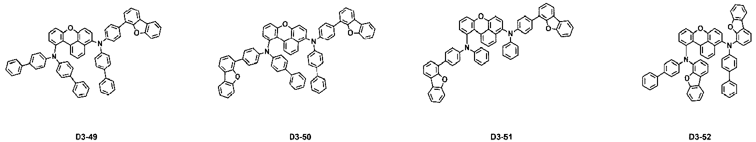

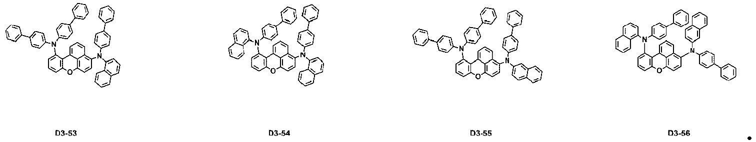

7. An organic light emitting device comprising:
a first electrode;
a second electrode; and
one or more organic material layers provided between the first electrode and the second electrode,
wherein one or more layers of the organic material layers comprise one or more types of the heterocyclic compound of any one of Claims 1 to 6.

8. The organic light emitting device of Claim 7, wherein the organic material layer comprises a hole transfer layer, and the hole transfer layer includes the heterocyclic compound.

9. The organic light emitting device of Claim 7, wherein the organic material layer comprises an electron blocking layer, and the electron blocking layer comprises the heterocyclic compound.

10. The organic light emitting device of Claim 7, further comprising one layer selected from the group consisting of a light emitting layer, a hole injection layer, a hole transfer layer, an electron injection layer, an electron transfer layer, an electron blocking layer and a hole blocking layer.
